(19) Europäisches Patentamt
European Patent Office
Office européen des brevets

(11) EP 4 450 624 A1

(12) **EUROPEAN PATENT APPLICATION**
published in accordance with Art. 153(4) EPC

(43) Date of publication:
**23.10.2024 Bulletin 2024/43**

(21) Application number: **22906685.7**

(22) Date of filing: **16.12.2022**

(51) International Patent Classification (IPC):
***C12N 15/113*** *(2010.01)* ***A61K 47/54*** *(2017.01)*
***A61K 48/00*** *(2006.01)* ***A61P 9/10*** *(2006.01)*

(52) Cooperative Patent Classification (CPC):
**A61K 47/54; A61K 48/00; A61P 9/10; C12N 15/113**

(86) International application number:
**PCT/CN2022/139500**

(87) International publication number:
**WO 2023/109940 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.12.2021 CN 202111545699**

(71) Applicant: **Tuojie Biotech (Shanghai) Co., Ltd.**
**Shanghai 201203 (CN)**

(72) Inventors:
- **LI, Yunfei**
  **Shanghai 201203 (CN)**
- **DENG, Yongyan**
  **Shanghai 201203 (CN)**
- **HOU, Zhe**
  **Shanghai 201203 (CN)**
- **ZHANG, Jianyu**
  **Shanghai 201203 (CN)**
- **WANG, Yanhui**
  **Shanghai 201203 (CN)**
- **MAO, Song**
  **Shanghai 201203 (CN)**
- **HUANG, Jinyu**
  **Shanghai 201203 (CN)**
- **LIU, Nan**
  **Shanghai 201203 (CN)**
- **CAI, Guoqing**
  **Shanghai 201203 (CN)**
- **LV, Zhenzhen**
  **Shanghai 201203 (CN)**
- **HUANG, Yanfen**
  **Shanghai 201203 (CN)**
- **ZHOU, Yaqin**
  **Shanghai 201203 (CN)**
- **LUO, Min**
  **Shanghai 201203 (CN)**
- **ZHANG, Fang**
  **Shanghai 201203 (CN)**

(74) Representative: **Dragotti & Associati S.R.L.**
**Via Nino Bixio, 7**
**20129 Milano (IT)**

# (54) LPA-TARGETING SIRNA AND CONJUGATE

(57) The present invention relates to LPA-targeting siRNA and a conjugate, and specifically relates to LPA-targeting siRNA, a siRNA conjugate, a composition, and a pharmaceutical use thereof. The present invention also relates to a pharmaceutical composition, a cell or a kit comprising the siRNA, and a method for the siRNA and the siRNA conjugate for treating and/or preventing related disorders suffered by a subject.

EP 4 450 624 A1

## Description

The present disclosure claims priority to Chinese Patent Application No. 202111545699.6 filed on December 16, 2021, which is incorporated herein by reference in its entirety.

## TECHNICAL FIELD

The present disclosure belongs to the field of biomedicine and particularly relates to an siRNA, a conjugate, and a composition for inhibiting the expression of apolipoprotein(a) gene (Apo(a) gene, LPA) and medical use thereof.

## BACKGROUND

Lipoprotein(a) [Lp(a)], first discovered by Norwegian geneticist Berg in 1963, was identified as a unique lipoprotein (Berg K. A new serum type system in man-the Lp system. Acta Pathol Microbiol Scand 1963; 59: 369-82). Lp(a) is composed of two parts, lipid and protein. The lipid part is mainly LDL-like particles located in the core, and the protein part is located in the periphery and is composed of apolipoprotein(a) [apo(a)] and apoB100 linked by a disulfide bond. apo(a) is expressed predominantly in the liver and only in human beings and non-human primates and is characterized by the presence of three internal disulfide-stabilized tricyclic structural domains (Kringle). In the human lineage, the amplification and differentiation of the Kringle IV domain in apo(a) results in ten different types of KIV domains, with further amplification of Kringle IV type 2 (KIV-2) producing copy number variation (CNV) within multiple alleles (1-40 copies) and the other Kringle IV encoding domains (KIV-1 and KIV-3 to KIV-10) being present only as a single copy (Schmidt K, Noureen A, Kronenberg F, et al. Structure, Function, and Genetics of Lipoprotein(a), [J]. Journal of Lipid Research, 2016, 57(8):1339). All Kringles are transcribed and translated, and therefore KIV-2 CNV leads to the size polymorphism of apo(a) encoded; its expression is inversely proportional to the number of KIV-2 domains present, and the Lp(a) content in plasma increases significantly when the KIV-2 copy number is low.

Patients with elevated Lp(a) have a 2-3 times higher risk of suffering from cardiovascular events than normal people, and the cardiovascular events caused include atherosclerotic cardiovascular disease, lower extremity arterial disease, aortic stenosis, etc. (EnAs EA, Varkey B, Dharmarajan T S, et al. Lipoprotein(a): An independent, genetic, and causal factor for cardiovascular disease and acute myocardial infarction[J]. Indian Heart Journal, 2019, 71(2)). Lp(a) may lead to undesirable atherosclerotic cardiovascular disease (ASCVD) by two mechanisms: in one aspect, since apo(a) has been shown to inhibit fibrinolysis *in vitro,* it may promote thrombosis at a place where there is plaque rupture or turbulence at a place where there is vessel stenosis, leading to vessel occlusion or promoting thrombosis; in another aspect, LDL-like particles may promote intimal cholesterol deposition and inflammation or the formation of oxidized phospholipids, leading to atherosclerotic stenosis or aortic stenosis (Albert Youngwoo Jang, Seung Hwan Han, Il Suk Sohn, et al. Lipoprotein(a) and Cardiovascular Diseases[J]. Circulation Journal, 2020, 84: 867-874). However, even at very high levels of Lp(a), the cholesterol level is below the traditional LDL threshold, and therefore the LDL-like particles may be less pathogenic in this aspect.

The 2016 Chinese Guidelines on Prevention and Treatment of Dyslipidemia in Adults defines an Lp(a) level of > 30 mg/dl as abnormal, and based on this standard, about 30% of patients with a history of cardiovascular events in China have abnormal Lp(a). The National Lipid Association recommended an Lp(a) of ≥ 50 mg/dl as an elevated level in 2019, and based on this standard, 20% of the global population has an elevated level of Lp(a). Although the elevated level of Lp(a) is common, no targeted therapeutic drugs are available, and no drugs for targeted lowering of Lp(a) have been approved for clinical use to date. The Lp(a) protein has similar structure with a plurality of lipoproteins and is difficult to become a direct target of micromolecule and macromolecule drugs. However, mRNA transcribed from the Lp(a) gene has high specificity, and the regulation mechanism after siRNA transcription can be used to specifically degrade the mRNA, thereby inhibiting the expression of the Lp(a). An siRNA targeting apo(a) gene (LPA) is therefore designed to attenuate its expression, thereby reducing the Lp(a) level in serum, which in turn reduces cardiovascular adverse events.

## SUMMARY

The present disclosure provides an siRNA targeting LPA.

In some embodiments, the present disclosure provides an siRNA, which comprises a sense strand and an antisense strand forming a double-stranded region;

the sense strand comprises a sequence differing by no more than 3 (e.g., 0, 1, 2, or 3) nucleotides from any one of the nucleotide sequences of SEQ ID NO: 1 or SEQ ID NO: 2 and comprises at least 15 (e.g., 16, 17, 18, 19, 20, or 21) contiguous nucleotides;

the antisense strand comprises a sequence differing by no more than 3 (e.g., 0, 1, 2, or 3) nucleotides from any

one of the nucleotide sequences of SEQ ID NO: 3 or SEQ ID NO: 4 and comprises at least 15 (e.g., 16, 17, 18, 19, 20, or 21) contiguous nucleotides.

In some embodiments, the antisense strand is at least partially reverse complementary to a target sequence to mediate RNA interference. In some embodiments, there are no more than 5, no more than 4, no more than 3, no more than 2, or no more than 1 mismatch between the antisense strand and the target sequence. In some embodiments, the antisense strand is fully reverse complementary to the target sequence.

In some embodiments, the sense strand is at least partially reverse complementary to the antisense strand to form a double-stranded region. In some embodiments, there are no more than 5, no more than 4, no more than 3, no more than 2, or no more than 1 mismatch between the sense strand and the antisense strand. In some embodiments, the sense strand is fully reverse complementary to the antisense strand.

In some embodiments, the siRNA of the present disclosure comprises one or two blunt ends.

In some specific embodiments, each strand of the siRNA independently comprises an overhang having 1 to 2 unpaired nucleotides.

In some embodiments, the siRNA of the present disclosure comprises an overhang at the 3' end of the antisense strand of the siRNA.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides (e.g., 19, 20, 21, 22, or 23 nucleotides).

In some embodiments, the sense strand and the antisense strand are identical or different in length; the sense strand is 19-23 nucleotides in length, and the antisense strand is 19-26 nucleotides in length. A ratio of the length of the sense strand to the length of the antisense strand of the siRNA provided by the present disclosure can be 19/19, 19/20, 19/21, 19/22, 19/23, 19/24, 19/25, 19/26, 20/19, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, a ratio of the length of the sense strand to the length of the antisense strand of the siRNA is 19/21, 21/23, or 23/25. In some embodiments, a ratio of the length of the sense strand to the length of the antisense strand of the siRNA is 19/21.

In some embodiments, the sense strand comprises at least 15 contiguous nucleotides and differs by no more than 2 nucleotides from any one of the nucleotide sequences of SEQ ID NO: 1 or SEQ ID NO: 2. In some embodiments, the nucleotide sequence differs by no more than 1 nucleotide; in some embodiments, the difference is 1 nucleotide.

In some embodiments, the antisense strand comprises at least 15 contiguous nucleotide sequences and differs by no more than 2 nucleotides from the nucleotide sequence of SEQ ID NO: 3 or SEQ ID NO: 4; in some embodiments, the nucleotide sequence differs by no more than 1 nucleotide; in some embodiments, the difference is 1 nucleotide.

In some embodiments, the sense strand comprises at least 15 contiguous nucleotides of any one of the nucleotide sequences of SEQ ID NO: 1 or SEQ ID NO: 2. In some embodiments, the sense strand comprises at least 16 contiguous nucleotides of any one of the nucleotide sequences of SEQ ID NO: 1 or SEQ ID NO: 2. In some embodiments, the sense strand comprises at least 18 contiguous nucleotides of any one of the nucleotide sequences of SEQ ID NO: 1 or SEQ ID NO: 2. In some embodiments, the sense strand comprises at least 19 contiguous nucleotides of any one of the nucleotide sequences of SEQ ID NO: 1 or SEQ ID NO: 2.

In some embodiments, the antisense strand comprises at least 15 contiguous nucleotides of any one of the nucleotide sequences of SEQ ID NO: 3 or SEQ ID NO: 4. In some embodiments, the antisense strand comprises at least 18 contiguous nucleotides of any one of the nucleotide sequences of SEQ ID NO: 3 or SEQ ID NO: 4. In some embodiments, the antisense strand comprises at least 19 contiguous nucleotides of any one of the nucleotide sequences of SEQ ID NO: 3 or SEQ ID NO: 4. In some embodiments, the antisense strand comprises at least 20 contiguous nucleotides of any one of the nucleotide sequences of SEQ ID NO: 3 or SEQ ID NO: 4. In some embodiments, the antisense strand comprises at least 21 contiguous nucleotides of any one of the nucleotide sequences of SEQ ID NO: 3 or SEQ ID NO: 4.

In some embodiments, the sense strand comprises a nucleotide sequence selected from the group consisting of the following: SEQ ID NO: 1 or SEQ ID NO: 2.

In some embodiments, the antisense strand comprises a nucleotide sequence selected from the group consisting of the following: SEQ ID NO: 3 or SEQ ID NO: 4.

In some embodiments, the siRNA of the present disclosure comprises or is selected from any one of the following:

group 1), a sense strand set forth in SEQ ID NO: 1 and an antisense strand set forth in SEQ ID NO: 3;
group 2), a sense strand set forth in SEQ ID NO: 2 and an antisense strand set forth in SEQ ID NO: 4.

In some embodiments, the siRNA of the present disclosure comprises or is selected from any one of the following:

group 1), the sense strand is the nucleotide sequence set forth in SEQ ID NO: 1, and the antisense strand is the nucleotide sequence set forth in SEQ ID NO: 3;
group 2), the sense strand is the nucleotide sequence set forth in SEQ ID NO: 2, and the antisense strand is the nucleotide sequence set forth in SEQ ID NO: 4.

In the present disclosure, according to the 5'-3' direction,

SEQ ID NO: 1 is GCUCCUUAUUGUUAUACGA;
SEQ ID NO: 2 is ACACCACAUCAACAUAAUA;
SEQ ID NO: 3 is UCGUAUAACAAUAAGGAGCUG;
SEQ ID NO: 4 is UAUUAUGUUGAUGUGGUGUCA.

In some embodiments, at least one nucleotide in the sense strand and/or the antisense strand is a modified nucleotide.
In some embodiments, all of the nucleotides are modified nucleotides.
The present disclosure provides an siRNA, which comprises a sense strand and an antisense strand forming a double-stranded region;

the sense strand comprises a sequence differing by no more than 3 nucleotides from any one of the nucleotide sequences of SEQ ID NO: 1 or SEQ ID NO: 2 and comprises at least 15 contiguous nucleotides; the antisense strand comprises a sequence differing by no more than 3 nucleotides from any one of the nucleotide sequences of SEQ ID NO: 3 or SEQ ID NO: 4 and comprises at least 15 contiguous nucleotides; at least one nucleotide at positions 2 to 8 (e.g., position 2, position 3, position 4, position 5, position 6, position 7, or position 8) of the 5' region of the antisense strand comprises a 2'-methoxy modification or a chemical modification of formula (I) or a tautomeric modification thereof;
formula (I) is selected from:

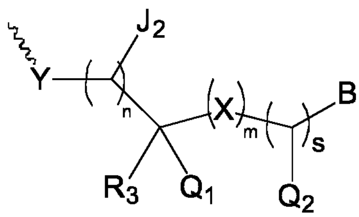 ;

(I)

wherein Y is selected from the group consisting of O, NH, and S;
each X is independently selected from the group consisting of $CR_4(R_4')$, S, NRs and NH-CO, wherein $R_4$, $R_4'$, and $R_5$ are each independently H or $C_1$-$C_6$ alkyl;
$J_2$ is H or $C_1$-$C_6$ alkyl;
n = 0, 1, or 2; m = 0, 1, or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;
Qi is

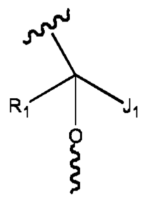 ,

and $Q_2$ is $R_2$; or $Q_1$ is $R_2$, and $Q_2$ is

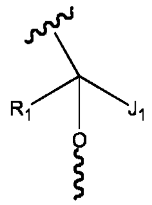

;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl, and $(CH_2)_qR_7$, wherein $R_7$ is selected from the group consisting of OH, halogen, methoxy, ethoxy, $N_3$, $C_2$-$C_6$ alkenyl, and $C_2$-$C_6$ alkynyl, and q = 1, 2, or 3;

$J_1$ is H or $C_1$-$C_6$ alkyl;

$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_rR_8$, wherein $R_8$ is selected from the group consisting of OH, halogen, methoxy, ethoxy, $N_3$, $C_2$-$C_6$ alkenyl, and $C_2$-$C_6$ alkynyl, and r = 1, 2, or 3;

optionally, $R_1$ and $R_2$ are directly linked to form a ring;

B is a base;

wherein the chemical modification of formula (I) or the tautomeric modification thereof is not

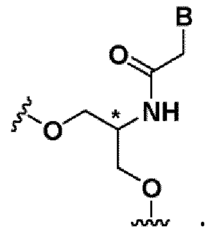

.

In some embodiments, when X is NH-CO, $R_1$ is not H.

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, formula (I) is selected from formula (I-1):

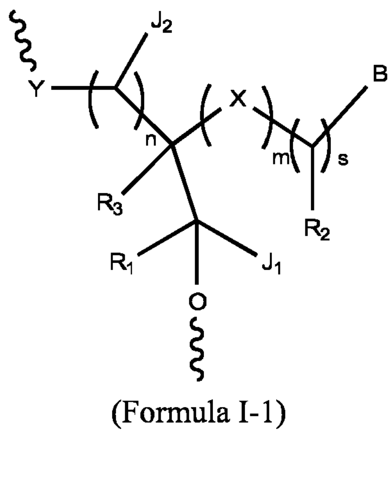

(Formula I-1)

wherein:

Y is selected from the group consisting of O, NH, and S;
each X is independently selected from the group consisting of $CR_4(R_4')$, S, NRs and NH-CO, wherein $R_4$, $R_4'$, and $R_5$ are each independently H or $C_1$-$C_6$ alkyl;
each $J_1$ and each $J_2$ are each independently H or $C_1$-$C_6$ alkyl;
n = 0, 1, or 2; m = 0, 1, or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl, and $(CH_2)_qR_7$, wherein $R_7$ is selected from the group consisting of OH, halogen, methoxy, ethoxy, $N_3$, $C_2$-$C_6$ alkenyl, and $C_2$-$C_6$ alkynyl, and q = 1, 2, or 3;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and (CHz)rRs, wherein $R_8$ is selected from the group consisting of OH, halogen, methoxy, ethoxy, $N_3$, $C_2$-$C_6$ alkenyl, and $C_2$-$C_6$ alkynyl, and r = 1, 2, or 3;
optionally, $R_1$ and $R_2$ are directly linked to form a ring;
B is as defined in formula (I).

In some embodiments of formula (I-1), B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, formula (I) is selected from formula (I-2):

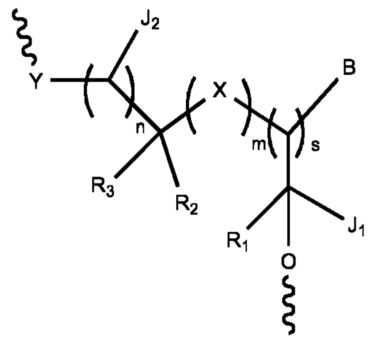

(I-2)

wherein,

Y is selected from the group consisting of O, NH, and S;
each X is independently selected from the group consisting of $CR_4(R_4')$, S, NRs and NH-CO, wherein $R_4$, $R_4'$, and $R_5$ are each independently H or $C_1$-$C_6$ alkyl;
n = 0, 1, or 2; m = 0, 1, or 2; s = 0 or 1;
each $J_1$ and each $J_2$ are each independently H or $C_1$-$C_6$ alkyl;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl, and $(CH_2)_qR_7$, wherein $R_7$ is selected from the group consisting of OH, halogen, methoxy, ethoxy, $N_3$, $C_2$-$C_6$ alkenyl, and $C_2$-$C_6$ alkynyl, and q = 1, 2, or 3;
$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino, and $(CH_2)_rR_8$, wherein $R_8$ is selected from the group consisting of OH, halogen, methoxy, ethoxy, $N_3$, $C_2$-$C_6$ alkenyl, and $C_2$-$C_6$ alkynyl, and r = 1, 2, or 3;

optionally, $R_1$ and $R_2$ are directly linked to form a ring;
B is as defined in formula (I).

In some embodiments of formula (I-2), B is a base; for example, B is selected from the group consisting of purine bases, pyrimidine bases, indole, 5-nitroindole, and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.
In some embodiments, B is a base at a position corresponding to the modified nucleotide of the antisense strand.
In some embodiments, the chemical modification or the tautomeric modification thereof described above is not

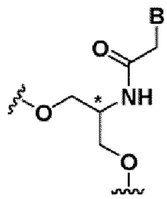
.

In some embodiments, each X is independently selected from the group consisting of $CR_4(R_4')$, S, NRs, and NH-CO, wherein $R_4$, $R_4'$, and $R_5$ are each independently H or Ci-$C_3$ alkyl;

n = 0, 1, or 2; m = 0, 1, or 2; s = 0 or 1;
each $J_1$ and each $J_2$ are each independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl, and $(CH_2)_qR_7$, wherein $R_7$ is selected from the group consisting of OH, halogen, methoxy, ethoxy, $N_3$, $C_2$-$C_4$ alkenyl, and $C_2$-$C_4$ alkynyl, and q = 1, 2, or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino, and $(CH_2)_rR_8$, wherein $R_8$ is selected from the group consisting of OH, halogen, methoxy, ethoxy, $N_3$, $C_2$-$C_4$ alkenyl, and $C_2$-$C_4$ alkynyl, and r = 1, 2, or 3;
optionally, $R_1$ and $R_2$ are directly linked to form a ring.
B is as defined in formula (I).

In some embodiments, B is a base; for example, B is selected from the group consisting of purine bases, pyrimidine bases, indole, 5-nitroindole, and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.
In some embodiments, B is a base at a position corresponding to the modified nucleotide of the antisense strand.
In some embodiments, each X is independently selected from the group consisting of $CR_4(R_4')$, S, $NR_5$, and NH-CO, wherein $R_4$, $R_4'$, and $R_5$ are each independently H, methyl, ethyl, n-propyl, or isopropyl;

n = 0, 1, or 2; m = 0, 1, or 2; s = 0 or 1;
each $J_1$ and each $J_2$ are each independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino, and $(CH_2)_pR_6$, wherein $R_6$ is selected from the group consisting of OH, F, Cl, methoxy, ethoxy, $N_3$, vinyl, allyl, ethynyl, and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl, and $(CH_2)_qR_7$, wherein $R_7$ is selected from the group consisting of OH, F, Cl, methoxy, ethoxy, $N_3$, vinyl, allyl, ethynyl, and propargyl, and q = 1 or 2;

$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino, and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;

optionally, $R_1$ and $R_2$ are directly linked to form a ring.

B is as defined in formula (I).

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

B is as defined in formula (I).

In some embodiments, B is a base; for example, B is selected from the group consisting of those of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, Y is O or NH; each X is independently selected from the group consisting of NH-CO, $CH_2$, and NH;

n = 0 or1; m = 0 or1; s = 0 or 1;

each $J_1$ and each $J_2$ are each independently H;

$R_1$ is selected from the group consisting of H, methyl, and $CH_2OH$;

$R_2$ is selected from the group consisting of H, OH, $NH_2$, methyl, and CHzOH;

$R_3$ is selected from the group consisting of H, OH, $NH_2$, methyl, and CHzOH; optionally, $R_1$ and $R_2$ are directly linked to form a ring;

B is as defined in formula (I).

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, Y is O or NH; each X is independently selected from the group consisting of NH-CO, $CH_2$, and NH;

n = 0 or1; m = 0 or1; s = 0 or 1;

each $J_1$ and each $J_2$ are each independently H;

$R_1$ is selected from the group consisting of H, methyl, and $CH_2OH$;

$R_2$ is selected from the group consisting of H, methyl, and $CH_2OH$;

$R_3$ is selected from the group consisting of H, OH, $NH_2$, methyl, and CHzOH; optionally, $R_1$ and $R_2$ are directly linked to form a ring.

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, in some embodiments, the chemical modification of formula (I) or the tautomeric modification thereof is selected from the group consisting of:

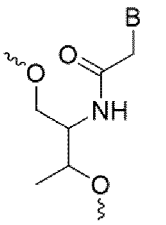, 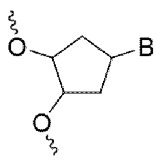, 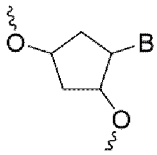, 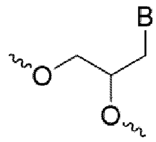, 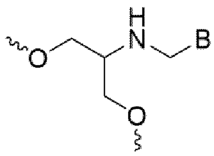,

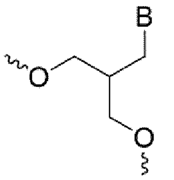, 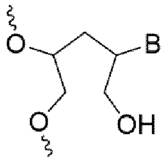, 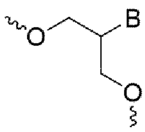, 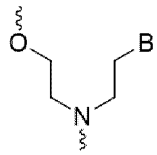, 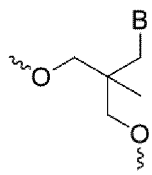,

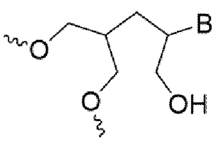, 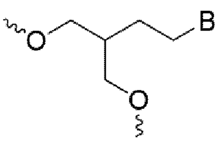,

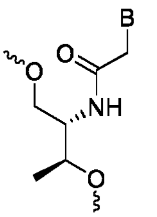, 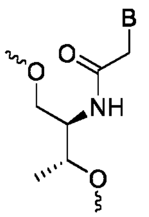, 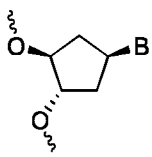, 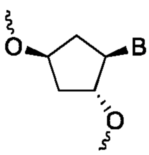, 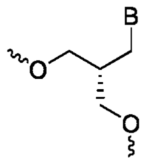,

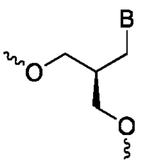, 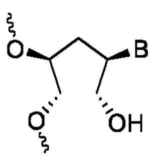, 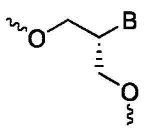, 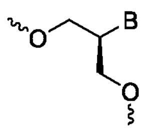, 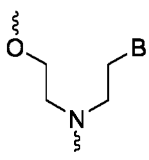,

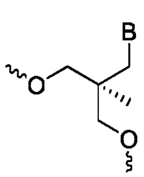, 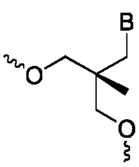, 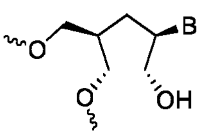, 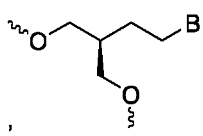, 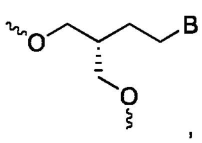,

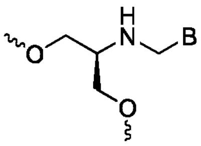, 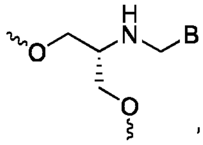, 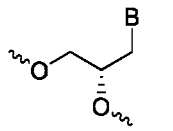, 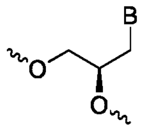,

wherein B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, the chemical modification of formula (I) or the tautomeric modification thereof is selected from the group consisting of:

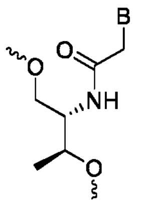, 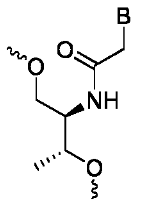, 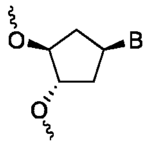, 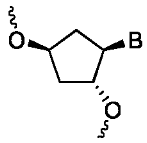, 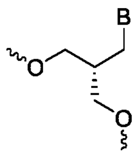,

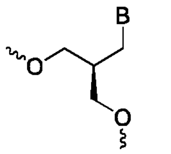,

,

wherein B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, the chemical modification of formula (I) or the tautomeric modification thereof is selected from the group consisting of:

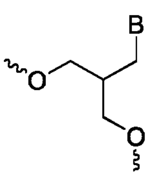, 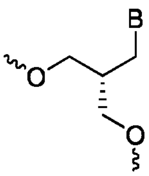, or 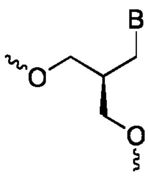;

wherein B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a corresponding position among positions 2 to 8 of the 5' region of the antisense strand.

In some embodiments, when the chemical modification of formula (I) or the tautomeric modification thereof is at position 5 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole. In some embodiments, B is a base corresponding to position 5 of the 5' region of the antisense strand.

In some embodiments, when the chemical modification of formula (I) or the tautomeric modification thereof is at position 6 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole. In some embodiments, B is a base corresponding to position 6 of the 5' region of the antisense strand.

In some embodiments, when the chemical modification of formula (I) or the tautomeric modification thereof is at position 7 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole. In some embodiments, B is a base corresponding to position 7 of the 5' region of the antisense strand.

In some embodiments, a nucleotide comprising a chemical modification of formula (I) or a tautomeric modification thereof is selected from a nucleotide comprising a chemical modification of formula (I') or a tautomeric modification thereof,

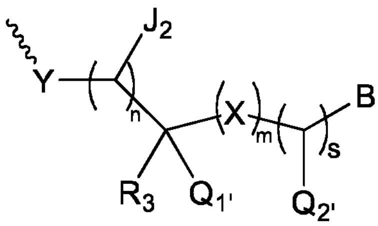

(I')

wherein Y is selected from the group consisting of O, NH, and S;

each X is independently selected from the group consisting of $CR_4(R_4')$, S, NRs and NH-CO, wherein $R_4$, $R_4'$, and $R_5$ are each independently H or $C_1$-$C_6$ alkyl;

$J_2$ is H or $C_1$-$C_6$ alkyl;

n = 0, 1, or 2; m = 0, 1, or 2; s = 0 or 1;

$R_3$ is selected from the group consisting of H, OH, halogen, $NH_2$, Ci-Cs alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;

$Q_{1'}$ is

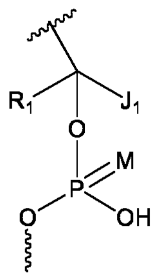

,

and $Q_{2'}$ is $R_2$; or $Q_{1'}$ is $R_2$, and $Q_{2'}$ is

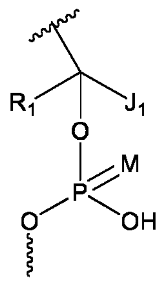

;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl, and $(CH_2)_qR_7$, wherein $R_7$ is selected from the group consisting of OH, halogen, methoxy, ethoxy, $N_3$, $C_2$-$C_6$ alkenyl, and $C_2$-$C_6$ alkynyl, and q = 1, 2, or 3;

$J_1$ is H or $C_1$-$C_6$ alkyl;

$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_rR_8$, wherein $R_8$ is selected from the group consisting of OH, halogen, methoxy, ethoxy, $N_3$, $C_2$-$C_6$ alkenyl, and $C_2$-$C_6$ alkynyl, and r = 1, 2, or 3;

optionally, $R_1$ and $R_2$ are directly linked to form a ring;

B is a base;

M is O or S;

wherein the chemical modification of formula (I') or the tautomeric modification thereof is not

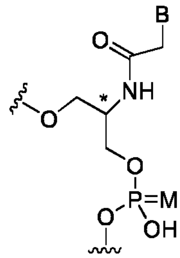

.

In some embodiments, when X is NH-CO, $R_1$ is not H.

In some embodiments of formula (I'), B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, formula (I') is selected from formula (I'-1):

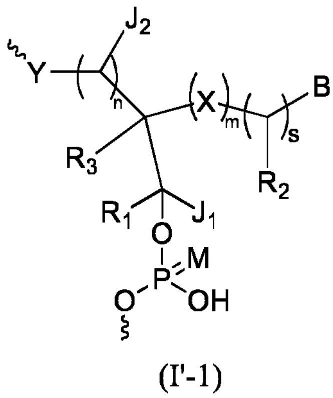

(I'-1)

wherein Y is selected from the group consisting of O, NH, and S;

each X is independently selected from the group consisting of $CR_4(R_4')$, S, $NR_5$ and NH-CO, wherein $R_4$, $R_4'$, and $R_5$ are each independently H or $C_1$-$C_6$ alkyl;

each $J_1$ and each $J_2$ are each independently H or $C_1$-$C_6$ alkyl;

n = 0, 1, or 2; m = 0, 1, or 2; s = 0 or 1;

$R_3$ is selected from the group consisting of H, OH, halogen, $NH_2$, Ci-Cs alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;

$R_1$ is selected from the group consisting of H, $C_1$-$C_6$ alkyl, $C_1$-$C_6$ alkoxy, $C_2$-$C_6$ alkenyl, $C_2$-$C_6$ alkynyl, and $(CH_2)_qR_7$, wherein $R_7$ is selected from the group consisting of OH, halogen, methoxy, ethoxy, $N_3$, $C_2$-$C_6$ alkenyl, and $C_2$-$C_6$

alkynyl, and q = 1, 2, or 3;

$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_rR_8$, wherein $R_8$ is selected from the group consisting of OH, halogen, methoxy, ethoxy, $N_3$, $C_2$-$C_6$ alkenyl, and $C_2$-$C_6$ alkynyl, and r = 1, 2, or 3;

M is O or S;

optionally, $R_1$ and $R_2$ are directly linked to form a ring;

B is as defined in formula (I').

In some embodiments of formula (I'-1), B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, formula (I') is selected from formula (I'-2):

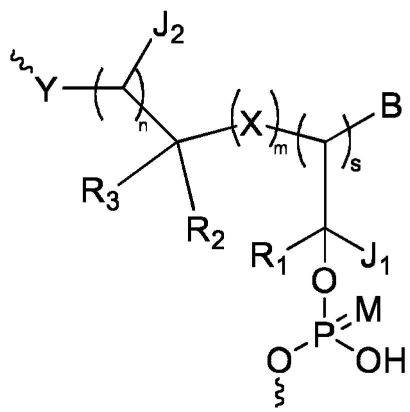

(I'-2)

wherein Y is selected from the group consisting of O, NH, and S;

each X is independently selected from the group consisting of $CR_4(R_4')$, S, NRs and NH-CO, wherein $R_4$, $R_4'$, and $R_5$ are each independently H or $C_1$-$C_6$ alkyl;

n = 0, 1, or 2; m = 0, 1, or 2; s = 0 or 1;

each $J_1$ and each $J_2$ are each independently H or $C_1$-$C_6$ alkyl;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;

$R_1$ is selected from the group consisting of H, $C_1$-$C_6$ alkyl, $C_1$-$C_6$ alkoxy, $C_2$-$C_6$ alkenyl, $C_2$-$C_6$ alkynyl, and $(CH_2)_qR_7$, wherein $R_7$ is selected from the group consisting of OH, halogen, methoxy, ethoxy, $N_3$, $C_2$-$C_6$ alkenyl, and $C_2$-$C_6$ alkynyl, and q = 1, 2, or 3;

$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino, and $(CH_2)_rR_8$, wherein $R_8$ is selected from the group consisting of OH, halogen, methoxy, ethoxy, $N_3$, $C_2$-$C_6$ alkenyl, and $C_2$-$C_6$ alkynyl, and r = 1, 2, or 3;

optionally, $R_1$ and $R_2$ are directly linked to form a ring;

M is O or S;

B is as defined in formula (I').

In some embodiments of formula (I'-2), B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, the chemical modification or the tautomeric modification thereof described above is not

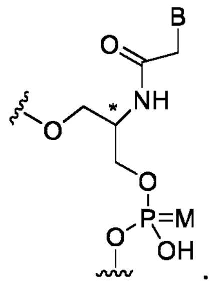

In some embodiments, when X is NH-CO, $R_1$ is not H.

In some embodiments, each X is independently selected from the group consisting of $CR_4(R_4')$, S, NRs, and NH-CO, wherein $R_4$, $R_4'$, and $R_5$ are each independently H or Ci-$C_3$ alkyl;

n = 0, 1, or 2; m = 0, 1, or 2; s = 0 or 1;

each $J_1$ and each $J_2$ are each independently H or $C_1$-$C_3$ alkyl;

$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino, and $(CH_2)_pR_6$, wherein $R_6$ is selected from the group consisting of OH, halogen, methoxy, ethoxy, $N_3$, $C_2$-$C_6$ alkenyl, and $C_2$-$C_6$ alkynyl, and p = 1, 2, or 3;

$R_1$ is selected from the group consisting of H, $C_1$-$C_3$ alkyl, $C_1$-$C_3$ alkoxy, $C_2$-$C_4$ alkenyl, $C_2$-$C_4$ alkynyl, and $(CH_2)_qR_7$, wherein $R_7$ is selected from the group consisting of OH, halogen, methoxy, ethoxy, $N_3$, $C_2$-$C_4$ alkenyl, and $C_2$-$C_4$ alkynyl, and q = 1, 2, or 3;

$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino, and $(CH_2)_rR_8$, wherein $R_8$ is selected from the group consisting of OH, halogen, methoxy, ethoxy, $N_3$, $C_2$-$C_4$ alkenyl, and $C_2$-$C_4$ alkynyl, and r = 1, 2, or 3;

optionally, $R_1$ and $R_2$ are directly linked to form a ring.

In some embodiments, each X is independently selected from the group consisting of $CR_4(R_4')$, S, NRs, and NH-CO, wherein $R_4$, $R_4'$, and $R_5$ are each independently H, methyl, ethyl, n-propyl, or isopropyl;

n = 0, 1, or 2; m = 0, 1, or 2; s = 0 or 1;

each $J_1$ and each $J_2$ are each independently H or methyl;

$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino, and $(CH_2)_pR_6$, wherein $R_6$ is selected from the group consisting of OH, F, Cl, methoxy, ethoxy, $N_3$, vinyl, allyl, ethynyl, and propargyl, and p = 1 or 2;

$R_1$ is selected from the group consisting of H, methyl, ethyl, n-propyl, isopropyl, methoxy, ethoxy, n-propoxy, isopropoxy, vinyl, allyl, ethynyl, propargyl, and $(CH_2)_qR_7$, wherein $R_7$ is selected from the group consisting of OH, F, Cl, methoxy, ethoxy, $N_3$, vinyl, allyl, ethynyl, and propargyl, and q = 1 or 2;

$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino, and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;

optionally, $R_1$ and $R_2$ are directly linked to form a ring.

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, Y is O or NH; each X is independently selected from the group consisting of NH-CO, $CH_2$, and NH;

n = 0 or1; m = 0 or1; s = 0 or 1;
each $J_1$ and each $J_2$ are each independently H;
$R_1$ is selected from the group consisting of H, methyl, and $CH_2OH$;
$R_2$ is selected from the group consisting of H, OH, $NH_2$, methyl, and CHzOH;
$R_3$ is selected from the group consisting of H, OH, $NH_2$, methyl, and CHzOH; optionally, $R_1$ and $R_2$ are directly linked to form a ring.

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, Y is O or NH; each X is independently selected from the group consisting of NH-CO, $CH_2$, and NH;

n = 0 or1; m = 0 or1; s = 0 or 1;
each $J_1$ and each $J_2$ are each independently H;
$R_1$ is selected from the group consisting of H, methyl, and CHzOH;
$R_2$ is selected from the group consisting of H, methyl, and $CH_2OH$;
$R_3$ is selected from the group consisting of H, OH, $NH_2$, methyl, and $CH_2OH$; optionally, $R_1$ and $R_2$ are directly linked to form a ring.

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, the chemical modification of formula (I') or the tautomeric modification thereof is selected from the group consisting of:

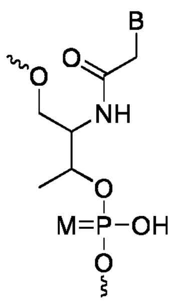 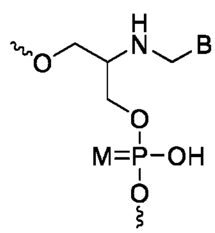 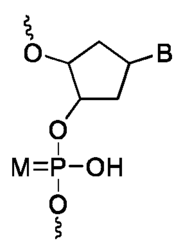 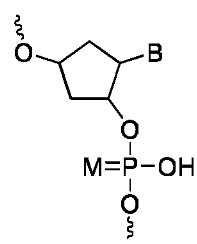 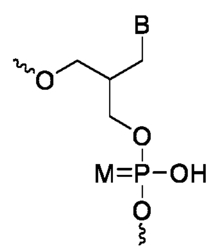

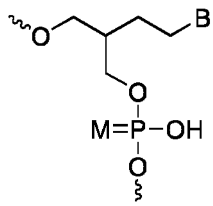 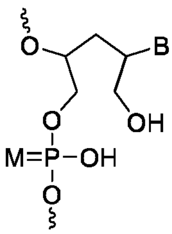 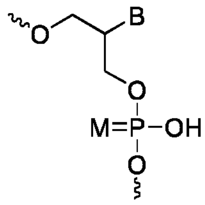 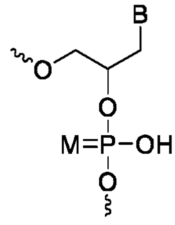 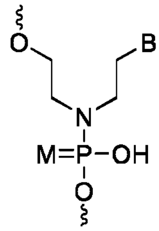

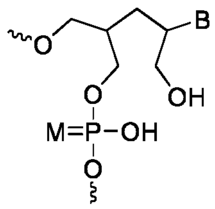 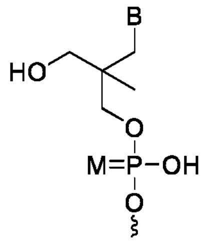

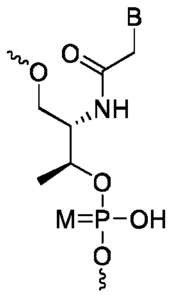 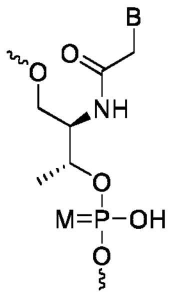 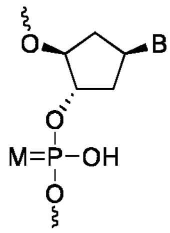 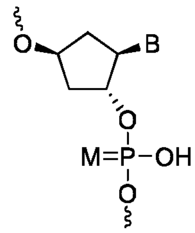 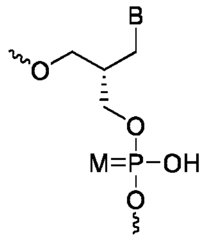

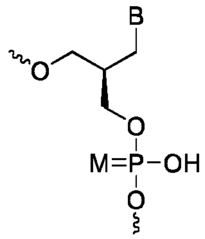 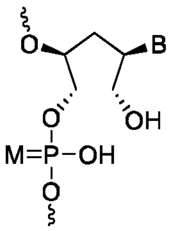 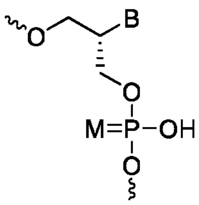 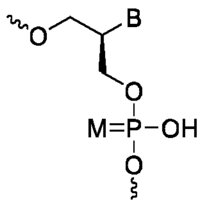 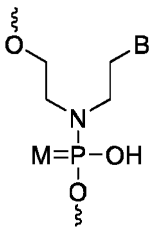

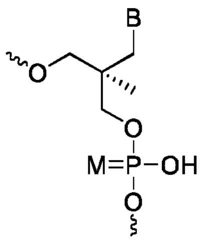 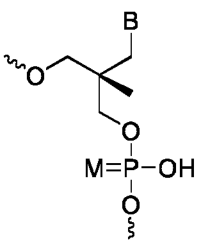 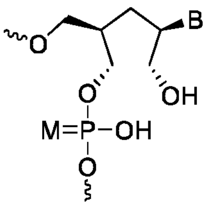 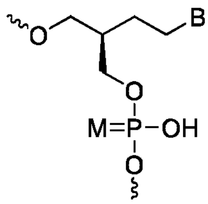 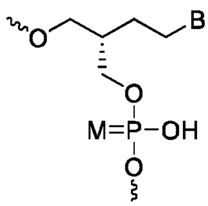

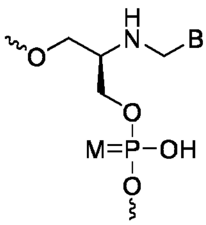 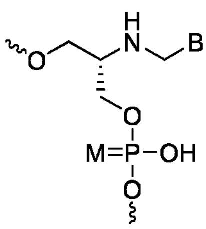 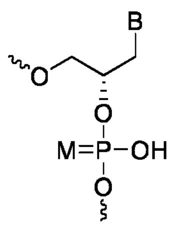 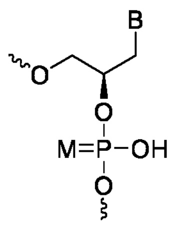

wherein M is O or S;
B is as defined in formula (I').

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, the chemical modification of formula (I') or the tautomeric modification thereof is selected from the group consisting of:

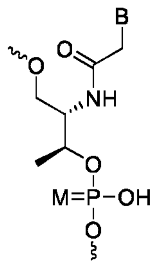
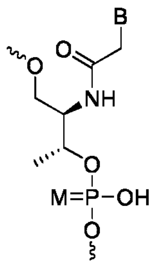
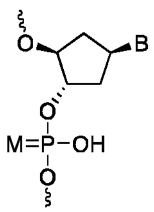
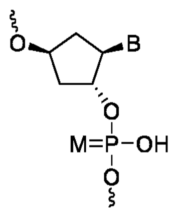
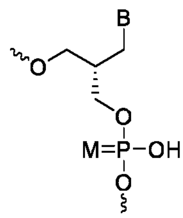
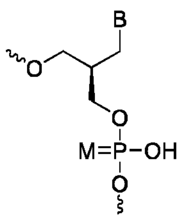
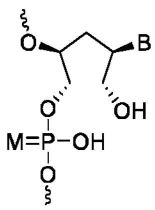
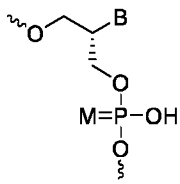
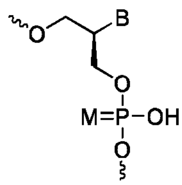
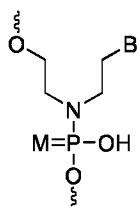
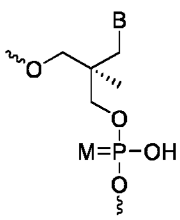
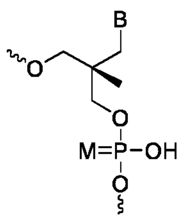

;

wherein M is O or S;
B is as defined in formula (I').

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, the chemical modification of formula (I') or the tautomeric modification thereof is selected from the group consisting of:

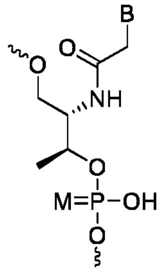
,
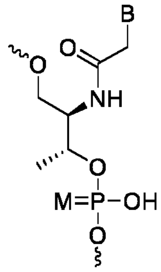
,
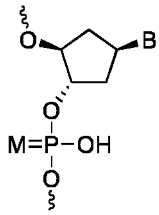
,
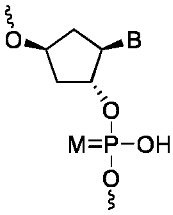
,
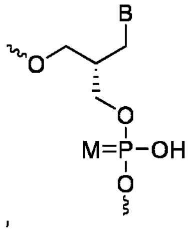
,

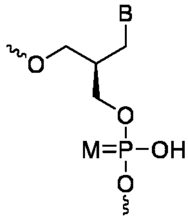

wherein M is O or S;
B is as defined in formula (I').

In some embodiments, B is a base; for example, B is selected from the group consisting of purine bases, pyrimidine bases, indole, 5-nitroindole, and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole, and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole.

In some embodiments, B is a base at a position corresponding to the modified nucleotide of the antisense strand.

In some embodiments, the chemical modification of formula (I') or the tautomeric modification thereof includes, but is not limited to:

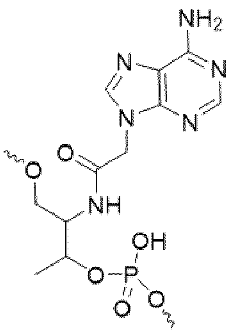
,
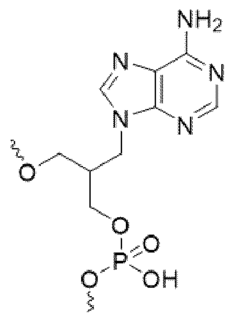
,
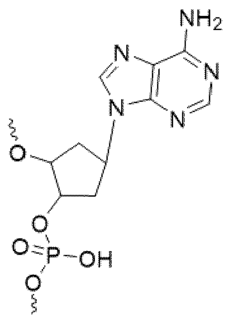
,
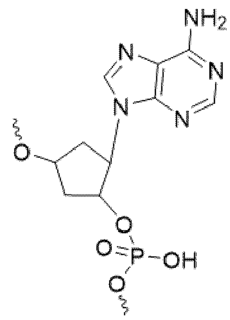
,

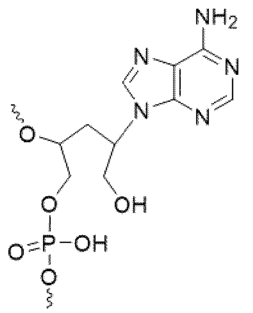
,

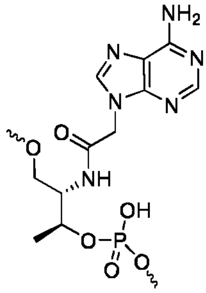 , 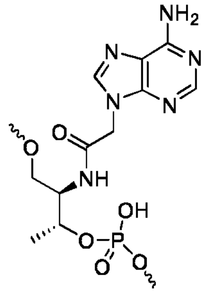 , 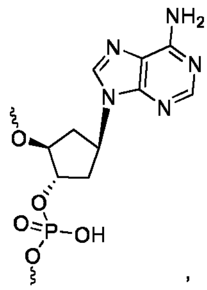 , 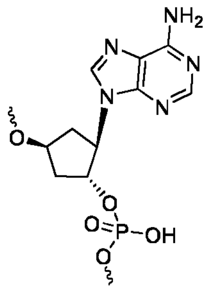 ,

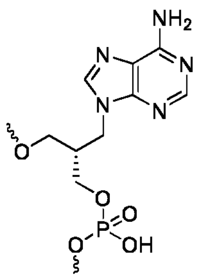 , 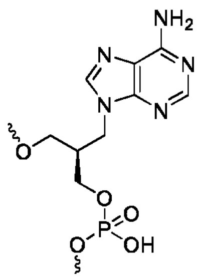 , 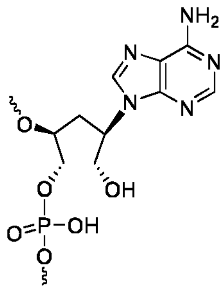

and those where adenine in the structures is replaced with guanine, cytosine, uracil, or thymine.

In some embodiments, at least one nucleotide at positions 2 to 8 (e.g., position 2, position 3, position 4, position 5, position 6, position 7, or position 8) of the 5' region of the antisense strand comprises a 2'-methoxy modification.

In some embodiments, any one of the nucleotides at positions 5, 6, and 7 of the 5' region of the antisense strand comprises a 2'-methoxy modification.

In some embodiments, the modified nucleotide is located at any one of positions 2 to 8 of the 5' region of the antisense strand.

In some embodiments, the modified nucleotide is located at any one of positions 5, 6, or 7 of the 5' region of the antisense strand.

In some embodiments, when the chemical modification of formula (I') or the tautomeric modification thereof is at position 5 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole; in some embodiments, B is a base corresponding to position 5 of the 5' region of the antisense strand.

In some embodiments, when the chemical modification of formula (I') or the tautomeric modification thereof is at position 6 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole; in some embodiments, B is a base corresponding to position 6 of the 5' region of the antisense strand.

In some embodiments, when the chemical modification of formula (I') or the tautomeric modification thereof is at position 7 of the 5' region, B is selected from the group consisting of adenine, guanine, 2,6-diaminopurine, 6-dimethylaminopurine, 2-aminopurine, cytosine, uracil, thymine, indole, 5-nitroindole, and 3-nitropyrrole; in some embodiments, B is a base corresponding to position 7 of the 5' region of the antisense strand.

In some embodiments, at least one additional nucleotide in the sense strand and/or the antisense strand of the siRNA is a modified nucleotide selected from the group consisting of: a 2'-methoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, a 2'-fluoro-modified nucleotide, a 2'-deoxynucleotide, a 2'-deoxy-2'-fluoro-modified nucleotide, a 3'-deoxy-thymine nucleotide, an isonucleotide, LNA, ENA, cET, UNA, and GNA. In some embodiments, the modified nucleotides are each independently selected from the group consisting of: a 2'-methoxy-modified nucleotide, a 2'-fluoro-modified nucleotide, and a 2'-deoxy-modified nucleotide.

In some embodiments, three contiguous nucleotides in the sense strand of the siRNA are 2'-fluoro-modified nucleotides.

In some embodiments, three contiguous nucleotides at positions 7-9 of the 5' end in the sense strand of the siRNA are 2'-fluoro-modified nucleotides.

In some embodiments, three contiguous nucleotides at positions 7-9 of the 5' end in the sense strand of the siRNA are 2'-fluoro-modified nucleotides, and the remaining positions of the sense strand are all non-2'-fluoro-modified nucleotides.

In some embodiments, in a 5'-end to 3'-end direction, nucleotides at positions 2, 4, 6, 9, 12, 14, 16, and 18 of

the antisense strand are each independently a 2'-fluoro-modified nucleotide.

In some other embodiments, in a 5'-end to 3'-end direction, nucleotides at positions 2, 4, 6, 10, 12, 14, 16, and 18 of the antisense strand are each independently a 2'-fluoro-modified nucleotide.

In some embodiments, the sense strand comprises or is selected from the nucleotide sequence of the formula shown below (5'-3'): $N_aN_aN_aN_aXN_aN_bN_bN_bN_aN_aN_aN_aN_aN_aN_aN_aN_aN_a$

wherein each X is independently $N_a$ or $N_b$;
$N_a$ is a 2'-methoxy-modified nucleotide, and $N_b$ is a 2'-fluoro-modified nucleotide.

In some embodiments, the sense strand comprises a nucleotide sequence of the formula shown below:

5'-$N_aN_aN_aN_aN_aN_aN_bN_bN_bN_aN_aN_aN_aN_aN_aN_aN_aN_aN_a$-3'; or,
5'-$N_aN_aN_aN_aN_bN_aN_bN_bN_bN_aN_aN_aN_aN_aN_aN_aN_aN_aN_a$-3';
wherein $N_a$ is a 2'-methoxy-modified nucleotide, and $N_b$ is a 2'-fluoro-modified nucleotide.

In some embodiments, the sense strand is selected from the group consisting of nucleotide sequences of the formulas shown below:

5'-$N_aN_aN_aN_aN_aN_aN_bN_bN_bN_aN_aN_aN_aN_aN_aN_aN_aN_aN_a$-3'; or,
5'-$N_aN_aN_aN_aN_bN_aN_bN_bN_bN_aN_aN_aN_aN_aN_aN_aN_aN_aN_a$-3';
wherein $N_a$ is a 2'-methoxy-modified nucleotide, and $N_b$ is a 2'-fluoro-modified nucleotide.

In some embodiments, the antisense strand comprises or is selected from the group consisting of nucleotide sequences of the formulas shown below:

5'-$N_a$'$N_b$'$N_a$'$N_b$'$N_a$'$N_b$'W'$N_a$'$N_a$'$N_b$'$N_a$'$N_b$'$N_a$'$N_b$'$N_a$'$N_b$'$N_a$'$N_b$'$N_a$'$N_a$'$N_a$'-3'; or,
5'-$N_a$'$N_b$'$N_a$'$N_b$'$N_a$'$N_b$'W'$N_a$'$N_b$'$N_a$'$N_a$'$N_b$'$N_a$'$N_b$'$N_a$'$N_b$'$N_a$'$N_b$'$N_a$'$N_a$'$N_a$'-3';
wherein $N_a$' is a 2'-methoxy-modified nucleotide, and $N_b$' is a 2'-fluoro-modified nucleotide; W' represents a 2'-methoxy-modified nucleotide or a nucleotide comprising a chemical modification of formula (I) or a tautomeric modification thereof.

In some specific embodiments, W' represents a nucleotide comprising a chemical modification of formula (I) or a tautomeric modification thereof.

In some specific embodiments, formula (I) is selected from the group consisting of:

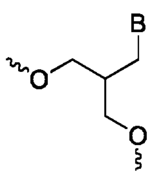 , 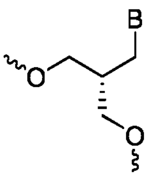 , or 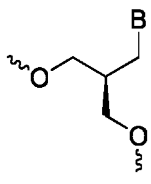 ;

wherein B is selected from the group consisting of guanine, adenine, cytosine, or uracil. In some specific embodiments, B is selected from the base corresponding to position 7 of the 5' region of the antisense strand.

In some specific embodiments, formula (I) is selected from the group consisting of:

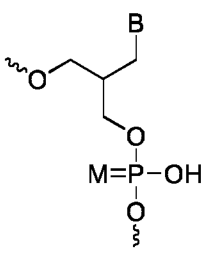 , 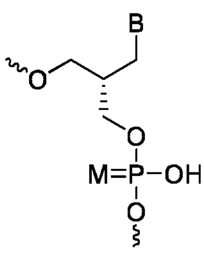 , and 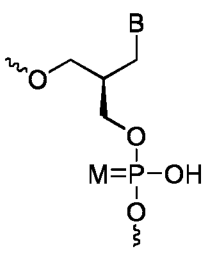 ;

wherein M is O or S; wherein B is selected from the group consisting of guanine, adenine, cytosine, or uracil. In some specific embodiments, B is selected from the base corresponding to position 7 of the 5' region of the antisense strand.

In some specific embodiments, M is S. In some specific embodiments, M is O.

In some specific embodiments, W' represents a 2'-methoxy-modified nucleotide.

In some embodiments, at least one phosphoester group in the sense strand and/or the antisense strand is a phosphoester group with a modification group. The modification group makes the siRNA have increased stability in a biological sample or environment. In some embodiments, the phosphoester group with a modification group is a phosphorothioate group.

In some embodiments, the phosphorothioate group is present in at least one of the positions selected from the group consisting of:

a position between the 1st and 2nd nucleotides of the 5' end of the sense strand;
a position between the 2nd and 3rd nucleotides of the 5' end of the sense strand;
an end of the 1st nucleotide of the 3' end of the sense strand;
a position between the 1st and 2nd nucleotides of the 3' end of the sense strand;
a position between the 2nd and 3rd nucleotides of the 3' end of the sense strand;
a position between the 1st and 2nd nucleotides of the 5' end of the antisense strand;
a position between the 2nd and 3rd nucleotides of the 5' end of the antisense strand;
an end of the 1st nucleotide of the 3' end of the antisense strand;
a position between the 1st and 2nd nucleotides of the 3' end of the antisense strand; and
a position between the 2nd and 3rd nucleotides of the 3' end of the antisense strand.

In some embodiments, the sense strand and/or the antisense strand comprise a plurality of phosphorothioate groups that are present:

between the 1st and 2nd nucleotides of the 5' end of the sense strand; and
between the 2nd and 3rd nucleotides of the 5' end of the sense strand; and
between the 1st and 2nd nucleotides of the 5' end of the antisense strand; and
between the 2nd and 3rd nucleotides of the 5' end of the antisense strand; and
optionally, an end of the 1st nucleotide of the 3' end of the sense strand; and/or optionally,
a position between the 1st and 2nd nucleotides of the 3' end of the sense strand; and/or
optionally, a position between the 2nd and 3rd nucleotides of the 3' end of the sense strand;
and/or optionally, a position between the 1st and 2nd nucleotides of the 3' end of the antisense strand; and/or optionally, a position between the 2nd and 3rd nucleotides of the 3' end of the antisense strand.

In some embodiments, the sense strand is selected from or comprises a nucleotide sequence of the formula shown below:

5'-NmsNmsNmNmNfNmNfNfNfNmNmNmNmNmNmNmNmNmNm-3', or
5'-NmsNmsNmNmNmNmNfNfNfNmNmNmNmNmNmNmNmNmNm-3', or
5'-NmsNmsNmNmNfNmNfNfNfNmNmNmNmNmNmNmNmNmNms-3', or
5'-NmsNmsNmNmNmNmNfNfNfNmNmNmNmNmNmNmNmNmNms-3', or
5'-NmsNmsNmNmNfNmNfNfNfNmNmNmNmNmNmNmNmNmsNm-3', or
5'-NmsNmsNmNmNmNmNfNfNfNmNmNmNmNmNmNmNmNmsNm-3', or
5'-NmsNmsNmNmNfNmNfNfNfNmNmNmNmNmNmNmNmNmsNms-3', or
5'-NmsNmsNmNmNmNmNfNfNfNmNmNmNmNmNmNmNmNmsNms-3',
wherein Nm represents any 2'-methoxy-modified nucleotide, such as 2'-methoxy-modified C, G, U, A, or T; Nf represents any 2'-fluoro-modified nucleotide, such as 2'-fluoro-modified C, G, U, A, or T;
the lowercase letter s indicates that the two nucleotides adjacent to the letter s are linked by a phosphorothioate group, and the lowercase letter s, when being the first at the 3' end,
indicates that the left nucleotide adjacent to the letter s ends in a phosphorothioate group.

In some embodiments, the antisense strand comprises a nucleotide sequence of the formula shown below:

5'-Nm'sNfsNm'NfNm'NfW'Nm'Nm'NfNm'NfNm'NfNm'NfNm'NfNm'sNm'sNm'-3', or
5'-Nm'sNfsNm'NfNm'NfW'Nm'NfNm'Nm'NfNm'NfNm'NfNm'NfNm'sNm'sNm'-3' wherein Nm' represents any 2'-methoxy-modified nucleotide, such as 2'-methoxy-modified C, G, U, A, or T; Nf represents any 2'-fluoro-modified nucleotide, such as 2'-fluoro-modified C, G, U, A, or T;
the lowercase letter s indicates that the two nucleotides adjacent to the letter s are linked by a phosphorothioate group, and the lowercase letter s, when being the first at the 3' end, indicates that the left nucleotide adjacent to the

letter s ends in a phosphorothioate group;
W' represents a 2'-methoxy-modified nucleotide or a nucleotide comprising a chemical modification of formula (I) or a tautomeric modification thereof.

In some embodiments, formula (I) is selected from the group consisting of:

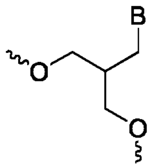, 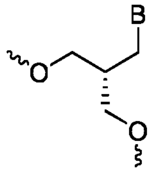, or 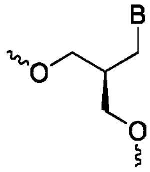;

wherein B is selected from the group consisting of guanine, adenine, cytosine, or uracil. In some embodiments, B is the base corresponding to position 7 of the 5' region of the antisense strand.

In some embodiments, formula (I) is selected from the group consisting of:

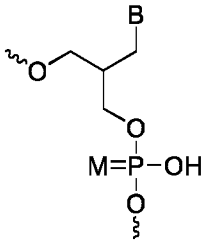, 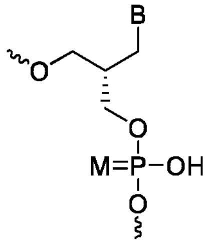, and 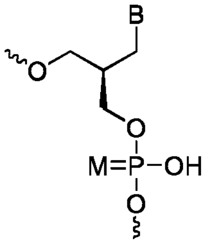;

wherein M is O or S; wherein B is selected from the group consisting of guanine, adenine, cytosine, or uracil. In some specific embodiments, B is the base corresponding to position 7 of the 5' region of the antisense strand.

In some specific embodiments, M is S. In some specific embodiments, M is O.

In some embodiments, the sense strand comprises a sequence differing by no more than 3 nucleotides from any one of the nucleotide sequences of SEQ ID NO: 1 or SEQ ID NO: 2 and comprises at least 15 contiguous nucleotides.

In some embodiments, the sense strand comprises a sequence differing by no more than 3 nucleotides from any one of the nucleotide sequences of SEQ ID NO: 1 or SEQ ID NO: 2 and comprises at least 19 contiguous nucleotides; in some embodiments, the nucleotide sequence differs by no more than 1 nucleotide.

In some embodiments, the antisense strand comprises a sequence differing by no more than 3 nucleotides from any one of the nucleotide sequences of SEQ ID NO: 5 or SEQ ID NO: 6 and comprises at least 21 contiguous nucleotides; in some embodiments, the nucleotide sequence differs by no more than 1 nucleotide; wherein W' represents a 2'-methoxy-modified nucleotide, or a nucleotide comprising a chemical modification of formula (I) or a tautomeric modification thereof.

In some embodiments, the nucleotide sequence of the sense strand is selected from any one of SEQ ID NO: 1 or SEQ ID NO: 2.

In some embodiments, the nucleotide sequence of the antisense strand is selected from any one of SEQ ID NO: 5 or SEQ ID NO: 6, wherein W' represents a 2'-methoxy-modified nucleotide, or a nucleotide comprising a chemical modification of formula (I) or a tautomeric modification thereof.

In some embodiments, formula (I) is selected from the group consisting of:

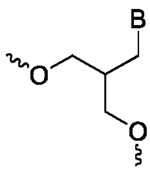, 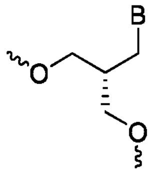, or 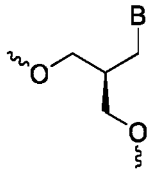;

wherein B is selected from the group consisting of guanine, adenine, cytosine, or uracil. In some embodiments, B is the base corresponding to position 7 of the 5' region of the antisense strand.

In some embodiments, formula (I) is selected from the group consisting of:

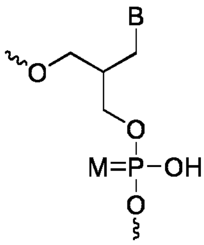, 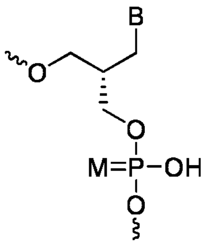, and 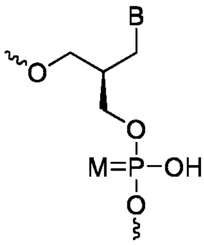;

wherein M is O or S; wherein B is selected from the group consisting of guanine, adenine, cytosine, or uracil; in some specific embodiments, B is the base corresponding to position 7 of the 5' region of the antisense strand.

In some specific embodiments, M is S. In some specific embodiments, M is O.

In some specific embodiments, the sense strand comprises the nucleotide sequence set forth in any one of SEQ ID NO: 39 to SEQ ID NO: 43.

In some specific embodiments, the antisense strand comprises the nucleotide sequence set forth in any one of SEQ ID NO: 44 to SEQ ID NO: 52.

In some specific embodiments, the sense strand is selected from the nucleotide sequence set forth in any one of SEQ ID NO: 39 to SEQ ID NO: 43.

In some specific embodiments, the antisense strand is selected from the nucleotide sequence set forth in any one of SEQ ID NO: 44:to SEQ ID NO: 52.

In some specific embodiments, the siRNA of the present disclosure comprises or is selected from any one of the following:

group 1), a sense strand set forth in SEQ ID NO: 42 and an antisense strand set forth in SEQ ID NO: 49;
group 2), a sense strand set forth in SEQ ID NO: 43 and an antisense strand set forth in SEQ ID NO: 50;
group 3), a sense strand set forth in SEQ ID NO: 42 and an antisense strand set forth in SEQ ID NO: 50;
group 4), a sense strand set forth in SEQ ID NO: 43 and an antisense strand set forth in SEQ ID NO: 49;
group 5), a sense strand set forth in SEQ ID NO: 39 and an antisense strand set forth in SEQ ID NO: 44;
group 6), a sense strand set forth in SEQ ID NO: 40 and an antisense strand set forth in SEQ ID NO: 45;
group 7), a sense strand set forth in SEQ ID NO: 39 and an antisense strand set forth in SEQ ID NO: 46;
group 8), a sense strand set forth in SEQ ID NO: 40 and an antisense strand set forth in SEQ ID NO: 47;
group 9), a sense strand set forth in SEQ ID NO: 41 and an antisense strand set forth in SEQ ID NO: 48.

In some specific embodiments, the siRNA of the present disclosure is selected from any one of the following:

the sense strand comprises the nucleotide sequence set forth in SEQ ID NO: 42 and the antisense strand comprises the nucleotide sequence set forth in SEQ ID NO: 49;
the sense strand comprises the nucleotide sequence set forth in SEQ ID NO: 43 and the antisense strand comprises the nucleotide sequence set forth in SEQ ID NO: 50;
the sense strand comprises the nucleotide sequence set forth in SEQ ID NO: 42 and the antisense strand comprises the nucleotide sequence set forth in SEQ ID NO: 50;
the sense strand comprises the nucleotide sequence set forth in SEQ ID NO: 43 and the antisense strand comprises the nucleotide sequence set forth in SEQ ID NO: 49;
the sense strand comprises the nucleotide sequence set forth in SEQ ID NO: 39 and the antisense strand comprises the nucleotide sequence set forth in SEQ ID NO: 44;
the sense strand comprises the nucleotide sequence set forth in SEQ ID NO: 40 and the antisense strand comprises the nucleotide sequence set forth in SEQ ID NO: 45;
the sense strand comprises the nucleotide sequence set forth in SEQ ID NO: 39 and the antisense strand comprises the nucleotide sequence set forth in SEQ ID NO: 46;
the sense strand comprises the nucleotide sequence set forth in SEQ ID NO: 40 and the antisense strand comprises the nucleotide sequence set forth in SEQ ID NO: 47;
the sense strand comprises the nucleotide sequence set forth in SEQ ID NO: 41 and the antisense strand comprises the nucleotide sequence set forth in SEQ ID NO: 48.

In some specific embodiments, the siRNA of the present disclosure is selected from any one of the following:

the sense strand is the nucleotide sequence set forth in SEQ ID NO: 42 and the antisense strand is the nucleotide

sequence set forth in SEQ ID NO: 49;
the sense strand is the nucleotide sequence set forth in SEQ ID NO: 43 and the antisense strand is the nucleotide sequence set forth in SEQ ID NO: 50;
the sense strand is the nucleotide sequence set forth in SEQ ID NO: 42 and the antisense strand is the nucleotide sequence set forth in SEQ ID NO: 50;
the sense strand is the nucleotide sequence set forth in SEQ ID NO: 43 and the antisense strand is the nucleotide sequence set forth in SEQ ID NO: 49;
the sense strand is the nucleotide sequence set forth in SEQ ID NO: 39 and the antisense strand is the nucleotide sequence set forth in SEQ ID NO: 44;
the sense strand is the nucleotide sequence set forth in SEQ ID NO: 40 and the antisense strand is the nucleotide sequence set forth in SEQ ID NO: 45;
the sense strand is the nucleotide sequence set forth in SEQ ID NO: 39 and the antisense strand is the nucleotide sequence set forth in SEQ ID NO: 46;
the sense strand is the nucleotide sequence set forth in SEQ ID NO: 40 and the antisense strand is the nucleotide sequence set forth in SEQ ID NO: 47;
the sense strand is the nucleotide sequence set forth in SEQ ID NO: 41 and the antisense strand is the nucleotide sequence set forth in SEQ ID NO: 48.

In the present disclosure, according to the 5'-3' direction,

SEQ ID NO: 39 is GmsCmsUmCmCfUmUfAfUfUmGmUmUmAmUmAmCmGmAm;
SEQ ID NO: 40 is AmsCmsAmCmCfAmCfAfUfCmAmAmCmAmUmAmAmUmAm;
SEQ ID NO: 41 is AmsCmsAmCmCmAmCfAfUfCmAmAmCmAmUmAmAmUmsAm;
SEQ ID NO: 42 is GmsCmsUmCmCmUmUfAfUfUmGmUmUmAmUmAmCmGmsAm;
SEQ ID NO: 43 is GmsCmsUmCmCmUmUfAfUfUmGmUmUmAmUmAmCmGmAm;
SEQ ID NO: 44 is UmsCfsGmUfAmUfAmAmCfAmAmUfAmAfGmGfAmGfCmsUmsGm;
SEQ ID NO: 45 is Ums AfsUmU fAmU fGmUmU fGmAmU fGmU fGmGfUmGfUmsCms Am;
SEQ ID NO: 46 is UmsCfsGmUfAmUfAmAfCmAfAmUfAmAfGmGfAmGfCmsUfsGm;
SEQ ID NO: 47 is UmsAfsUmUfAmUfGmUfUmGfAmUfGmUfGmGfUmGfUmsCfsAm;
SEQ ID NO: 48 is UmsAfsUmUfAmUfGmUmUmGfAmUfGmUfGmGfUmGfUmsCmsAm;
SEQ ID NO: 49 is UmsCfsGmUfAmUf(-)hmpNA(A)AmCmAfAmUfAmAfGmGfAmGfCmsUmsGm;
SEQ ID NO: 50 is UmsCfsGmUfAmUfAmAmCmAfAmUfAmAfGmGfAmGfCmsUmsGm;
wherein, Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside;
s indicates that the two nucleotides adjacent to the letter s are linked by a phosphorothioate diester group;
(-)hmpNA(A) represents

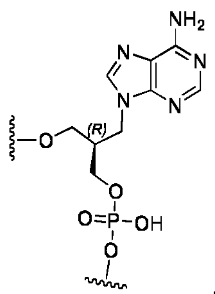
.

The present disclosure also provides an siRNA conjugate, which comprises any siRNA described above and a targeting ligand linked to the siRNA.

In some embodiments, the siRNA and the targeting ligand are linked covalently or non-covalently.

In some embodiments, the targeting ligand targets the liver; in some embodiments, the targeting ligand binds to asialoglycoprotein receptor (ASGPR); in some embodiments, the targeting ligand comprises a galactose cluster or a galactose derivative cluster, wherein the galactose derivative is selected from the group consisting of N-acetyl-galactosamine, N-trifluoroacetylgalactosamine, N-propionylgalactosamine, N-n-butyrylgalactosamine, or N-isobutyrylgalactosamine.

In some embodiments, the targeting ligand is linked to the 3' end of the sense strand of the siRNA.

In some embodiments, the targeting ligand is linked to an end of the siRNA by a phosphoester group, a phosphorothioate group, or a phosphonic acid group; in some embodiments, the targeting ligand is linked to an end of the siRNA by a phosphodiester group.

In some embodiments, the targeting ligand is indirectly linked to an end of the siRNA by a phosphoester group, a phosphorothioate group, or a phosphonic acid group; in some embodiments, the targeting ligand is indirectly linked to an end of the siRNA by a phosphodiester group.

In some embodiments, the targeting ligand is directly linked to an end of the siRNA by a phosphoester group, a phosphorothioate group, or a phosphonic acid group; in some embodiments, the targeting ligand is directly linked to an end of the siRNA by a phosphodiester group.

In some embodiments, the targeting ligand is directly linked to an end of the sense strand of the siRNA by a phosphoester group or a phosphorothioate group; in some embodiments, the targeting ligand is linked to an end of the sense strand of the siRNA by a phosphodiester group.

In some embodiments, the targeting ligand is directly linked to the 3' end of the sense strand of the siRNA by a phosphoester group or a phosphorothioate group; in some embodiments, the targeting ligand is directly linked to the 3' end of the sense strand of the siRNA by a phosphodiester group.

In some embodiments, to promote entry of the siRNA into a cell, a lipophilic group such as cholesterol can be introduced into an end of the sense strand of the siRNA, and the lipophilic group may be covalently bonded to a small interfering nucleic acid; for example, cholesterol, lipoprotein, vitamin E, etc., are introduced to the end to facilitate going through the cell membrane consisting of a lipid bilayer and interacting with the mRNA in the cell. Meanwhile, the siRNA can also be modified by non-covalent bonding, for example, bonding to a phospholipid molecule, a polypeptide, a cationic polymer, etc, by a hydrophobic bond or an ionic bond to increase stability and biological activity.

In some specific embodiments, the targeting ligand is selected from the group consisting of the following structure or a pharmaceutically acceptable salt thereof:

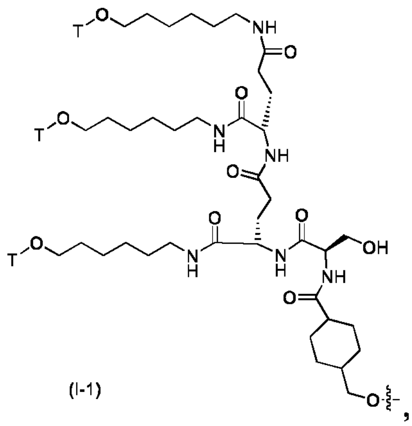

(I-1)

,

wherein T is a targeting moiety.

In some specific embodiments, the targeting ligand is selected from the group consisting of the following structure or a pharmaceutically acceptable salt thereof:

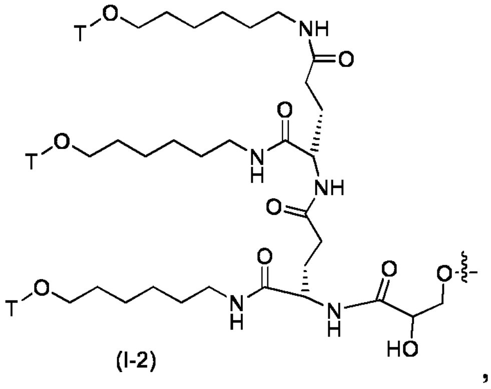

(I-2)

,

wherein T is a targeting moiety.

In some specific embodiments, the targeting ligand has the structure below:

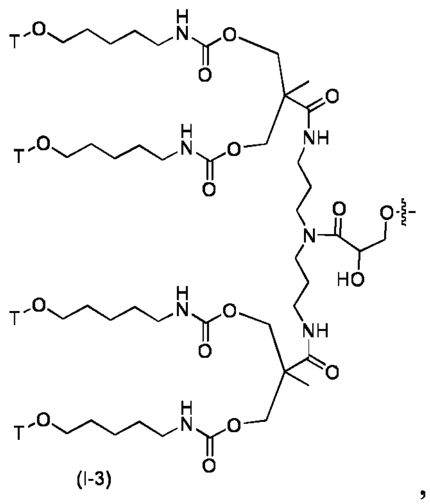

(I-3)

,

wherein T is a targeting moiety.

In some specific embodiments, the targeting ligand is selected from the group consisting of the following structure or a pharmaceutically acceptable salt thereof:

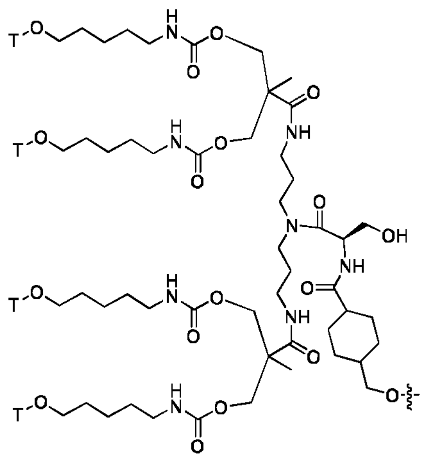

(I-4)

,

wherein T is a targeting moiety.

In some embodiments, the targeting moiety of the targeting ligand consists of one or more targeting groups or targeting moieties, and the targeting ligand assists in directing the delivery of the therapeutic agent linked thereto to the desired target location. In some cases, the targeting moiety can bind to a cell or cellular receptor and initiate endocytosis to promote entry of the therapeutic agent into the cell. The targeting moiety can comprise a compound with affinity for a cellular receptor or a cell surface molecule or an antibody. Various targeting ligands comprising targeting moieties can be linked to therapeutic agents and other compounds to target the agents at cells and specific cellular receptors. In some embodiments, the types of the targeting moieties include carbohydrates, cholesterol and cholesterol groups, or steroids. Targeting moieties that can bind to cellular receptors include saccharides such as galactose and galactose derivatives (e.g., N-acetyl-galactosamine, N-trifluoroacetylgalactosamine, N-propionylgalactosamine, N-n-butyrylgalactosamine, N-isobutyrylgalactosamine, mannose, and mannose derivatives). Targeting moieties that bind to asialoglycoprotein receptors (ASGPRs) are known to be particularly used for directing the delivery of oligomeric compounds to the liver. Asialoglycoprotein receptors are extensively expressed on liver cells (hepatocytes). The targeting moieties of cellular receptors targeting ASCPR include galactose and galactose derivatives. Specifically, clusters of galactose derivatives, including clusters consisting of 2, 3, 4, or more than 4 N-acetyl-galactosamines (GalNAc or NAG), can promote the uptake of certain compounds in hepatocytes. The GalNAc cluster coupled to the oligomeric compound is used for directing the composition to the liver where the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into the interior of the cell.

In some embodiments, the targeting ligand can comprise 2, 3, 4, or more than 4 targeting moieties. In some embodiments, the targeting ligand disclosed herein can comprise 1, 2, 3, 4, or more than 4 targeting moieties linked to the branching group by $L_2$.

In some embodiments, each targeting moiety comprises a galactosamine derivative, which is N-acetyl-galactosamine. Other sugars that can be used as targeting moieties and that have affinity for asialoglycoprotein receptors can be selected from the group consisting of galactose, galactosamine, N-formyl-galactosamine, N-acetyl-galactosamine, N-propionyl-galactosamine, N-n-butyryl-galactosamine, N-isobutyrylgalactosamine, etc.

In some embodiments, the targeting ligand in the present disclosure comprises N-acetylgalactosamine as a targeting moiety,

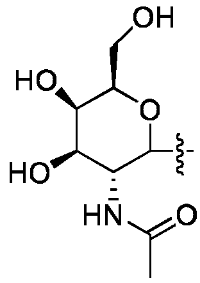
(H-2)

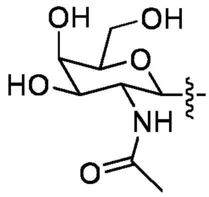
(H-2)

.

In some embodiments, the targeting ligand comprises three terminal galactosamines or galactosamine derivatives (such as N-acetyl-galactosamine), each of which has affinity for asialoglycoprotein receptors. In some embodiments, the targeting ligand comprises three terminal N-acetyl-galactosamines (GalNAc or NAG) as targeting moieties.

In some embodiments, the targeting ligand comprises four terminal galactosamines or galactosamine derivatives (such as N-acetyl-galactosamine), each of which has affinity for asialoglycoprotein receptors. In some embodiments, the targeting ligand comprises four terminal N-acetyl-galactosamines (GalNAc or NAG) as targeting moieties.

The terms commonly used in the art when referring to three terminal N-acetyl-galactosamines include tri-antennary, tri-valent, and trimer.

The terms commonly used in the art when referring to four terminal N-acetyl-galactosamines include tetra-antennary, tetra-valent, and tetramer.

In some specific embodiments, the targeting ligand provided by the present disclosure is selected from the group consisting of the following structure or a pharmaceutically acceptable salt thereof,

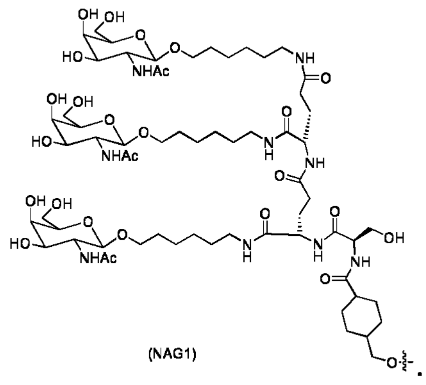
(NAG1)

In some specific embodiments, the targeting ligand provided by the present disclosure is selected from the group consisting of the following structure or a pharmaceutically acceptable salt thereof,

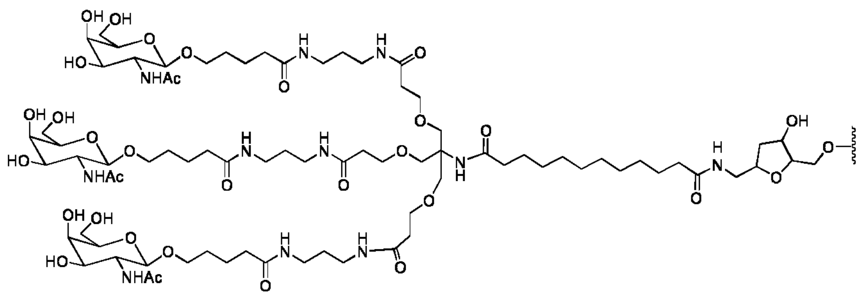

or

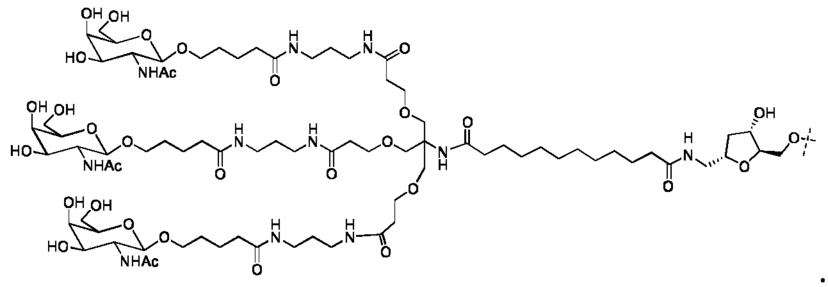

.

In some embodiments, the N-acetyl-galactosamine moiety in the above targeting ligand can be replaced with N-trifluoroacetylgalactosamine, N-propionylgalactosamine, N-n-butyrylgalactosamine, or N-isobutyrylgalactosamine.

In some specific embodiments, the sense strand of the siRNA conjugate comprises: any one of SEQ ID NO: 53 to SEQ ID NO: 57.

In some specific embodiments, the antisense strand of the siRNA conjugate comprises any one of SEQ ID NO: 49 to SEQ ID NO: 52 and SEQ ID NO: 58 to SEQ ID NO: 60. In some specific embodiments, the siRNA conjugate comprises or is selected from any one of the following:

group 1), a sense strand set forth in SEQ ID NO: 56 and an antisense strand set forth in SEQ ID NO: 49;
group 2), a sense strand set forth in SEQ ID NO: 57 and an antisense strand set forth in SEQ ID NO: 50;
group 3), a sense strand set forth in SEQ ID NO: 56 and an antisense strand set forth in SEQ ID NO: 50;
group 4), a sense strand set forth in SEQ ID NO: 57 and an antisense strand set forth in SEQ ID NO: 49;
group 5), a sense strand set forth in SEQ ID NO: 53 and an antisense strand set forth in SEQ ID NO: 46;
group 6), a sense strand set forth in SEQ ID NO: 54 and an antisense strand set forth in SEQ ID NO: 47;
group 7), a sense strand set forth in SEQ ID NO: 55 and an antisense strand set forth in SEQ ID NO: 48.

In some specific embodiments, the siRNA of the present disclosure is selected from any one of the following:

the sense strand comprises the nucleotide sequence set forth in SEQ ID NO: 56 and the antisense strand comprises the nucleotide sequence set forth in SEQ ID NO: 49;
the sense strand comprises the nucleotide sequence set forth in SEQ ID NO: 57 and the antisense strand comprises the nucleotide sequence set forth in SEQ ID NO: 50;
the sense strand comprises the nucleotide sequence set forth in SEQ ID NO: 56 and the antisense strand comprises the nucleotide sequence set forth in SEQ ID NO: 50;
the sense strand comprises the nucleotide sequence set forth in SEQ ID NO: 57 and the antisense strand comprises the nucleotide sequence set forth in SEQ ID NO: 49;
the sense strand comprises the nucleotide sequence set forth in SEQ ID NO: 53 and the antisense strand comprises the nucleotide sequence set forth in SEQ ID NO: 46;
the sense strand comprises the nucleotide sequence set forth in SEQ ID NO: 54 and the antisense strand comprises the nucleotide sequence set forth in SEQ ID NO: 47;
the sense strand comprises the nucleotide sequence set forth in SEQ ID NO: 55 and the antisense strand comprises the nucleotide sequence set forth in SEQ ID NO: 48.

In some specific embodiments, the siRNA of the present disclosure is selected from any one of the following:

the sense strand comprises the nucleotide sequence set forth in SEQ ID NO: 56 and the antisense strand comprises the nucleotide sequence set forth in SEQ ID NO: 49;
the sense strand comprises the nucleotide sequence set forth in SEQ ID NO: 57 and the antisense strand comprises the nucleotide sequence set forth in SEQ ID NO: 50;
the sense strand comprises the nucleotide sequence set forth in SEQ ID NO: 56 and the antisense strand comprises the nucleotide sequence set forth in SEQ ID NO: 50;
the sense strand comprises the nucleotide sequence set forth in SEQ ID NO: 57 and the antisense strand comprises the nucleotide sequence set forth in SEQ ID NO: 49;
the sense strand comprises the nucleotide sequence set forth in SEQ ID NO: 53 and the antisense strand comprises the nucleotide sequence set forth in SEQ ID NO: 46;
the sense strand comprises the nucleotide sequence set forth in SEQ ID NO: 54 and the antisense strand comprises the nucleotide sequence set forth in SEQ ID NO: 47;
the sense strand comprises the nucleotide sequence set forth in SEQ ID NO: 55 and the antisense strand comprises the nucleotide sequence set forth in SEQ ID NO: 48.

In the present disclosure, according to the 5'-3' direction,

SEQ ID NO: 53 is GmsCmsUmCmCfUmUfAfUfUmGmUmUmAmUmAmCmGmAm-NAG0052';
SEQ ID NO: 54 is AmsCmsAmCmCfAmCfAfUfCmAmAmCmAmUmAmAmUmAm-NAG0052';
SEQ ID NO: 55 is AmsCmsAmCmCmAmCfAfUfCmAmAmCmAmUmAmAmUmsAm-NAG0052';
SEQ ID NO: 56 is GmsCmsUmCmCmUmUfAfUfUmGmUmUmAmUmAmCmGmsAm-NAG0052';
SEQ ID NO: 57 is GmsCmsUmCmCmUmUfAfUfUmGmUmUmAmUmAmCmGmAm-NAG0052';
SEQ ID NO: 46 is UmsCfsGmUfAmUfAmAfCmAfAmUfAmAfGmGfAmGfCmsUfsGm;
SEQ ID NO: 47 is UmsAfsUmUfAmUfGmUfUmGfAmUfGmUfGmGfUmGfUmsCfsAm;
SEQ ID NO: 48 is UmsAfsUmUfAmUfGmUmUmGfAmUfGmUfGmGfUmGfUmsCmsAm;
SEQ ID NO: 49 is UmsCfsGmUfAmUf(-)hmpNA(A)AmCmAfAmUfAmAfGmGfAmGfCmsUmsGm;
SEQ ID NO: 50 is UmsCfsGmUfAmUfAmAmCmAfAmUfAmAfGmGfAmGfCmsUmsGm;
wherein, Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside;
s indicates that the two nucleotides adjacent to the letter s are linked by a phosphorothioate diester group;
(-)hmpNA(A) represents

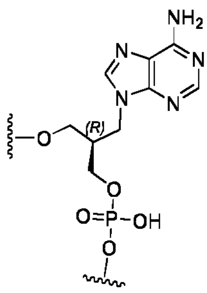

,

and NAG0052' represents

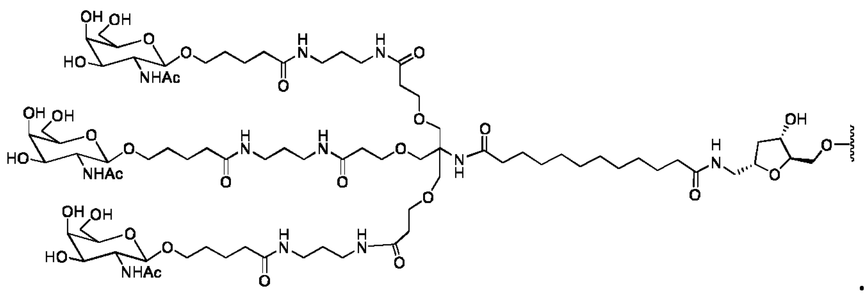

.

In some embodiments, the siRNA conjugate is of the following structures or pharmaceutically acceptable salts

thereof:

Sense strand

Antisense strand

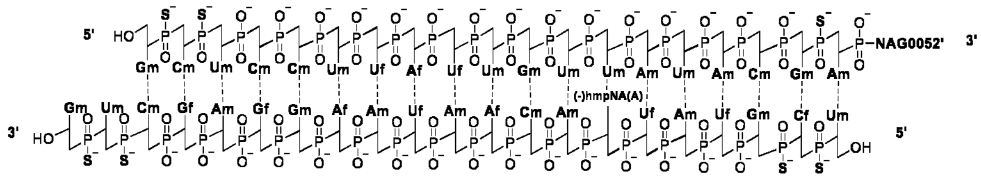

Sense strand

Antisense strand

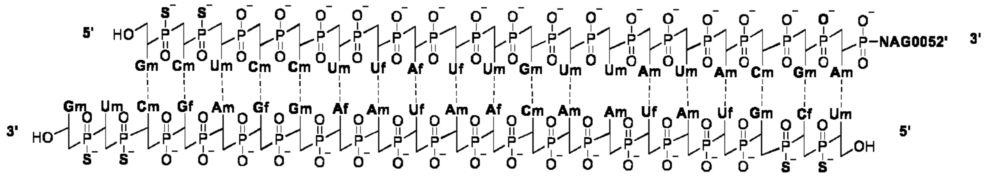

Sense strand

Antisense strand

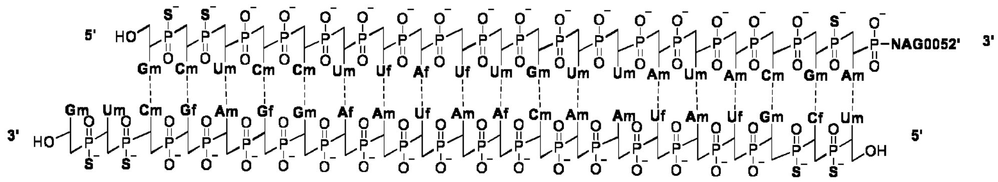

Sense strand

Antisense strand

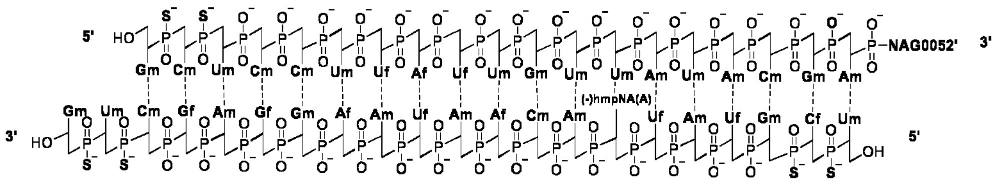

Sense strand

Antisense strand

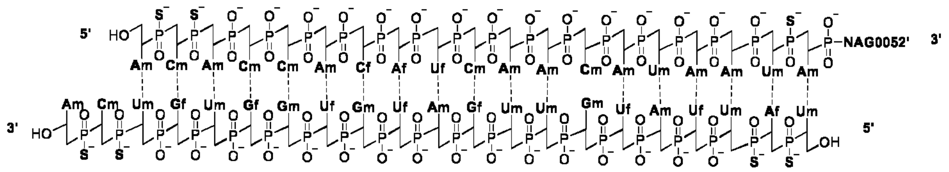

wherein,

Af = adenine 2'-F ribonucleoside;
Cf = cytosine 2'-F ribonucleoside;
Uf = uracil 2'-F ribonucleoside;
Gf = guanine 2'-F ribonucleoside;
Am = adenine 2'-OMe ribonucleoside; Cm = cytosine 2'-OMe ribonucleoside; Gm = guanine 2'-OMe ribonucleoside;
Um = uracil 2'-OMe ribonucleoside.

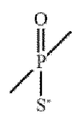

represents a phosphorothioate group,

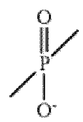

represents a phosphodiester group, NAG0052' represents

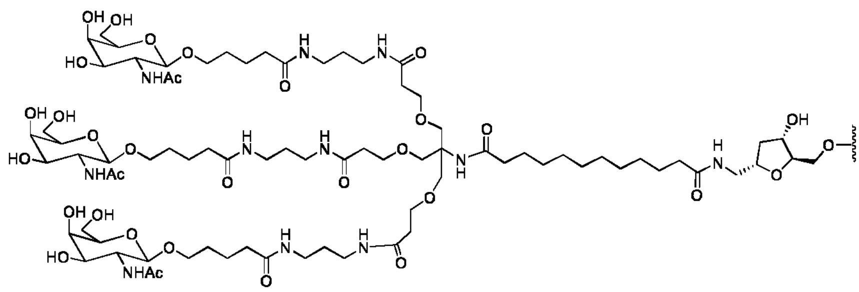

,

and (-)hmpNA(A) represents

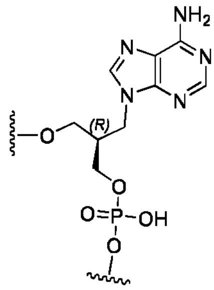

.

In some embodiments, the pharmaceutically acceptable salt can be a conventional salt in the art, including but not limited to sodium salts, potassium salts, ammonium salts, amine salts, etc.

In some embodiments, the siRNA conjugate is selected from the group consisting of TJR100396, TJR100436, TJR100397, TJR100437, TJR100391, TJR100392, and TJR100395.

In some embodiments, the siRNA conjugate is selected from TJR100396, which is of the following structure:

Sense strand

Antisense strand

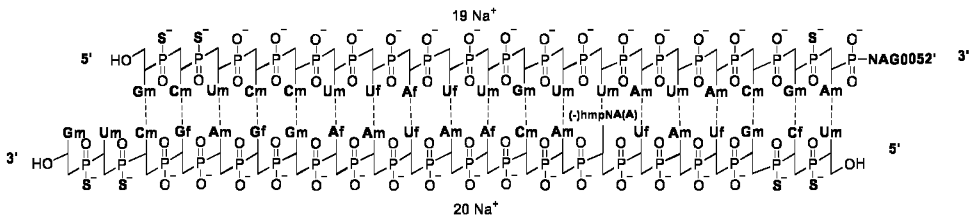

In some embodiments, the siRNA conjugate is selected from TJR100436, which is of the following structure:

Sense strand

Antisense strand

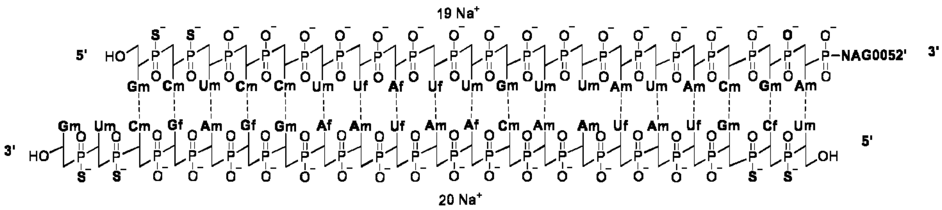

In some embodiments, the siRNA conjugate is selected from TJR100397, which is of the following structure:

Sense strand

Antisense strand

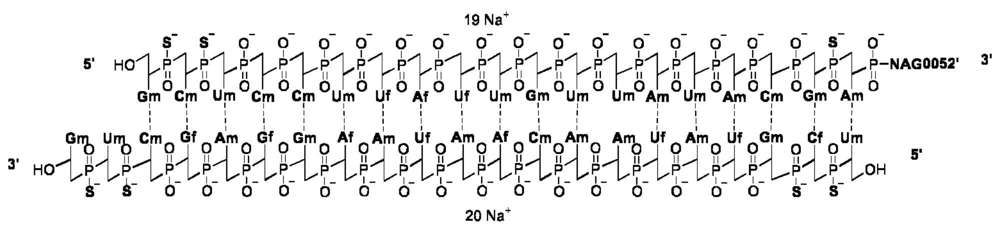

In some embodiments, the siRNA conjugate is selected from TJR100437, which is the following structure:

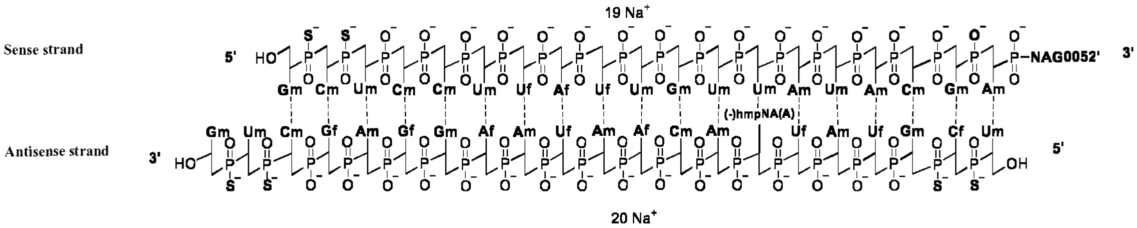

In some embodiments, the siRNA conjugate is selected from TJR100395, which is of the following structure:

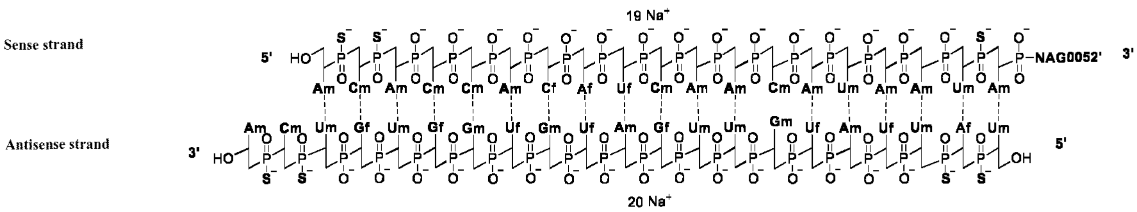

Af = adenine 2'-F ribonucleoside;
Cf = cytosine 2'-F ribonucleoside;
Uf = uracil 2'-F ribonucleoside;
Gf = guanine 2'-F ribonucleoside;
Am = adenine 2'-OMe ribonucleoside;
Cm = cytosine 2'-OMe ribonucleoside;
Gm = guanine 2'-OMe ribonucleoside;
Um = uracil 2'-OMe ribonucleoside.

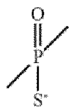

represents a phosphorothioate group,

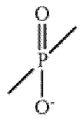

represents a phosphodiester group, NAG0052' represents

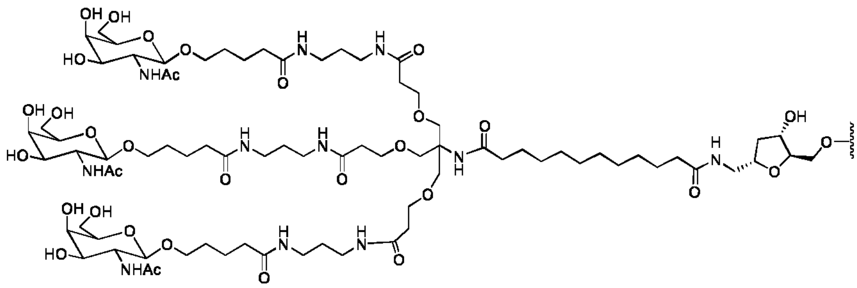

,

and (-)hmpNA(A) represents

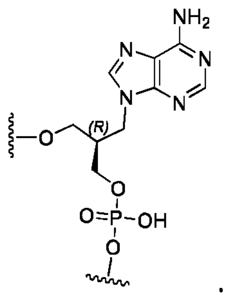 .

Another aspect of the present disclosure provides a composition, which comprises the conjugate described above, and one or more pharmaceutically acceptable excipients, such as a carrier, a vehicle, a diluent, and/or a delivery polymer.
In the present disclosure, various delivery systems are known and can be used for the siRNA or siRNA conjugate of the present disclosure, e.g., encapsulation in liposomes, microparticles, microcapsules, recombinant cells capable of expressing the compound, receptor-mediated endocytosis, and construction of a nucleic acid as part of a retroviral vector or other vectors.
Another aspect of the present disclosure provides use of the conjugate or the composition comprising the conjugate described above in the preparation of a medicament for treating a disease in a subject; in some embodiments, the disease is selected from a hepatic disease. Another aspect of the present disclosure provides a method for treating a disease in a subject, which comprises administering to the subject the conjugate or the composition described above.
Another aspect of the present disclosure provides a method for inhibiting mRNA expression in a subject, which comprises administering to the subject the conjugate or the composition described above.
Another aspect of the present disclosure provides a method for delivering an expression-inhibiting oligomeric compound to the liver *in vivo,* which comprises administering to a subject the conjugate or the composition described above.
The conjugate, the composition, and the methods disclosed herein can reduce the target mRNA level in a cell, a cell population, a cell population, a tissue, or an object, which comprises administering to the object a therapeutically effective amount of the expression-inhibiting oligomer described herein. The expression-inhibiting oligomer is linked to a targeting ligand, thereby inhibiting target mRNA expression in the object.
In some embodiments, the object has been previously identified as having pathogenic upregulation of the target gene in the targeted cell or tissue.
The subject described in the present disclosure refers to an object having a disease or condition that would benefit from reduction or inhibition of target mRNA expression. Delivery can be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In optional embodiments, the pharmaceutical composition provided by the present disclosure can be administered by injection, for example, intravenous, intramuscular, intradermal, subcutaneous, intraduodenal, or intraperitoneal injection.
In optional embodiments, after the targeting ligand and an expression-inhibiting polymer compound are linked to form a conjugate, the conjugate can be packaged in a kit.
The present disclosure also provides a pharmaceutical composition, which comprises the siRNA or the siRNA conjugate of the present disclosure.
In some embodiments, the pharmaceutical composition can further comprise a pharmaceutically acceptable auxiliary material and/or adjuvant; the auxiliary material can be one or more of various formulations or compounds conventionally used in the art. For example, the pharmaceutically acceptable auxiliary material can include at least one of a pH buffer, a protective agent, and an osmotic pressure regulator.
In some embodiments, when the siRNA, the siRNA conjugate, or the pharmaceutical composition described above is in contact with a target gene-expressing cell, the siRNA conjugate or the pharmaceutical composition described above inhibits the target gene expression by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.
In some embodiments, when the siRNA, the siRNA conjugate, or the pharmaceutical composition described above is in contact with a target gene-expressing cell, the siRNA conjugate or the pharmaceutical composition described

above results in a percent remaining expression of the target gene mRNA of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

The present disclosure also provides a cell, which comprises the siRNA or the siRNA conjugate of the present disclosure.

The present disclosure also provides a kit, which comprises the siRNA or the siRNA conjugate of the present disclosure.

The present disclosure also provides a method for silencing a target gene or mRNA of a target gene in a cell, which comprises the step of introducing into the cell the siRNA, the siRNA conjugate, and/or the pharmaceutical composition according to the present disclosure.

The present disclosure also provides a method for silencing a target gene or mRNA of a target gene in a cell *in vivo* or *in vitro,* which comprises the step of introducing into the cell the siRNA, the siRNA conjugate, and/or the pharmaceutical composition according to the present disclosure.

The present disclosure also provides a method for inhibiting a target gene or the expression of mRNA of a target gene, which comprises administering to a subject in need thereof an effective amount or an effective dose of the siRNA, the siRNA conjugate, and/or the pharmaceutical composition according to the present disclosure.

In some embodiments, administration is carried out through routes of administration including intramuscular, intrabronchial, intrapleural, intraperitoneal, intraarterial, lymphatic, intravenous, subcutaneous, cerebrospinal, or combinations thereof.

In some embodiments, the effective amount or the effective dose of the siRNA, the siRNA conjugate, and/or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

In some embodiments, the target gene is LPA.

The present disclosure provides the siRNA and/or the pharmaceutical composition and/or the siRNA conjugate described above for use in treating and/or preventing a disease associated with elevated lipoprotein(a) and/or apolipoprotein(a) levels in a subject or any other related conditions, pathology, or syndromes; in some embodiments, the disease associated with elevated lipoprotein(a) and/or apolipoprotein(a) levels is selected from a cardiovascular disease; in some embodiments, the cardiovascular disease is selected from the group consisting of

ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides the siRNA and/or the pharmaceutical composition and/or the siRNA conjugate described above for use in treating and/or preventing a disease, and the disease is selected from a cardiovascular disease; in some embodiments, the cardiovascular disease is selected from the group consisting of ischemic stroke,

atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides the siRNA and/or the pharmaceutical composition and/or the siRNA conjugate described above for use in reducing lipoprotein(a) and/or apolipoprotein(a) levels.

The present disclosure provides use of the siRNA and/or the pharmaceutical composition and/or the siRNA conjugate described above in the preparation of a medicament for inhibiting the expression of LPA.

The present disclosure provides use of the siRNA and/or the pharmaceutical composition and/or the siRNA conjugate described above in the preparation of a medicament for treating and/or preventing a disease associated with elevated lipoprotein(a) and/or apolipoprotein(a) levels in a subject; in some embodiments, the disease associated with elevated lipoprotein(a) and/or apolipoprotein(a) levels is selected from a cardiovascular disease; in some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides use of the siRNA and/or the pharmaceutical composition and/or the siRNA conjugate described above in the preparation of a medicament for treating and/or preventing a disease, and the disease is selected from a cardiovascular disease; in some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides use of the siRNA and/or the pharmaceutical composition and/or the siRNA conjugate described above in the preparation of a medicament for reducing lipoprotein(a) and/or apolipoprotein(a) levels.

The present disclosure provides a method for inhibiting the expression of LPA, which comprises administering to a subject an effective amount or an effective dose of the siRNA and/or the pharmaceutical composition and/or the siRNA conjugate described above.

The present disclosure provides a method for treating and/or preventing a disease associated with elevated lipoprotein(a) and/or apolipoprotein(a) levels in a subject, which comprises administering to the subject an effective amount or an effective dose of the siRNA and/or the pharmaceutical composition and/or the siRNA conjugate described above; in some embodiments, the disease associated with elevated lipoprotein(a) and/or apolipoprotein(a) levels is selected from a cardiovascular disease; in some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides a method for treating and/or preventing a disease, which comprises administering to a subject an effective amount or an effective dose of the siRNA and/or the pharmaceutical composition and/or the siRNA conjugate described above, and the disease is selected from a cardiovascular disease; in some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides a method for reducing lipoprotein(a) and/or apolipoprotein(a) levels, which comprises administering to a subject an effective amount or an effective dose of the siRNA and/or the pharmaceutical composition and/or the siRNA conjugate described above.

The present disclosure provides a method for delivering an siRNA that inhibits the expression and/or replication of LPA to the liver *in vivo,* which comprises administering to a subject the siRNA and/or the pharmaceutical composition and/or the siRNA conjugate described above.

The present disclosure also provides an siRNA or an siRNA conjugate, wherein one or more bases U, e.g., 1, 2, 3, 3, 5, 6, 7, 8, 9, or 10 bases U, of any siRNA or siRNA conjugate of the present disclosure are replaced with bases T.

The pharmaceutically acceptable salts of the compounds described herein are selected from the group consisting of inorganic salts or organic salts. The compounds described herein can react with acidic or basic substances to form corresponding salts.

In another aspect, where the configuration is not specified, the compounds of the present disclosure can be present in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms can be present in substituents such as an alkyl group. All such isomers and mixtures thereof are included within the scope of the present disclosure.

In addition, where the configuration is not specified, the compounds and intermediates of the present disclosure can also be present in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol, imine-enamine, and lactam-lactim isomerization. An example of a lactam-lactim

equilibrium is present between A and B as shown below.

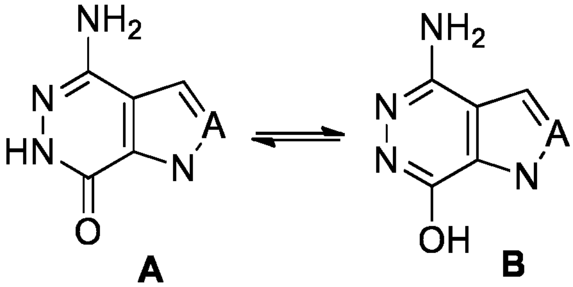

A B

All compounds in the present disclosure can be drawn as form A or form B. All tautomeric forms are within the scope of the present disclosure. The names of the compounds do not exclude any tautomers.

The compounds of the present disclosure can be asymmetric; for example, the compounds have one or more stereoisomers. Unless otherwise specified, all stereoisomers include, for example, enantiomers and diastereomers. The compounds of the present disclosure containing asymmetric carbon atoms can be separated in optically active pure form or in racemic form. The optically active pure form can be isolated from a racemic mixture or synthesized using chiral starting materials or chiral reagents.

Optically active (R)- and (S)-enantiomers and D- and L-isomers can be prepared by chiral synthesis, chiral reagents, or other conventional techniques. If one enantiomer of a certain compound of the present disclosure is desired, it can be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by isolating. Furthermore, separation of enantiomers and diastereomers is generally accomplished by chromatography using a chiral stationary phase, optionally in combination with chemical derivatization (e.g., carbamate formation from amines). The present disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is at least 1000 times greater than the natural abundance, at least 2000 times greater than the natural abundance, at least 3000 times greater than the natural abundance, at least 4000 times greater than the natural abundance, at least 5000 times greater than the natural abundance, at least 6000 times greater than the natural abundance, or higher times greater than the natural abundance. The present disclosure also comprises various deuterated forms of the compounds of formula I and formula II. Each available hydrogen atom connected to a carbon atom may be independently replaced by a deuterium atom. Those skilled in the art are able to synthesize the deuterated forms of the compounds of formula I and formula II with reference to the relevant literature. Commercially available deuterated starting materials can be used in preparing the deuterated forms of the compounds of formula I and formula II, or they can be synthesized using conventional techniques with deuterated reagents, including but not limited to deuterated borane, trideuterated borane in tetrahydrofuran, deuterated lithium aluminum hydride, deuterated iodoethane, deuterated iodomethane, and the like.

Where the configurations are not specified, in the chemical structures of the compounds of the present disclosure, a bond "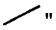" indicates an unspecified configuration; that is, if chiral isomers exist in the chemical structures, the bond "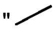" may be "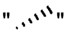" or "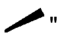" or includes both the configurations "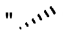" and "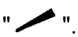". Although all of the above structural formulae are drawn as certain isomeric forms for the sake of simplicity, the present disclosure may include all isomers, such as tautomers, rotamers, geometric isomers, diastereomers, racemates, and enantiomers. In the chemical structure of the compound of the present disclosure, a bond "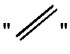" does not specify a configuration; that is, the configuration for the bond "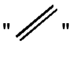" can be an E configuration or a Z configuration, or includes both the E configuration and the

Z configuration.

**Terms and Definitions**

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. Unless otherwise specified, in the context of the present disclosure, the terms "apolipoprotein(a) gene", apo(a) gene, and LPA are used interchangeably in the present disclosure. LPAs include, but are not limited to, human LPA, cynomolgus monkey LPA, mouse LPA, and rat LPA, the amino acids and complete coding sequences and mRNA sequences of which are readily accessible using publicly available databases, e.g., GenBank, UniProt, OMIM, and the Macaca genome project website.

The term "target sequence" refers to a contiguous portion of the nucleotide sequence of an mRNA molecule formed during the transcription of LPA, including mRNA that is a product of RNA processing of a primary transcription product. The targeted portion in the target sequence should be long enough to serve as a substrate for iRNA-directed cleavage. In one embodiment, the target sequence is within the protein encoding region of LPA.

As used herein, in the context of RNA-mediated gene silencing, the sense strand (also referred to as SS or SS strand) refers to a strand that comprises a sequence that is identical or substantially identical to a target mRNA sequence; the antisense strand (also referred to as AS or AS strand) refers to a strand having a sequence complementary to a target mRNA sequence.

In the context describing the siRNA sense strand described herein, the term "a sequence differing by no more than 3 nucleotides from any one of the nucleotide sequences of SEQ ID NO: 1 and SEQ ID NO: 2 and comprises at least 15 contiguous nucleotides" is intended to mean that the siRNA sense strand described herein comprises at least 15 contiguous nucleotides of any one of the sense strands in SEQ ID NO: 1 and SEQ ID NO: 2 or a sequence differing by no more than 3 nucleotides (optionally, by no more than 2 nucleotides; optionally, by 1 nucleotide) from at least 15 contiguous nucleotides of any one of the sense strands of SEQ ID NO: 1 and SEQ ID NO: 2. Optionally, the siRNA sense strand described herein comprises at least 16 contiguous nucleotides of any one of the sense strands of SEQ ID NO: 1 and SEQ ID NO: 2 or a sequence differing by no more than 3 nucleotides (optionally, by no more than 2 nucleotides; optionally, by 1 nucleotide) from at least 16 contiguous nucleotides of any one of the sense strands of SEQ ID NO: 1 and SEQ ID NO: 2.

In the context describing the siRNA antisense strand described herein, the term "a sequence differing by no more than 3 nucleotides from any one of the antisense strands of SEQ ID NO: 3 and SEQ ID NO: 4 and comprises at least 15 contiguous nucleotides" is intended to mean that the siRNA antisense strand described herein comprises at least 15 contiguous nucleotides of any one of the antisense strands of SEQ ID NO: 3 and SEQ ID NO: 4 or a sequence differing by no more than 3 nucleotides (optionally, by no more than 2 nucleotides; optionally, by 1 nucleotide) from at least 15 contiguous nucleotides of any one of the antisense strands of SEQ ID NO: 3 and SEQ ID NO: 4.

In the present disclosure, the "5' region" of the sense or antisense strand, i.e., the "5' end" or "5' terminus", is used interchangeably. For example, the nucleotides at positions 2 to 8 of the 5' region of the antisense strand may be replaced with the nucleotides at positions 2 to 8 of the 5' end of the antisense strand. Likewise, the "3' region", "3' terminus" and "3' end" of the sense or antisense strand are also used interchangeably.

Unless otherwise specified, in the context of the present disclosure, "G", "C", "A", "T", and "U" each represent a nucleotide and contain the bases guanine, cytosine, adenine, thymidine, and uracil, respectively. The lowercase letter d indicates that the nucleotide downstream of and adjacent to the letter d is a deoxyribonucleotide; the lowercase letter m indicates that the nucleotide upstream of and adjacent to the letter m is a methoxy-modified nucleotide; the lowercase letter f indicates that the nucleotide upstream of and adjacent to the letter f is a fluoro-modified nucleotide; the lowercase letter s indicates that the two nucleotides adjacent to the letter s are linked by a phosphorothioate group.

As used in the present disclosure, the term "2'-fluoro-modified nucleotide" refers to a nucleotide in which the hydroxy group at the 2' position of the ribosyl group of the nucleotide is substituted with fluorine, and the term "non-2'-fluoro-modified nucleotide" refers to a nucleotide or a nucleotide analog in which the hydroxy group at the 2' position of the ribosyl group of the nucleotide is substituted with a non-fluorine group.

As used in the present disclosure, the term "2'-methoxy-modified nucleotide" refers to a nucleotide in which the 2'-hydroxy group of the ribosyl group is substituted with a methoxy group.

As used herein, the terms "complementary" and "reverse complementary" are used interchangeably and have the meaning well known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand and guanines are always paired with cytosines, the two strands

are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from an untreated or control (e.g., buffer-only control or inert agent control) treated subject, cell, or sample. For example, the remaining mRNA expression level can be used to characterize the degree of inhibition of target gene expression by the siRNA; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using Dual-Glo® Luciferase Assay System: the Firefly chemiluminescence value (Fir) and the Renilla chemiluminescence value (Ren) are each read, and the relative value Ratio = Ren/Fir is calculated; in the present disclosure, the ratio of the remaining mRNA expression level (or residual activity%) = Ratio (siRNA-treated group) / Ratio (siRNA-free control group), and the inhibition rate (%) = 100% - remaining mRNA expression level (%).

Unless otherwise specified, the "compound", "ligand", "nucleic acid-ligand conjugate", "siRNA conjugate", "nucleic acid", "conjugate", "chemical modification", "targeting ligand", "dsRNA", and "RNAi" of the present disclosure can each independently exist in the form of a salt, mixed salts, or a non-salt (e.g., a free acid or free base). When existing in the form of a salt or mixed salts, it can be a pharmaceutically acceptable salt.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts; sodium salts are preferred. Salts derived from organic bases include, but are not limited to, salts of the following: primary, secondary and tertiary amines, substituted amines including naturally occurring substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

"Effective amount" or "effective dose" refers to the amount of a drug, a compound, or a pharmaceutical composition necessary to obtain any one or more beneficial or desired therapeutic results. For preventive use, the beneficial or desired results include elimination or reduction of risk, reduction of severity or delay of the onset of a condition, including the biochemistry, histology, and/or behavioral symptoms of the condition, complications thereof, and intermediate pathological phenotypes that appear during the progression of the condition. For therapeutic applications, the beneficial or desired results include clinical results, such as reducing the incidence of various conditions related to the target gene, target mRNA, or target protein of the present disclosure or alleviating one or more symptoms of the condition, reducing the dose of other agents required to treat the condition, enhancing the therapeutic effect of another agent, and/or delaying the progression of conditions related to the target gene, target mRNA, or target protein of the present disclosure in the patient.

As used herein, "patient", "subject", and "individual" are used interchangeably and include human or non-human animals, e.g., mammals, e.g., humans or monkeys.

The siRNA provided by the present disclosure can be obtained using a preparation method conventional in the art (e.g., solid-phase synthesis and liquid-phase synthesis). Solid-phase synthesis has been commercially available as customization service. A modified nucleotide group can be introduced into the siRNA of the present disclosure using a nucleoside monomer with a corresponding modification. Methods of preparing a nucleoside monomer with a corresponding modification and introducing a modified nucleotide group into an siRNA are also well known to those skilled in the art.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs.

The terms "blunt" and "blunt ended" are used interchangeably and mean that there are no unpaired nucleotides or nucleotide analogs at a given terminus of an siRNA, i.e., no nucleotide overhang. In most cases, an siRNA whose both ends are blunt ended will be double-stranded over its entire length.

The terms "about" and "approximately" mean that a numerical value is within an acceptable error range for the specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "comprising essentially" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprising" should be assumed to be within an acceptable error range for that specific value.

Unless otherwise specified, "optionally" or "optional" means that the event or circumstance subsequently described may, but does not necessarily, occur, and this description includes instances where the event or circumstance occurs or does not occur. For example, "optionally, $R_1$ and $R_2$ are directly linked to form a ring" means that $R_1$ and $R_2$ being directly linked to form a ring may occur but does not necessarily exist, and this description includes an instance where $R_1$ and $R_2$ are directly linked to form a ring and an instance where $R_1$ and $R_2$ do not form a ring.

As used herein, the terms "contain" or "comprise" are intended to be inclusive of the stated elements, integers, or steps, but not to exclude any other elements, integers, or steps. When the term "contain" or "comprise" is used herein, unless otherwise indicated, it also encompasses the situation of being consisting of the stated element, integer, or step. For example, when reference is made to "comprise" a particular sequence, it is also intended to encompass the situation of being consisting of that particular sequence.

The term "alkyl" refers to a saturated aliphatic hydrocarbon group which is a straight-chain or branched-chain group containing 1 to 20 carbon atoms. In some embodiments, it is selected from the group consisting of alkyl groups containing 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched-chain isomers thereof, and the like. In some embodiments, it is selected from the group consisting of alkyl groups containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted, and when it is substituted, the substituent may be substituted at any accessible point of attachment; the substituent, in some embodiments, is selected from the group consisting of one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "alkoxy" refers to -O-(alkyl) and -O-(unsubstituted cycloalkyl), wherein the alkyl group is as defined above. Non-limiting examples of alkoxy groups include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent, in some embodiments, is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy,

and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano. Likewise, the definitions of "alkynyloxy", "alkenyloxy", "cycloalkoxy", "heterocycloalkoxy", and "cycloalkenyloxy" are similar to the above definition of "alkoxy".

The term "alkenyl" refers to a straight-chain or branched-chain non-aromatic hydrocarbon group containing at least one carbon-carbon double bond and having 2-10 carbon atoms. Up to 5 carbon-carbon double bonds may be present in such groups. For example, "$C_2$-$C_6$" alkenyl is defined as an alkenyl group having 2-6 carbon atoms. Examples of alkenyl groups include, but are not limited to: ethenyl, propenyl, butenyl, and cyclohexenyl. The straight, branched, or cyclic portion of an alkenyl group may contain a double bond and is optionally mono-, di-, tri-, tetra-, or penta-substituted at any position as permitted by normal valency.

The term "cycloalkenyl" refers to a monocyclic hydrocarbon group having the specified number of carbon atoms and at least one carbon-carbon double bond.

The term "alkynyl" refers to a straight-chain or branched-chain hydrocarbon group containing 2-10 carbon atoms and containing at least one carbon-carbon triple bond. Up to 5 carbon-carbon triple bonds may be present. Thus, "$C_2$-$C_6$ alkynyl" refers to an alkenyl group having 2-6 carbon atoms. Examples of alkynyl groups include, but are not limited to: ethynyl, 2-propynyl, and 2-butynyl. The straight or branched portion of an alkynyl group may contain a triple bond as permitted by normal valency and is optionally mono-, di-, tri-, tetra-, or penta-substituted at any position as permitted by normal valency.

The term "keto" refers to any alkyl, alkenyl, alkynyl, cycloalkyl, cycloalkenyl, heterocyclyl, heteroaryl, or aryl group as described herein attached through a carbonyl bridge. Examples of keto groups include, but are not limited to: alkanoyl (e.g., acetyl, propionyl, butyryl, pentanoyl, or hexanoyl), alkenoyl (e.g., acryloyl), alkynoyl (e.g., ethynoyl, propynoyl, butynoyl, pentynoyl, or hexynoyl), aroyl (e.g., benzoyl), and heteroaroyl (e.g., pyrroloyl, imidazoloyl, quinolinoyl, or pyridinoyl).

The term "alkoxycarbonyl" refers to any alkoxy group as defined above attached through a carbonyl bridge (i.e., -C(O)O-alkyl). Examples of alkoxycarbonyl groups include, but are not limited to: methoxycarbonyl, ethoxycarbonyl, isopropoxycarbonyl, n-propoxycarbonyl, tert-butoxycarbonyl, benzyloxycarbonyl, or n-pentoxycarbonyl.

The term "aryloxycarbonyl" refers to any aryl group as defined above attached through an oxycarbonyl bridge (i.e., -C(O)O-aryl). Examples of aryloxycarbonyl groups include, but are not limited to: phenoxycarbonyl and naphthyloxycarbonyl.

The term "heteroaryloxycarbonyl" refers to any heteroaryl group as defined above attached through an oxycarbonyl bridge (i.e., -C(O)O-heteroaryl). Examples of heteroaryloxycarbonyl groups include, but are not limited to: 2-pyridinyloxycarbonyl, 2-oxazolyloxycarbonyl, 4-thiazolyloxycarbonyl, or pyrimidinyloxycarbonyl.

The term "cycloalkyl" or "carbocycle" refers to a saturated or partially unsaturated, monocyclic or polycyclic hydrocarbon substituent; the cycloalkyl ring contains 3 to 20 carbon atoms. In some embodiments, it is selected from the group consisting of those containing 3 to 7 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, and the like. Polycyclic cycloalkyl groups include spiro, fused, and bridged cycloalkyl groups. Cycloalkyl may be substituted or unsubstituted. When it is substituted, the substituent may be substituted at any accessible point of attachment. In some embodiments, it is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The cycloalkyl ring may be fused to an aryl or heteroaryl ring, wherein the ring attached to the parent structure is a cycloalkyl group; non-limiting examples include indanyl, tetrahydronaphthyl, benzocycloheptyl, and the like. Cycloalkyl may be optionally substituted or unsubstituted, and when it is substituted, the substituent, in some embodiments, is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The term "heterocycloalkyl" or "heterocycle" or "heterocyclyl" refers to a saturated or partially unsaturated, monocyclic or polycyclic hydrocarbon substituent containing 3 to 20 ring atoms, wherein one or more of the ring atoms are heteroatoms selected from the group consisting of nitrogen, oxygen, or $S(O)_m$ (where m is an integer of 0 to 2), excluding a ring moiety of -O-O-, -O-S-, or -S-S-, and the other ring atoms are carbon atoms. In some embodiments, it

is selected from the group consisting of those containing 3 to 12 ring atoms, of which 1 to 4 are heteroatoms. In some embodiments, it is selected from the group consisting of those containing 3 to 7 ring atoms. Non-limiting examples of monocyclic heterocycloalkyl groups include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like. Polycyclic heterocycloalkyl groups include spiro, fused, and bridged heterocycloalkyl groups. Non-limiting examples of "heterocycloalkyl" groups include:

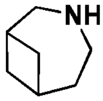, 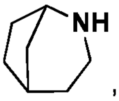, 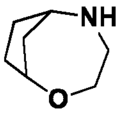, 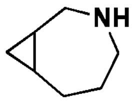, 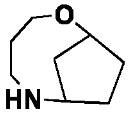, 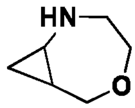,

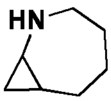, 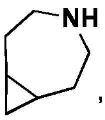, 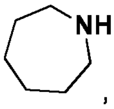, 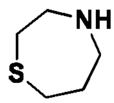, 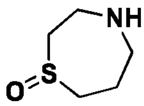, 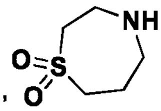,

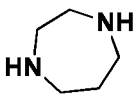, 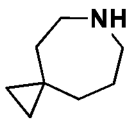, 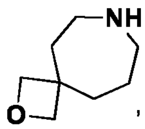, 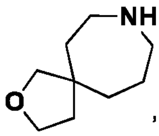, 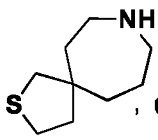, 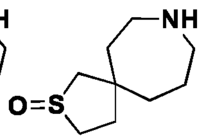,

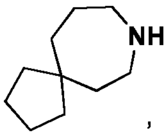, 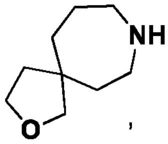, 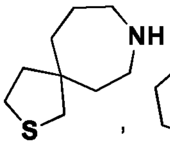, 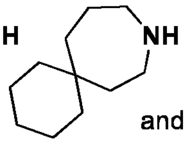 and 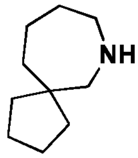,

and the like.

The term "hydroxy" refers to the -OH group.

The term "halogen" refers to fluorine, chlorine, bromine, or iodine.

The term "haloalkyl" refers to an alkyl group substituted with a halogen, wherein the alkyl group is as defined above.

The term "cyano" refers to -CN.

The term "nitro" refers to $-NO_2$.

The term "oxo" refers to the =O group; for example, a carbon atom is attached to an oxygen atom through a double bond, wherein a keto or aldehyde group is formed.

The term "amino" refers to $-NH_2$.

The term "carboxyl" refers to -C(O)OH.

The term "aldehyde" refers to -CHO.

In the chemical structural formulas of the present disclosure, " 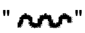 " or 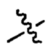 may be linked to any group or groups in accordance with the scope of the invention described herein; the asterisk "*" indicates a chiral center.

In the present disclosure, a "phosphoester group" is a phosphodiester group unless otherwise specified.

In the present disclosure, the phosphorothioate group refers to a phosphodiester group modified by replacing one non-bridging oxygen atom with a sulfur atom, and is used interchangeably with

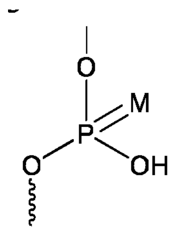

and

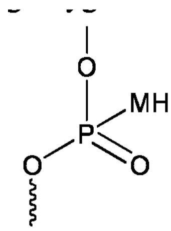

M is a S atom.

In the context of the present disclosure, the

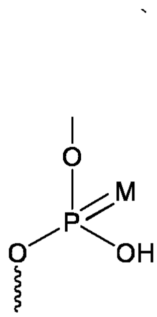

in the group

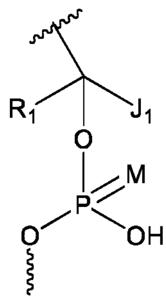

can be replaced with any group capable of linking to an adjacent nucleotide.

The term "link", "connect", or "attach", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents

include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, keto, alkoxycarbonyl, aryloxycarbonyl, heteroaryloxycarbonyl, or halogens (e.g., F, Cl, Br, or I). When the substituent is ketone or oxo (i.e., =O), two (2) hydrogens on the atom are replaced.

"Substituted with one or more" means that it may be substituted with a single substituent or multiple substituents. In the case of substitution with multiple substituents, there may be a plurality of identical substituents, or one combination of or a plurality of combinations of different substituents.

Some abbreviations in the present disclosure are defined as follows:

DCE: dichloroethane;
$Sc(OTf)_3$: scandium trifluoromethanesulfonate;
TFH: tetrahydrofuran;
Pd/C: palladium-carbon;
TFA: trifluoroacetic acid;
DMF: dimethylformamide;
DIPEA: N-ethyl diisopropylamine;
HoBt: 1-hydroxybenzotriazole;
EDCI: 1-ethyl-(3-dimethylaminopropyl)carbodiimide hydrochloride;
DMTrCl: 4,4'-dimethoxytrityl chloride;
DIEA: N,N-diisopropylethylamine;
HATU: 2-(7-azabenzotriazole)-N,N,N',N'-tetramethyluronium hexafluorophosphate;
LiOH: lithium hydroxide;
DMAP: 4-dimethylaminopyridine;
HBTU: benzotriazole-N,N,N',N'-tetramethyluronium hexafluorophosphate;
DMTrCl: 1-[chloro(4-methoxyphenyl)benzyl]-4-methoxybenzene
$CF_3SO_3H$: trifluoromethanesulfonic acid;
BnBr: benzyl bromide;
DEPBT: 3-(diethoxyphosphoryloxy)-1,2,3-benzotriazin-4-one;
Bz: a benzoyl protecting group;
MMTr: methoxyphenyl diphenylmethyl;
DMTr: a dimethoxytrityl protecting group.

## BRIEF DESCRIPTION OF THE DRAWINGS

FIG. 1 shows the TTR mRNA expression levels at day 7 after administration of TRD002218 and TRD007205.
FIG. 2 shows the TTR mRNA expression levels at day 28 after administration of TRD002218 and TRD007205.

## DETAILED DESCRIPTION

The present disclosure is further described below with reference to examples; however, these examples are not intended to limit the scope of the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

### Part One. Anti-Off-Target Modifications

### Example 1. Preparation of Chemical Modifications

#### 1.1. Synthesis of compound 1-1a and compound 1-1b

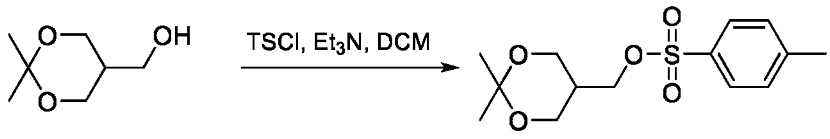


1 2

Compound 1 (500 mg, 3.42 mmol) and triethylamine ($Et_3N$, 692 mg, 6.84 mmol, 0.95 mL) were dissolved in dichloromethane (DCM, 10 mL). A solution of 4-toluenesulfonyl chloride (tsCl, 717 mg, 3.76 mmol) in dichloromethane (10 mL) was added dropwise under an ice bath. After the dropwise addition, the mixture was stirred overnight at room temperature. After the reaction was complete, the reaction mixture was quenched with water. The aqueous phase was extracted three times with dichloromethane (15 mL). The combined organic phases were washed first with saturated aqueous sodium bicarbonate solution (10 mL) and then with saturated brine (20 mL) and were then concentrated under reduced pressure to remove the solvent to give a crude product 2 (820 mg, 80%). The crude product was directly used in the next step. MS m/z: $C_{14}H_{21}O_5S$, $[M+H]^+$ calculated: 301.10, found: 301.2.

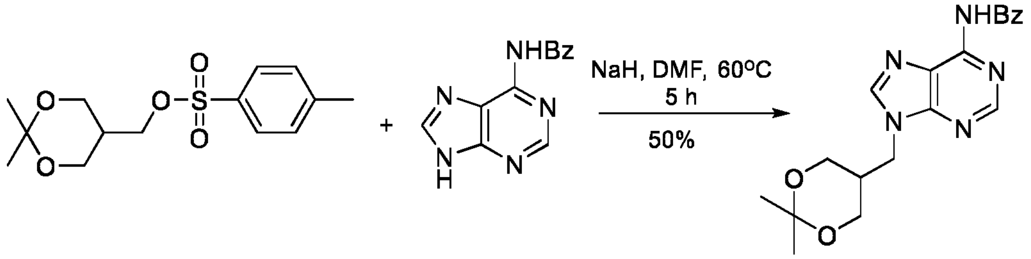


2 3 4

Compound 3 (239 mg, 1.22 mmol) was dissolved in dimethylformamide (DMF, 10 mL). A solution of NaH (60% in mineral oil, 93 mg, 2.33 mmol) was added under an ice bath. The mixture was stirred for 30 min, and then compound 2 (350 mg, 1.16 mmol) was added dropwise. After the dropwise addition, the mixture was stirred at 60 °C for 5 h. After the reaction was complete, the reaction mixture was quenched with water. The aqueous phase was extracted three times with ethyl acetate (15 mL). The combined organic phases were washed first with water (10 mL) three times and then with saturated brine (10 mL) and were then concentrated under reduced pressure to remove the solvent. The residue was purified by preparative reversed-phase HPLC ($C^{18}$, conditions: 5-50% (A: $H_2O$, B: $CH_3CN$), flow rate: 70 mL/min) and lyophilized to give compound 4 (220 mg). MS m/z: $C_{19}H_{21}N_5O_3Na$, $[M+Na]^+$ calculated: 390.16, found: 390.3.

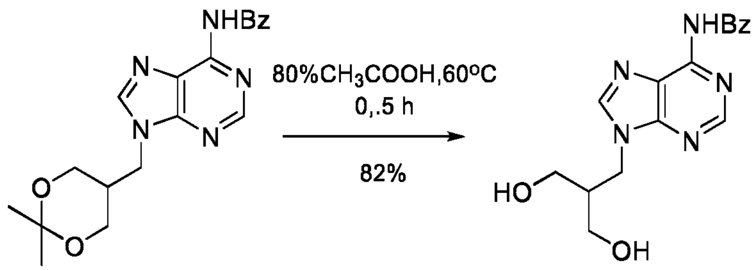


4 5

Compound 4 (1.50 g, 4.08 mmol) was dissolved in 20 mL of a mixed solution of acetic acid and water (4:1) at room temperature, and the mixture was stirred at 60 °C for 30 min. After the reaction was complete, the reaction mixture was concentrated under reduced pressure to remove the solvent. The residue was purified by preparative reversed-phase HPLC ($C^{18}$, conditions: 5-25% (A: $H_2O$, B: $CH_3CN$), flow rate: 70 mL/min) and lyophilized to give compound 5 (1.10 g). MS m/z: $C_{16}H_{18}N_5O_3$, $[M+H]^+$ calculated: 328.13, found: 328.4.

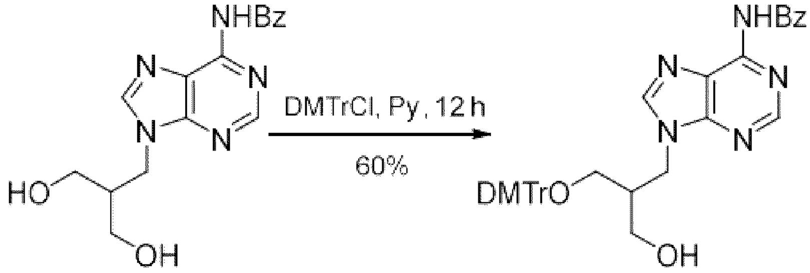


5 6

Compound 5 (1.00 g, 3.05 mmol) was dissolved in pyridine (Py, 10 mL). A solution of 4,4'-dimethoxytrityl chloride (DMTrCl, 1.50 g, 4.58 mmol) in pyridine (5 mL) was added dropwise under an ice bath. After the dropwise addition, the mixture was stirred overnight at room temperature. After the reaction was complete, the reaction mixture was quenched with water and concentrated under reduced pressure to remove the solvent. The residue was purified by preparative reversed-phase HPLC ($C^{18}$, conditions: 5-80% (A: $H_2O$, B: $CH_3CN$), flow rate: 70 mL/min) and lyophilized to give compound 6 (1.00 g). MS m/z: $C_{37}H_{36}N_5O_5$, $[M-H]^+$ calculated: 630.26, found: 630.5. The racemic compound 6 was resolved using a chiral column (Daicel CHIRALPAK® IE 250 × 4.6 mm, 5 μm, A: n-hexane, B: ethanol) to give 6A(-) (410 mg) and 6B(+) (435 mg).

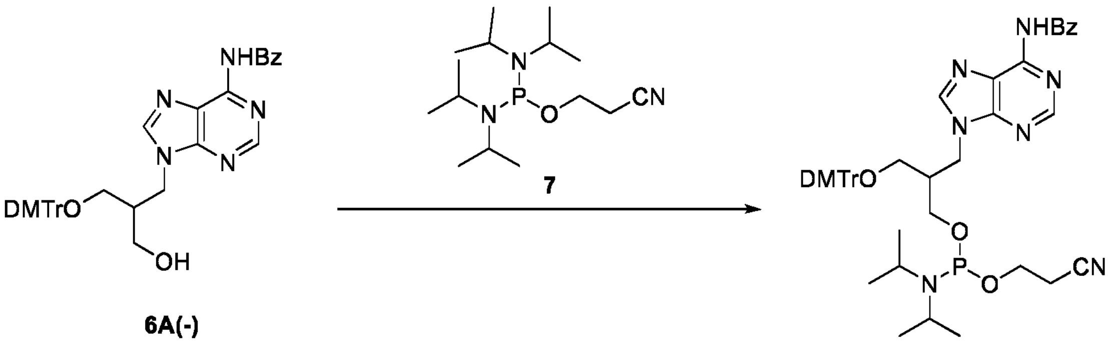

6A(-) 7 1-1a

Compound 6A(-) (200 mg, 0.32 mmol), tetrazole (11 mg, 0.16 mmol), N-methylimidazole (5 mg, 0.06 mmol), and 3A molecular sieve (500 mg) were dissolved in 10 mL of acetonitrile. Compound 7 (144 mg, 0.48 mmol) was added at room temperature. The mixture was stirred overnight at room temperature. After the reaction was complete, the molecular sieve was filtered out, and dichloromethane (30 mL) was added. The mixture was washed with saturated aqueous sodium bicarbonate solution (10 mL) three times and then with saturated brine (20 mL). The filtrate was concentrated to dryness by rotary evaporation, and the residue was purified by preparative reversed-phase HPLC ($C^{18}$, conditions: 5-100% (A: water, B: $CH_3CN$), flow rate: 70 mL/min) and lyophilized to give compound 1-1a (200 mg). MS m/z: $C_{40}H_{39}N_6O_7P$, $[M\text{-diisopropyl}+OH]^+$ calculated: 747.26, found: 747.6. 1H NMR (400 MHz, acetonitrile-$d_3$) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

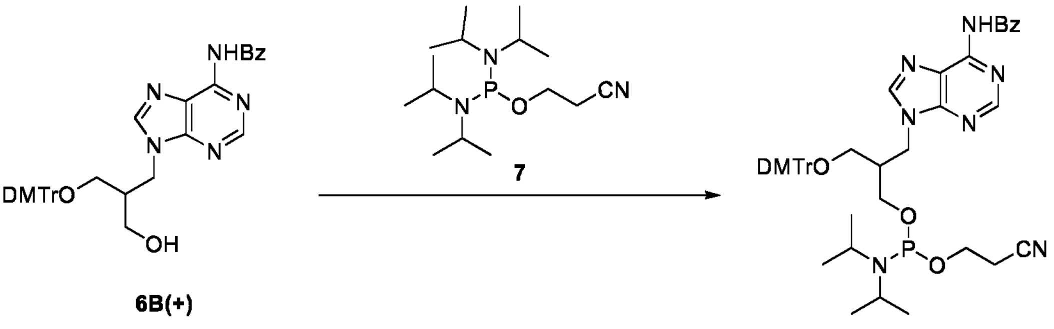

6B(+) 7 1-1b

Compound 6B(+) (200 mg, 0.32 mmol), tetrazole (11 mg, 0.16 mmol), N-methylimidazole (5 mg, 0.06 mmol), and 3A molecular sieve (500 mg) were dissolved in 10 mL of acetonitrile. Compound 7 (144 mg, 0.48 mmol) was added at room temperature. The mixture was stirred overnight at room temperature. After the reaction was complete, the molecular sieve was filtered out, and dichloromethane (30 mL) was added. The mixture was washed with saturated aqueous sodium bicarbonate solution (10 mL) three times and then with saturated brine (20 mL). The filtrate was concentrated to dryness by rotary evaporation, and the residue was purified by preparative reversed-phase HPLC ($C^{18}$, conditions: 5-100% (A: water, B: $CH_3CN$), flow rate: 70 mL/min) and lyophilized to give compound **1-1b** (200 mg). MS m/z: $C_{40}H_{39}N6O_7P$, $[M\text{-diisopropyl+OH}]^+$ calculated: 747.26, found: 747.5.

### 1.2. Synthesis of compound 1-6a

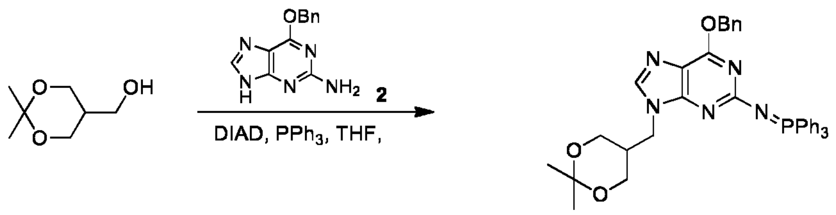


1 3

Compound 1 (10 g, 68.404 mmol), compound 2 (15 g, 62.186 mmol), and triphenylphosphine (32.62 g, 124.371 mmol) were dissolved in anhydrous THF (30 mL). DIAD (24.656 mL, 124.371 mmol) was slowly added dropwise at 0 °C. The reaction mixture was left to react at 25 °C for 12 h, and LCMS analysis showed the reaction had been complete. The reaction mixture was extracted with ethyl acetate (200 mL) and water (200 mL). The organic phase was dried. The filtrate was concentrated, and the resulting residue was purified using a normal-phase column (DCM/MeOH = 10/1) to give the target product 3 20 g).

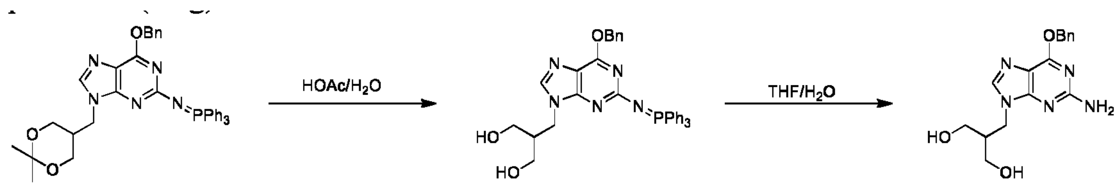


3 4 5

Compound 3 (20 g, 28.585 mmol) was dissolved in acetic acid (24 mL, 426.016 mmol) and H2O (12 mL). The mixture was stirred at 60 °C for 1 h. The reaction mixture was then concentrated to dryness by rotary evaporation. THF (12 mL) and H2O (12 mL) were added. The mixture was stirred at 80 °C for 7 h. LCMS analysis showed the reaction had been complete. The reaction mixture was extracted with ethyl acetate (200 mL) and water (100 mL). Solid sodium carbonate was added to the aqueous phase until a large amount of solid precipitated from the aqueous phase. The solid was collected by filtration and washed with water. The filter cake was dried using an oil pump to give the target compound 5 (9 g).

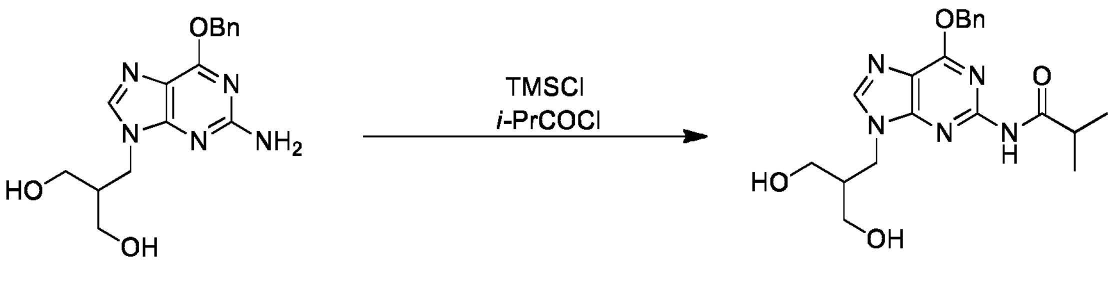


5 6

Compound 5 (6.8 g, 18.581 mmol) was dissolved in pyridine (80 mL) in a nitrogen atmosphere. TMSCLl (14.250 mL, 111.489 mmol) was slowly added at 0 °C. The mixture was stirred for 2 h. Isobutyryl chloride (2.044 mL, 19.511 mmol) was then added at 0 °C.

The mixture was stirred at 25 °C for 1 h, and LCMS analysis showed the reaction had been complete. The reaction mixture was extracted with dichloromethane (200 mL) and water (200 mL). After the organic phase was dried and concentrated to dryness by rotary evaporation, a sample to be purified was prepared. The sample was purified using a normal-phase column (elution with DCM:MeOH =10:1, peak at 4.8%) to give compound 6 as a yellow oil (12 g).

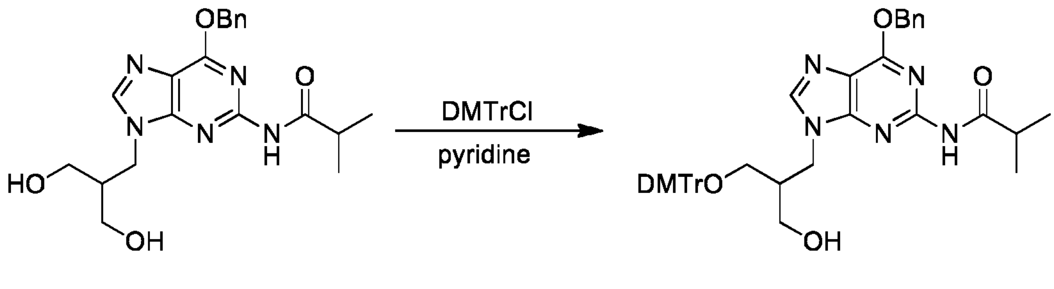


6 7

Compound 6 (5.5 g, 12.392 mmol) was dissolved in pyridine (30 mL) in a nitrogen atmosphere. Molecular sieve 4A 1/16 (7 g, 12.392 mmol) was added, and then solid DMTrCl (5.04 g, 14.870 mmol) was added in batches at 0 °C. The mixture was left to react at 25 °C for 2 h, and TLC analysis (PE:EtOAc = 1:1, Rf = 0.69) showed the reaction had been complete. The reaction mixture and TJN200879-040-P1 were combined and treated together. The reaction mixture was extracted with ethyl acetate (200 mL) and water (200 mL). After the organic phase was dried and concentrated to dryness by rotary evaporation, a sample to be purified was prepared. The sample was purified using a normal-phase column (elution with PE:EtOAc, peak at 84%) to give compound 7 as a yellow oil (12 g).

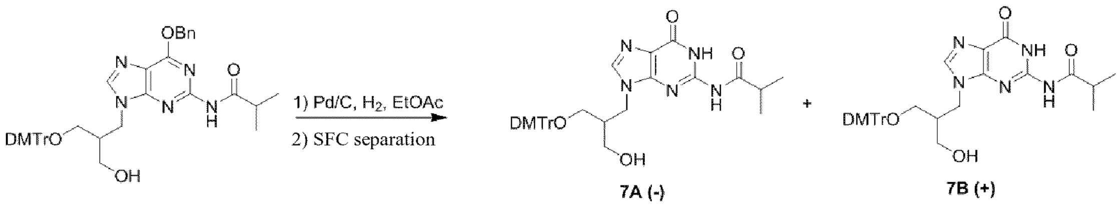


7A (-) + 7B (+)

7

Compound 7 (12 g, 15.389 mmol) was dissolved in EtOAc (140 mL). Wet palladium on carbon Pd/C (7 g, 15.389 mmol) was added. The reaction mixture was left to react at 25 °C in a hydrogen atmosphere (15 Psi) for 2 h. TLC analysis (PE:EtOAc = 0: 1, Rf = 0.09) showed the reaction had been complete. The reaction mixture was filtered. After the filter cake was rinsed three times with ethyl acetate (30 mL), the filtrate was collected. After the filtrate was concentrated to dryness by rotary evaporation, 50 mL of dichloromethane and 2 mL of triethylamine were added to prepare a sample to be purified. The sample was purified using a normal-phase column (elution with DCM:MeOH =10:1, peak at 0.5%) to give 9 g (a yellow foamy solid). The resulting racemic compound was resolved by SFC to give products, the target compound 7A(-) (3.9 g) and the target compound 7B(+) (3.8 g).

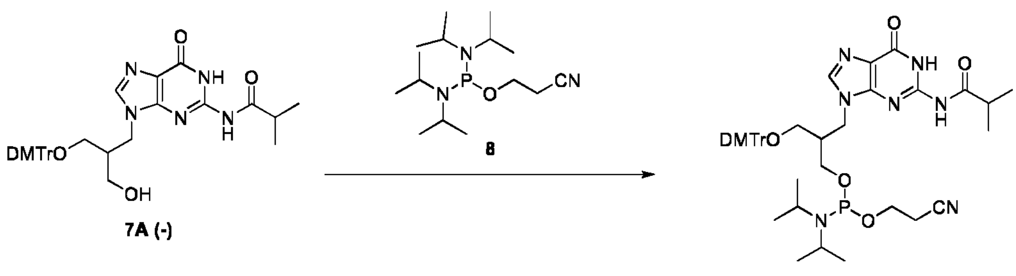

7A (-) 8 1-6a

Compound 7A(-) (3.30 g, 5.40 mmol), tetrazole (190 mg, 2.70 mmol), 1-methylimidazole (90 mg, 1.10 mmol),

and 3A molecular sieve (500 mg) were dissolved in 30 mL of acetonitrile. Compound 8 (2.50 g, 8.10 mmol) was added at room temperature. The mixture was stirred at room temperature for 2 h. After the reaction was complete, the molecular sieve was filtered out, and DCM (150 mL) was added. The mixture was washed with saturated aqueous sodium bicarbonate solution (30 mL × 3) and then with saturated brine (30 mL). The filtrate was concentrated to dryness by rotary evaporation, and the residue was purified by preparative reversed-phase HPLC (C18, conditions: 5-100% (A: water, B: CH3CN), flow rate: 70 mL/min) and lyophilized to give compound 1-6a (2.9 g, 66%). MS m/z: C43H55N7O7P [M+H]+, calculated: 812.38, found: 812.5. 1H NMR (400 MHz, acetonitrile-d3) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

**1.3. Synthesis of compound 1-7a**

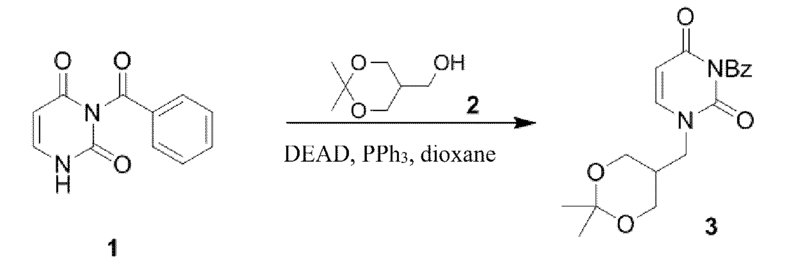


1 3

Compound 1 (5 g, 23.1272 mmol), compound 2 (6.76 g, 46.254 mmol), and triphenylphosphine (7.28 g, 27.753 mmol) were dissolved in 30 mL of dioxane in a nitrogen atmosphere. DEAD (5.502 mL, 27.753 mmol) was slowly added dropwise at 0 °C. After the dropwise addition, the reaction mixture was slowly warmed to 25 °C and left to react for another hour. 100 mL of H2O and 100 mL of EtOAc were added to the reaction mixture for extraction. After the organic phases were combined, dried, filtered, and concentrated, a sample to be purified was prepared. The sample was purified using a normal-phase column (elution with PE:EtOAc = 1:1) to give the target product (4 g).

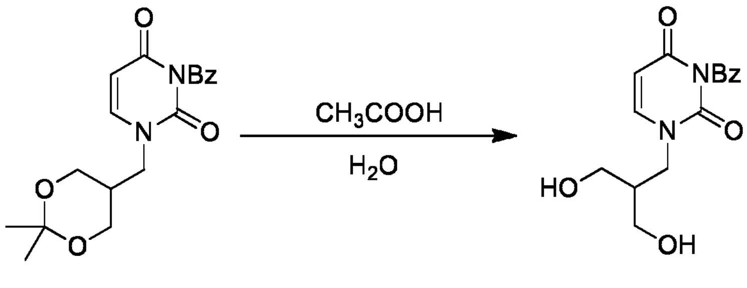


3 4

Compound 3 (3.3 g) was dissolved in HOAc (16 mL) and H2O (4 mL), and the solution was heated in an oil bath at 60 °C for 0.5 h. The reaction mixture was concentrated to dryness by rotary evaporation, and the resulting residue was purified using a normal-phase column (elution with PE:EtOAc = 0:1) to give the target product 4 (3 g).

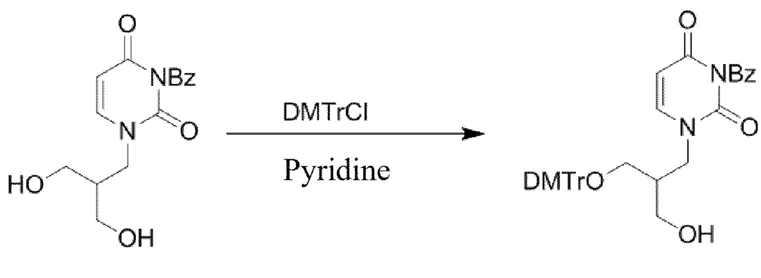


4 5

Compound 4 (3 g, 8.873 mmol) was dissolved in 5 mL of pyridine. A solution of DMTrCl (3.91 g, 11.535 mmol) in 10 mL of pyridine was slowly added dropwise at 0 °C in a nitrogen atmosphere. After the dropwise addition, the reaction mixture was warmed to 25 °C and left to react for another hour. 50 mL of water and 100 mL of ethyl acetate were added to the reaction mixture for extraction. The aqueous phase was extracted three times with 100 mL of ethyl acetate. The organic phases were combined, dried, filtered, and concentrated, and the residue was purified using a normal-phase column (with PE:EtOAc = 2:1) to give the target product 5 (4 g).

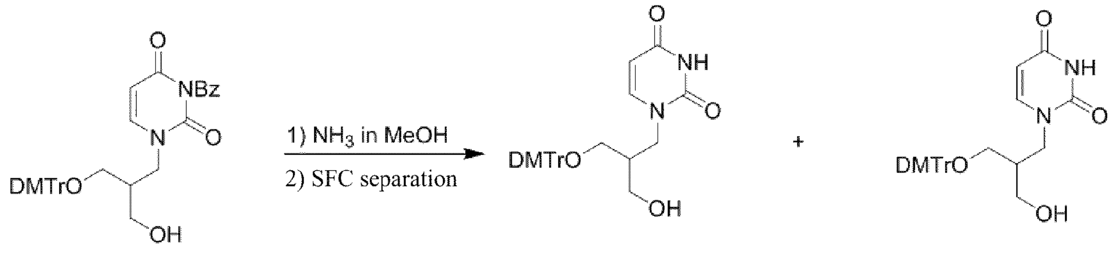


5 6A (-) 6B (+)

Compound 5 (4 g, 5.769 mmol) was dissolved in methanol (10 mL). A saturated solution of NH3 in methanol (40 mL) was added. The mixture was left to react at 0 °C for 6 h. The reaction mixture was concentrated to dryness by rotary evaporation, and the residue was purified using a normal-phase column (PE:EtOAc = 0:1) to give a racemic compound (2.4 g). The racemic compound was resolved by SFC to give the target product 6A (750 mg, 100% purity) and the target product 6B (400 mg, 99.16% purity).

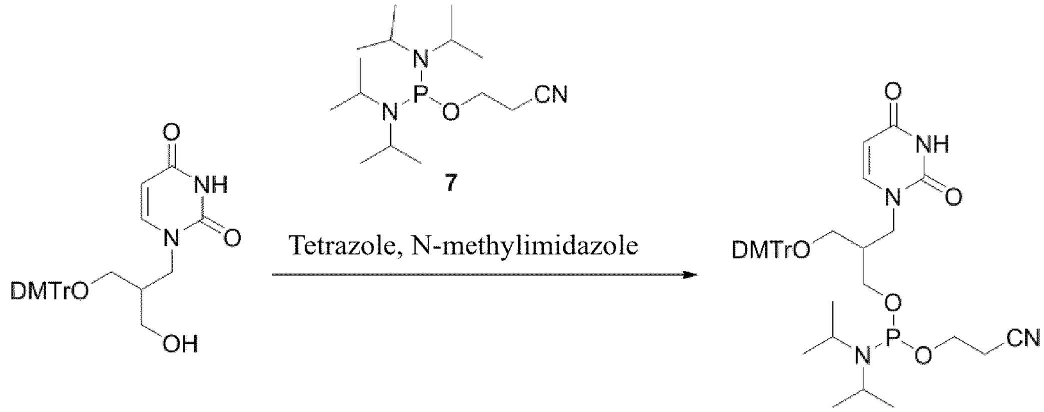


7

6A (-) 1-7a

Compound 6A(-) (700 mg, 1.40 mmol), tetrazole (50 mg, 0.70 mmol), 1-methylimidazole (23 mg, 0.28 mmol), and 3A molecular sieve (500 mg) were dissolved in 10 mL of acetonitrile. Compound 7 (630 mg, 2.10 mmol) was added at room temperature. The mixture was stirred at room temperature for 2 h. After the reaction was complete, the molecular sieve was filtered out, and DCM (50 mL) was added. The mixture was washed with saturated aqueous sodium bicarbonate solution (10 mL × 3) and then with saturated brine (20 mL). The filtrate was concentrated to dryness by rotary evaporation, and the residue was purified by preparative reversed-phase HPLC (C18, conditions: 5-100% (A: water, B: CH3CN), flow rate: 70 mL/min) and lyophilized to give compound 1-7a (700 mg, 72%). MS m/z: C38H47N4O7PNa [M+Na]+, calculated: 725.32, found: 725.5.

### 1.4. Synthesis of compound 1-8a

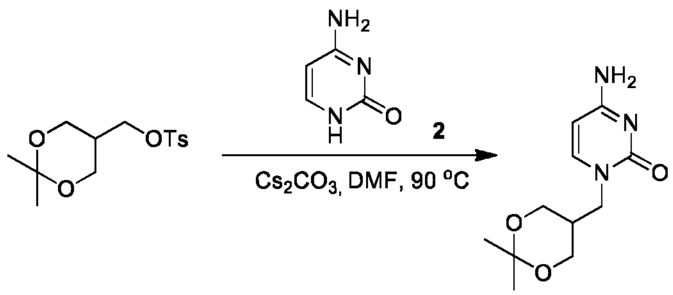


1 3

Compound 1 (8.5 g, 76.508 mmol) and compound 2 (30.64 g, 91.809 mmol) were dissolved in DMF (150 mL). CS2CO3 (29.91 g, 91.809 mmol) was added. The mixture was left to react in a nitrogen atmosphere at 90 °C for 12 h. LCMS analysis showed the reaction had been complete. The reaction mixture was filtered and concentrated to dryness by rotary evaporation using an oil pump, and the residue was separated and purified using a normal-phase column (80 g, DCM/MeOH = 10/1 to 5/1) to give the target product 3 (13.5 g, 80% purity).

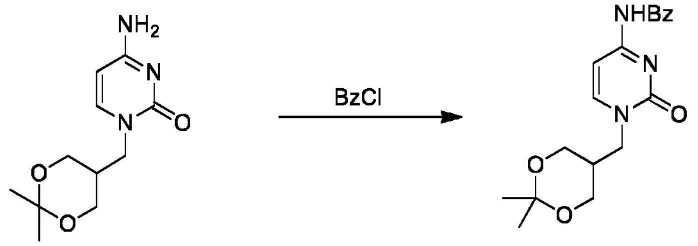


3 4

Compound 3 (10.5 g, 35.105 mmol) was dissolved in pyridine (65 mL) and CH3CN (65 mL). BzCl (4.894 mL, 42.126 mmol) was added dropwise to the solution. The mixture was left to react at 25 °C for 2 h. LCMS analysis showed most of the starting material had been consumed. The reaction mixture was quenched with H2O (100 mL) and extracted with EtOAc (100 mL × 3). The organic phase was dried and concentrated to dryness by rotary evaporation, and the residue was separated (combined with TJN200872-101) and purified by column chromatography (80 g, PE/EtOAc = 10/1 to 0/1, DCM/MeOH = 10/1) to give the target product 4 (14 g, 90% purity).

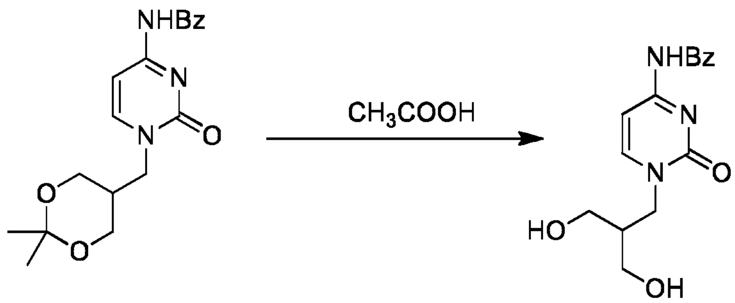


4 5

Compound 4 (14 g, 36.694 mmol) was dissolved in HOAc (56 mL, 314.796 mmol) and H2O (14 mL). The mixture was left to react at 60 °C for 2 h, and LCMS analysis showed the reaction had been complete. The reaction mixture was concentrated using an oil pump, and the residue was separated using a normal-phase column (40 g, DCM/MeOH = 1/0 to 5/1) to give the target product 5 (8.4 g, 90% purity & 2.4 g, 80% purity).

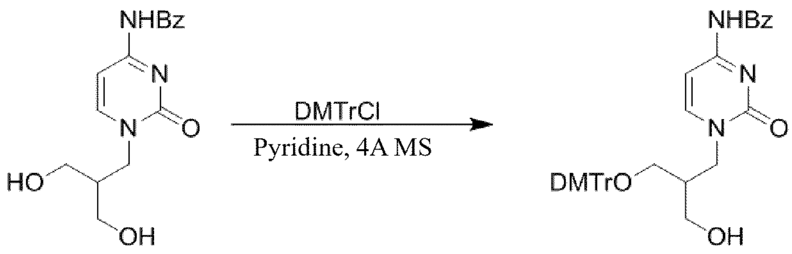


5 6

Compound 5 (7.4 g, 21.957 mmol), DMAP (0.54 g, 4.391 mmol), and molecular sieve 4A (11.1 g, 2.967 mmol) were dissolved in pyridine (60 mL). The mixture was stirred under an ice bath for 10 min, and then DMTrCl (8.93 g, 26.348 mmol) was added. The mixture was stirred for 1.8 h, and LCMS analysis showed about 19% of the starting material remained and about 60% was target MS. The reaction mixture and TJN200872-105&106 were combined and purified together. H2O (50 mL) was added to the reaction mixture, and extraction was performed with DCM (50 mL × 3). The organic phase was dried and concentrated to dryness by rotary evaporation, and the residue was separated by column chromatography (120 g, PE/(EA:DCM:TEA = 1:1:0.05) = 1/0 to 0/1 to DCM/MeOH = 10/1) to give compound 6 as a yellow solid (11 g, 89% purity, TJN200872-105&106&107). The starting material was recovered (3.0 g, 70% purity).

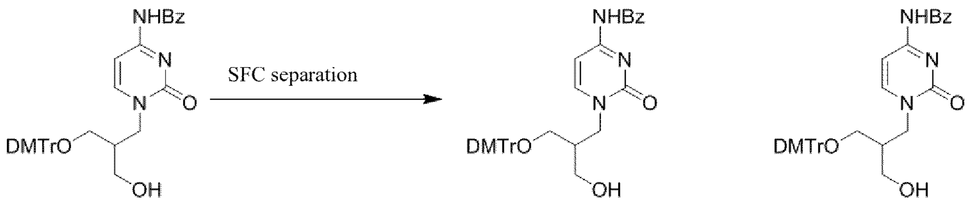


6 6A (+) 6B (-)

Compound 6 (15 g, 22.041 mmol) was resolved by SFC (DAICEL CHIRALPAK AD (250 mm × 50 mm,10 μm); 0.1% NH3H2O EtOH, B: 45%-45%; 200 mL/min) to give the target product 6A (5.33 g, 94.29% purity) and the target product 6B (6.14 g, 97.91% purity). 1.0 g of compound 6 was recovered.

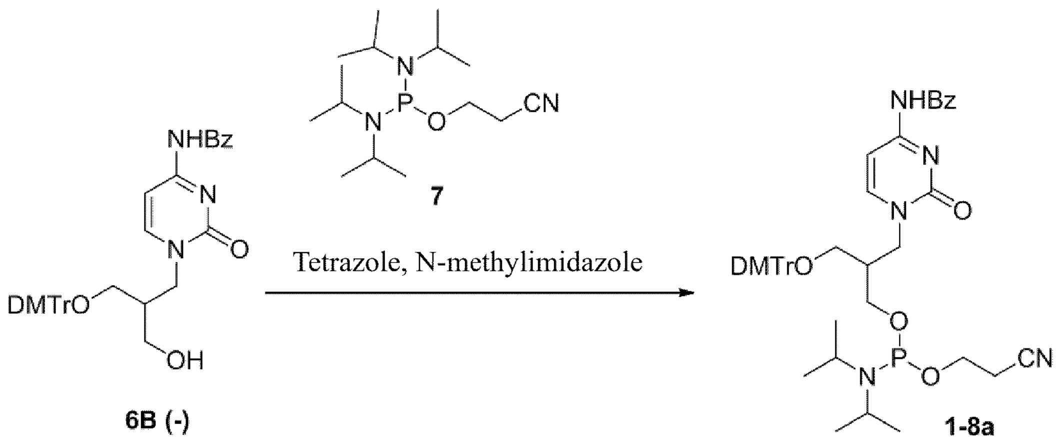


7

6B (-) 1-8a

Compound 6B(-) (5.4 g, 8.92 mmol), tetrazole (312 mg, 4.46 mmol), 1-methylimidazole (146 mg, 1.78 mmol), and 3A molecular sieve (500 mg) were dissolved in 40 mL of acetonitrile. Compound 7 (4 g, 13.4 mmol) was added at room temperature. The mixture was stirred at room temperature for 2 h. After the reaction was complete, the molecular sieve was filtered out, and DCM (200 mL) was added. The mixture was washed with saturated aqueous sodium bicarbonate solution (30 mL × 3) and then with saturated brine (50 mL). The filtrate was concentrated to dryness by rotary evaporation, and the residue was purified by preparative reversed-phase HPLC (C18, conditions: 5-100% (A: water, B: CH3CN), flow rate: 70 mL/min) and lyophilized to give compound 1-8a (5.8 g, 80%). MS m/z: C45H51N5O7P, [M+H]+, calculated: 804.36, found: 804.4.

**Example 2. Synthesis of siRNA**

The siRNA synthesis was the same as the conventional phosphoramidite solid-phase synthesis. In synthesizing the modified nucleotide at position 7 of the 5' end of the AS strand, the original nucleotide of the parent sequence was replaced with the phosphoramidite monomer synthesized above. The synthesis process is briefly described below: Nucleoside phosphoramidite monomers were linked one by one according to the synthesis program on a Dr. Oligo48 synthesizer (Biolytic), starting at a universal CPG support. Other than the nucleoside phosphoramidite monomer at position 7 of the 5' end of the AS strand described above, the other nucleoside monomer materials 2'-F RNA, 2'-O-methyl RNA, and other nucleoside phosphoramidite monomers were purchased from Hongene, Shanghai or Genepharma, Suzhou. 5-Ethylthio-1H-tetrazole (ETT) was used as an activator (a 0.6 M solution in acetonitrile), a 0.22 M solution of PADS in acetonitrile and collidine (1:1 by volume) (Kroma, Suzhou) as a sulfurizing agent, and iodopyridine/water solution (Kroma) as an oxidant.

After completion of solid phase synthesis, oligoribonucleotides were cleaved from the solid support and soaked in a solution of 28% ammonia water and ethanol (3:1) at 50 °C for 16 h. The mixture was centrifuged, and the supernatant was transferred to another centrifuge tube. After the supernatant was concentrated to dryness by evaporation, the residue was purified by C18 reversed-phase chromatography using 0.1 M TEAA and acetonitrile as the mobile phase, and DMTr was removed using 3% trifluoroacetic acid solution. The target oligonucleotides were collected, then lyophilized, identified as the target products by LC-MS, and quantified by UV (260 nm).

The resulting single-stranded oligonucleotides were paired in an equimolar ratio in a complementary manner and annealed. The final double-stranded siRNA was dissolved in 1× PBS, and the solution was adjusted to the concentration required for the experiment so it was ready to be used.

**Example 3. psiCHECK Activity Screening**

The synthesis of siRNA samples is as described before. The plasmids were obtained from Sangon Biotech (Shanghai) Co., Ltd. The experimental consumables for psiCHECK are shown in Table 1.

Table 1. Experimental consumables and reagents for psiCHECK

| **Reagent consumables** | | | | |
|---|---|---|---|---|
| **Name** | **Company** | **Catalog number/model** | **Batch No.** | **Shelf life** |
| Huh 7 cells | Cobioer, Nanjing | ATCC-Cobioer/CBP60202 | / | / |
| Dual-Glo® Luciferase Assay System | Promega | E2940 | 0000363099 | 2020/5/13 |
| Lipofectamine® 2000 | Invitrogen | 11668-019 | / | 2021/6/14 |

Experimental procedure: Cell plating and cell transfection were carried out. The specific amounts for preparing the transfection complex are shown in Table 2.

Table 2. Amounts required for transfection complex in each well of a 96-well plate

| | Amount/well | Opti-MEM |
|---|---|---|
| Plasmid mixture | 0.05 μL | 10 μL |
| Lipofectamine 2000 | 0.2 μL | 10 μL |
| Notes: Lipo: 0.2 μL/well; plasmid: 0.05 μL/well; Opti-MEM: 10 μL/well. | | |

According to Table 3, dilutions with different concentrations were prepared as working solutions for immediate use based on the different experimental requirements. 24 h after transfection, assays were carried out according to the instructions of the Dual-Glo® Luciferase Assay System kit.

Calculation of a relative value:

Calculation of a relative value: Ratio = Ren/Fir (a renilla/firefly ratio);

Calculation of the inhibition rate:

1 - (Ratio + siRNA/reporter gene only) × 100% = inhibition rate (%);

In the present disclosure, residual activity% (also referred to as the remaining mRNA expression level% or remaining mRNA expression ratio) = 100% - inhibition rate (%).

Table 3. Multi-concentration dilution protocol

| Final concentration (nM) | Addition of water and sample |
|---|---|
| / | / |
| 40 | 4 μL siRNA (20 μM) + 96 μL $H_2O$ |
| 13.33333333 | 30 μL siRNA + 60 μL $H_2O$ |
| 4.444444444 | 30 μL siRNA + 60 μL $H_2O$ |
| 1.481481481 | 30 μL siRNA + 60 μL $H_2O$ |
| 0.49382716 | 30 μL siRNA + 60 μL $H_2O$ |
| 0.164609053 | 30 μL siRNA + 60 μL $H_2O$ |
| 0.054869684 | 30 μL siRNA + 60 μL $H_2O$ |
| 0.018289895 | 30 μL siRNA + 60 μL $H_2O$ |
| 0.006096632 | 30 μL siRNA + 60 μL $H_2O$ |
| 0.002032211 | 30 μL siRNA + 60 μL $H_2O$ |
| 0.000677404 | 30 μL siRNA + 60 μL $H_2O$ |

**Example 4. Characterization of Chemical Modifications**

The siRNAs were synthesized using the compounds of Example 1 and the method of Example 2, and the on-target activity and off-target activity of each siRNA were verified using the method of Example 3. The siRNAs had identical sense strands and contained the following modified nucleotides/chemical modifications, respectively, at position 7 of the 5' end of the antisense strand:

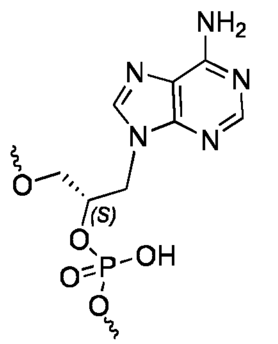
GNA(A)

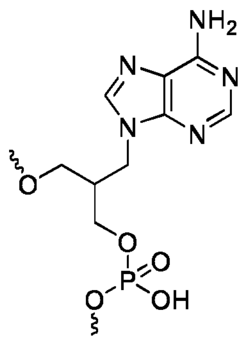
(-)hmpNA(A)

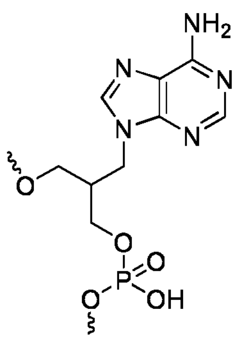
(+)hmpNA(A)

wherein the nucleotide synthesized using 2-hydroxymethyl-1,3-propanediol as the starting material was defined as hmpNA;
(+)hmpNA(A) was obtained by solid-phase synthesis using the nucleoside phosphoramidite monomer 1-1b of example section 1.1, and its absolute configuration
was (S)-hmpNA(A); (-)hmpNA(A) was obtained by solid-phase synthesis using the nucleoside phosphoramidite

monomer 1-1a of example section 1.1, and its absolute configuration was (R)-hmpNA(A).

Similarly, the following structures were obtained by solid-phase synthesis and by changing the base species of hmpNA, and their absolute configurations were determined:

(+)hmpNA(G), with the absolute configuration (S)-hmpNA(G);
(-)hmpNA(G), with the absolute configuration (R)-hmpNA(G);
(+)hmpNA(C), with the absolute configuration (S)-hmpNA(C);
(-)hmpNA(C), with the absolute configuration (R)-hmpNA(C);
(+)hmpNA(U), with the absolute configuration (R)-hmpNA(U); and
(-)hmpNA(U), with the absolute configuration (S)-hmpNA(U).

The absolute configurations (S)-hmpNA(G), (R)-hmpNA(G), (S)-hmpNA(C), (R)-hmpNA(C), (S)-hmpNA(U), and (R)-hmpNA(U) were determined from their intermediates or derivatives by X-Ray diffraction.
The structures of the intermediates or derivatives are:

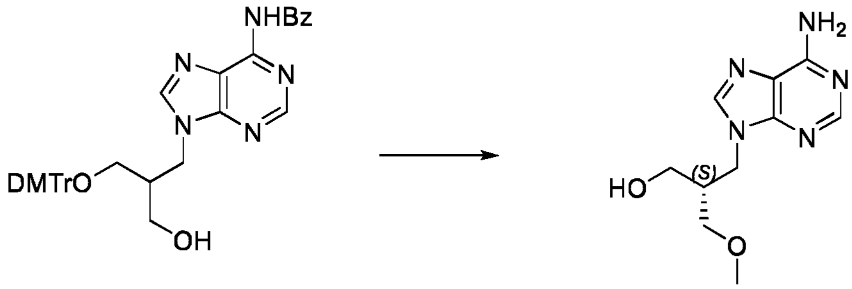

**15.1 6B(+)** **TJ-NA067**

TJ-NA067: determined as a colorless massive crystal (0.30 × 0.10 × 0.04 mm3), belonging to the monoclinic system with a P21 space group. Lattice parameter a = 16.0496(5) Å, b = 4.86260(10) Å, c = 16.4686(5) Å, $\alpha$ = 90°, $\beta$ = 118.015(4)°, $\gamma$ = 90°, V = 1134.65(7) Å3, Z = 4. Calculated density Dc = 1.389 g/cm3; the number of electrons in a unit cell F(000) = 504.0; linear absorption coefficient of a unit cell $\mu$ (Cu K$\alpha$) = 0.840 mm-1; diffraction experiment temperature T = 150.00(11) K.

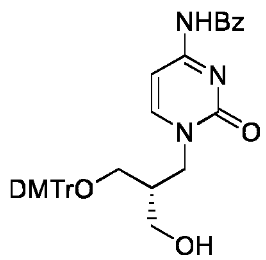

**15.8 6A (+)**

6A(+): determined as a colorless massive crystal (0.30 × 0.20 × 0.10 mm3), belonging to the monoclinic system with a P21 space group. Lattice parameter a = 22.6688(7) Å, b = 8.5595(2) Å, c = 23.3578(5) Å, $\alpha$ = 90°, $\beta$ = 113.876(3)°, $\gamma$= 90°, V = 4144.3(2) Å3, Z = 2. Calculated density Dc = 0.999 g/cm3; the number of electrons in a unit cell F(000) = 1318.0; linear absorption coefficient of a unit cell $\mu$ (Cu K$\alpha$) = 0.570 mm-1; diffraction experiment temperature T = 100.01(18) K.

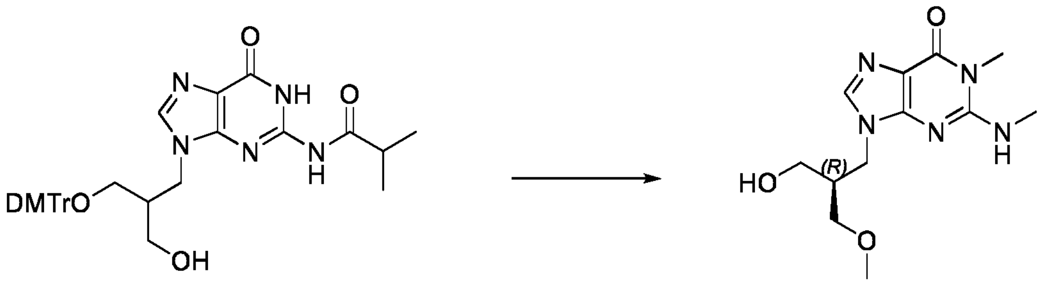

**15.6 7A (-)** **TJ-NA068**

**TJ-NA048:** determined as a colorless acicular crystal (0.30 × 0.04 × 0.04 mm3), belonging to the monoclinic system with a P1 space group. Lattice parameter a = 7.6165(4) Å, b = 11.3423(5) Å, c = 17.3991(8) Å, $\alpha$ = 85.007(4)°, $\beta$ = 88.052(4)°, $\gamma$ = 70.532(4)°, V = 1411.75(12) Å3, Z = 2. Calculated density Dc = 1.366 g/cm3; the number of electrons in a unit cell F(000) = 620.0; linear absorption coefficient of a unit cell $\mu$ (Cu K$\alpha$) = 0.856 mm-1; diffraction experiment temperature T = 150.00(13) K.

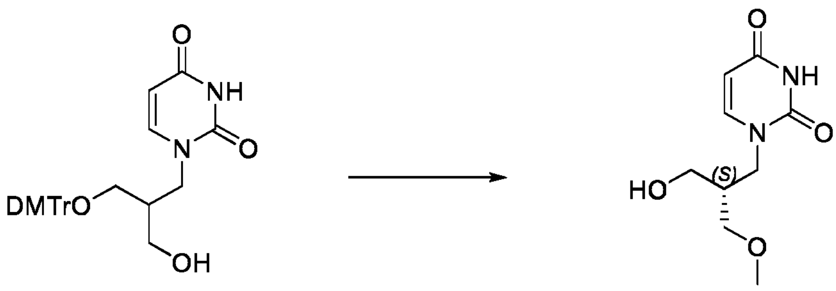

**15.7 6A (-)** **TJ-NA092**

TJ-NA092: determined as a colorless prismatic crystal (0.30 × 0.10 × 0.10 mm3), belonging to the triclinic system with a P1 space group. Lattice parameter a = 5.17960(10) Å, b = 8.0667(2) Å, c = 12.4077(2) Å, $\alpha$ = 93.146(2)°, $\beta$ = 101.266(2)°, $\gamma$ = 96.134(2)°, V = 503.993(18) Å3, Z = 2. Calculated density Dc = 1.412 g/cm3; the number of electrons in a unit cell F(000) = 228.0; linear absorption coefficient of a unit cell $\mu$ (Cu K$\alpha$) = 0.945 mm-1; diffraction experiment temperature T = 100.00(10) K.

**Example 5. Sequence-Dependence Experiment of siRNAs Comprising Different Chemical Modifications**

The experimental compounds of the present disclosure were tested on multiple different sequences. siRNAs targeting the mRNAs of different genes were used, and position 7 of the 5' end of the AS strands was modified using **(+)hmpNA(A), (-)hmpNA(A),** and the compound GNA(A), which was used as a control (the sequences are shown in Table 4-1 and Table 4-2).

Table 4-1. siRNA sense strands targeting different genes

| **siRNA target gene** | SS strand 5'-3' |
|---|---|
| ANGPTL3 | GmsAmsAmCmUfAmCfUfCfCmCmUmUmUmCmUmUmCmAm (SEQ ID NO:68) |
| HBV-S | CmsCmsAmUmUfUmGfUfUfCmAmGmUmGmGmUmUmCmsGm (SEQ ID NO:69) |
| HBV-X | CmsAmsCmCmUfCmUfGfCfAmCmGmUmCmGmCmAmUmsGm (SEQ ID NO:70) |

Table 4-2. siRNA antisense strands targeting different genes and comprising chemical modifications

| Target mRNA | siRNA double strand No. | AS strand modification (SEQ ID NO:) 5'-3' |
|---|---|---|
| ANGPTL3 | TRD5841 | UmsGfsAmAfGmAfGNA(A)AmGfGmGmAfGmUfAmGf UmUfCmsUmsUm (SEQ ID NO:71) |
| | TRD5845 | UmsGfsAmAfGmAf**(+)hmpNA(A)**AmGfGmGmAfGmUfAmGf UmUfCmsUmsUm (SEQ ID NO:72) |
| | TRD5846 | UmsGfsAmAfGmAf**(-)hmpNA(A)**AmGfGmGmAfGmUfAmGf UmUfCmsUmsUm (SEQ ID NO:73) |
| HBV-S | TRD5848 | UmsGfsAmAmCmCfGNA(A)CmUmGmAmAmCmAfAm AfUmGmGmsCmsAm (SEQ ID NO:74) |
| | TRD5852 | UmsGfsAmAmCmCf**(+)hmpNA(A)**CmUmGmAmAmCmAfAmAf UmGmGmsCmsAm (SEQ ID NO:75) |
| | TRD5853 | UmsGfsAmAmCmCf**(-)hmpNA(A)**CmUmGmAmAmCmAfAmAf UmGmGmsCmsAm (SEQ ID NO:76) |
| HBV-X | TRD5855 | UmsAfsUmGfCmGfGNA(A)CmGfUmGmCfAmGfAmGfGm UfGmsAmsAm (SEQ ID NO:77) |
| | TRD5859 | UmsAfsUmGfCmGf**(+)hmpNA(A)**CmGfUmGmCfAmGfAmGfGmUf GmsAmsAm (SEQ ID NO:78) |
| | TRD5860 | UmsAfsUmGfCmGf**(-)hmpNA(A)**CmGfUmGmCfAmGfAmGfGmUf GmsAmsAm (SEQ ID NO:79) |

The results of the on-target activity experiment are shown in Table 5. GNA(A) showed significant sequence dependence, and different sequences had significantly different on-target activity. The experimental compounds of the present disclosure did not show significant sequence dependence, which indicates that they are more universally applicable.

Table 5. On-target activity results of siRNAs for different target sequences

| Double strand code | Remaining percentage of target gene mRNA (on-target activity) expression (mean) | | | | | | | | | | | |
|---|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM | IC50 value (nM) |
| TRD 5841 | 57.5% | 51.1% | 55.5% | 68.3% | 76.5% | 85.9% | 82.9% | 87.8% | 81.5% | 64.0% | 97.8% | >40 |
| TRD 5845 | 28.2% | 30.5% | 41.7% | 55.0% | 63.9% | 78.0% | 77.1% | 84.1% | 95.8% | 83.2% | 91.9% | 1.9953 |
| TRD 5846 | 31.6% | 26.8% | 34.1% | 59.1% | 84.8% | 102.1% | 97.2% | 108.9% | 95.6% | 107.2% | 102.1% | 1.9055 |
| TRD 5848 | 46.5% | 35.1% | 26.6% | 36.0% | 67.3% | 76.3% | 88.4% | 104.1% | 91.6% | 95.1% | 98.1% | 0.7943 |
| TRD 5852 | 24.7% | 17.5% | 13.1% | 21.1% | 40.5% | 64.1% | 84.3% | 94.5% | 88.4% | 100.2% | 95.1% | 0.2951 |

(continued)

| Double strand code | Remaining percentage of target gene mRNA (on-target activity) expression (mean) | | | | | | | | | | | |
|---|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM | IC50 value (nM) |
| TRD 5853 | 17.5% | 11.5% | 9.9% | 13.5% | 30.3% | 54.5% | 74.6% | 86.3% | 90.3% | 91.0% | 84.1% | 0.1905 |
| TRD 5855 | 41.3% | 40.7% | 36.9% | 73.6% | 71.7% | 87.0% | 89.0% | 85.8% | 94.9% | 104.4% | 101.6% | 4.2658 |
| TRD 5860 | 43.5% | 37.1% | 34.1% | 50.8% | 77.6% | 88.5% | 86.6% | 100.0% | 95.1% | 97.8% | 110.8% | 1.5488 |

The experimental results of the off-target activity are shown in Table 6. It can be seen that the experimental compounds of the present disclosure significantly reduced the off-target activity of siRNA relative to the parent sequences.

Table 6. Off-target activity results of siRNAs for different target sequences

| Double strand code | Remaining percentage of target gene mRNA (off target activity) expression (mean) | | | | | | | | | | |
|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM |
| TRD 5855 | 71.1% | 78.2% | 81.6% | 92.0% | 91.0% | 94.1% | 87.3% | 93.6% | 99.4% | 119.9% | 96.6% |
| TRD 5859 | 79.8% | 81.0% | 86.0% | 96.4% | 101.9% | 98.8% | 99.8% | 118.4% | 101.3% | 93.3% | 103.2% |
| TRD 5860 | 78.4% | 75.6% | 80.6% | 86.1% | 83.2% | 95.9% | 91.6% | 91.5% | 95.6% | 97.3% | 98.6% |

**Example 6. Evaluation of Different Modifications at Position 9 and Position 10 of AS strands**

In this experiment, the *in vivo* inhibition efficiency of the siRNA conjugates of the present disclosure with 2'-fluoro modifications at different sites against the target gene mRNA expression level was investigated.

Male C57BL/6 mice aged 6-8 weeks were randomized into groups of 6, 3 mice per time point, and each group of mice was given test conjugates (2 conjugates, TRD007047 and TRD006870), a control conjugate (TRD002218), and PBS. All the animals were dosed once by subcutaneous injection based on their body weight. The siRNA conjugates were administered at a dose of 1 mg/kg (calculated based on siRNA) in a volume of 5 mL/kg. The mice were sacrificed 7 days after administration, and their livers were collected and stored with RNA later (Sigma Aldrich). Then the liver tissue was homogenized using a tissue homogenizer, and the total RNA was extracted from the liver tissue using a tissue RNA extraction kit (FireGen Biomedicals, FG0412) by following the procedure described in the instructions. The total RNA was reverse-transcribed into cDNA, and the TTR mRNA expression level in liver tissue was measured by real-time fluorescence quantitative PCR. In the fluorescence quantitative PCR method, the glyceraldehyde 3-phosphate dehydrogenase (GAPDH) gene was used as an internal reference gene, and the TTR and GAPDH mRNA expression levels were measured using Taqman probe primers for TTR and GAPDH, respectively. The compounds are shown in Table 7, the compound groups for the *in vivo* experiment on mice are shown in Table 8, and the primers are shown in Table 9.

Unless otherwise specified, the sequences listed in the following tables and elsewhere in the specification are all in the 5' to 3' direction.

Table 7. Modification of position 9 and position 10 of AS strands

| | SS strand (SEQ ID NO:) | AS strand (SEQ ID NO:) |
|---|---|---|
| TRD002218 | CmsAmsGmUmGfUmUfCfUfUmGmC mUmCmUmAmUmAmAm-L96 (SEQ ID NO:80) | UmsUfsAmUmAmGfAmGmCmAmAmGm AmAfCmAfCmUmGmsUmsUm (SEQ ID NO:83) |
| TRD007047 | CmsAmsGmUmGfUmUfCfUfUmGmC mUmCmUmAmUmAmsAms-NAG1 (SEQ ID NO:81) | UmsUfsAmUfAmGf**(-)hmpNA(A)**GmCfAm AmGfAmAfCmAfCmUfGmsUmsUm SEQ ID NO:84) |
| TRD006870 | CmsAmsGmUmGfUmUfCfUfUmGmC mUmCmUmAmUmAmsAms-NAG1 (SEQ ID NO:82) | UmsUfsAmUfAmGf**(-)hmpNA(A)**GmCmAf AmGfAmAfCmAfCmUfGmsUmsUm (SEQ ID NO:85) |

The structure of NAG1 is:

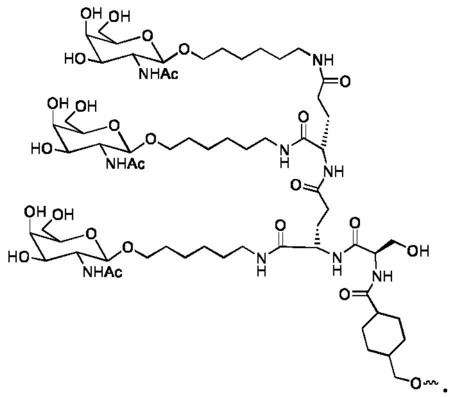

The compound NAG1 was prepared by following the method described in the patent WO2021254360A1; the structure of L96 is:

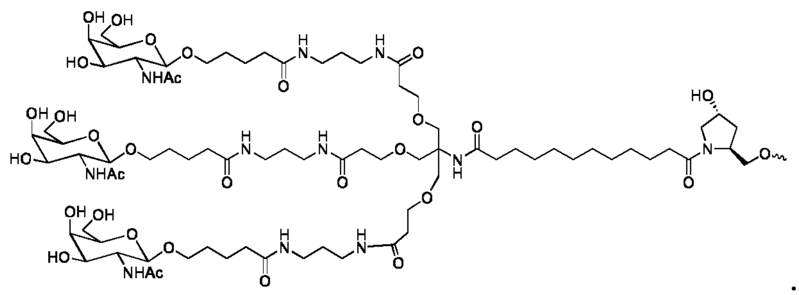

The control compound L96 was prepared by following the method described in the patent WO2014025805A1.

Table 8. The groups for the *in vivo* experiment on mice

| No. | Dose | mRNA quantification | Number of animals | Note |
|---|---|---|---|---|
| PBS | - | D7, 28 | 6 | 3 mice per time point |
| TRD002218 | 1mpk s.c. | D7, 28 | 6 | 3 mice per time point |
| TRD007047 | 1mpk s.c. | D7, 28 | 6 | 3 mice per time point |
| TRD006870 | 1mpk s.c. | D7, 28 | 6 | 3 mice per time point |

Table 9. The sequences of detection primers

| Primer name | Forward primer |
|---|---|
| mTTR-F | GGGAAGACCGCGGAGTCT (SEQ ID NO:86) |
| mTTR-R | CAGTTCTACTCTGTACACTCCTTCTACAAA (SEQ ID NO:87) |
| mTTR-P | 5'6-FAM-CTGCACGGGCTCACCACAGATGA-3'BHQ1 (SEQ ID NO:88) |
| mGAPDH-F | CGGCAAATTCAACGGCACAG (SEQ ID NO:89) |
| mGAPDH-R | CCACGACATACTCAGCACCG (SEQ ID NO:90) |
| mGAPDH-P | 5'TET-ACCATCTTCCAGGAGCGAGACCCCACT-3'BHQ2 (SEQ ID NO:91) |

The *in vivo* inhibition efficiency of the siRNA conjugates of the present disclosure with fluoro modifications at different sites against the target gene mRNA expression level 28 days after administration was shown in Table 10. The siRNA conjugates with fluoro modifications at different sites inhibited more TTR mRNA expression than the reference positive control TRD002218 28 days after administration. Both the modification methods showed high inhibition efficiency and the inhibitory effects were not significantly different, which indicates that both the AS strand position 9 modification and position 10 modification methods can mediate higher inhibition efficiency.

Table 10. 7-day and 28-day test results

| | | 7 days | | 28 days | |
|---|---|---|---|---|---|
| 9/10F | No. | Remaining mRNA | SD | Remaining mRNA | SD |
| | PBS | 100% | 11% | 100% | 9% |
| PC | TRD002218 | 31% | 7% | 49% | 5% |
| mTTR 9F | TRD007047 | 15% | 5% | 39% | 10% |
| mTTR 10F | TRD006870 | 13% | 4% | 36% | 3% |

The TTR mRNA expression level was calculated according to the equation below: TTR mRNA expression level = [(TTR mRNA expression level of test group/GAPDH mRNA expression level of test group)/(TTR mRNA expression level of control group/GAPDH mRNA expression level of control group)] $\times$ 100%

**Part Two. Targeting Ligands**

**Example 7. Preparation of NAG0052 and L96**

The compounds NAG0024 and NAG0026 were purchased from WuXi AppTec (Tianjin) Co. Ltd. Unless otherwise specified, all reagents used in the following examples were commercially available.

**(1) Synthesis of compound NAG0052**

The starting material compound 1 was purchased from Jiangsu Beida Pharmatech Ltd.

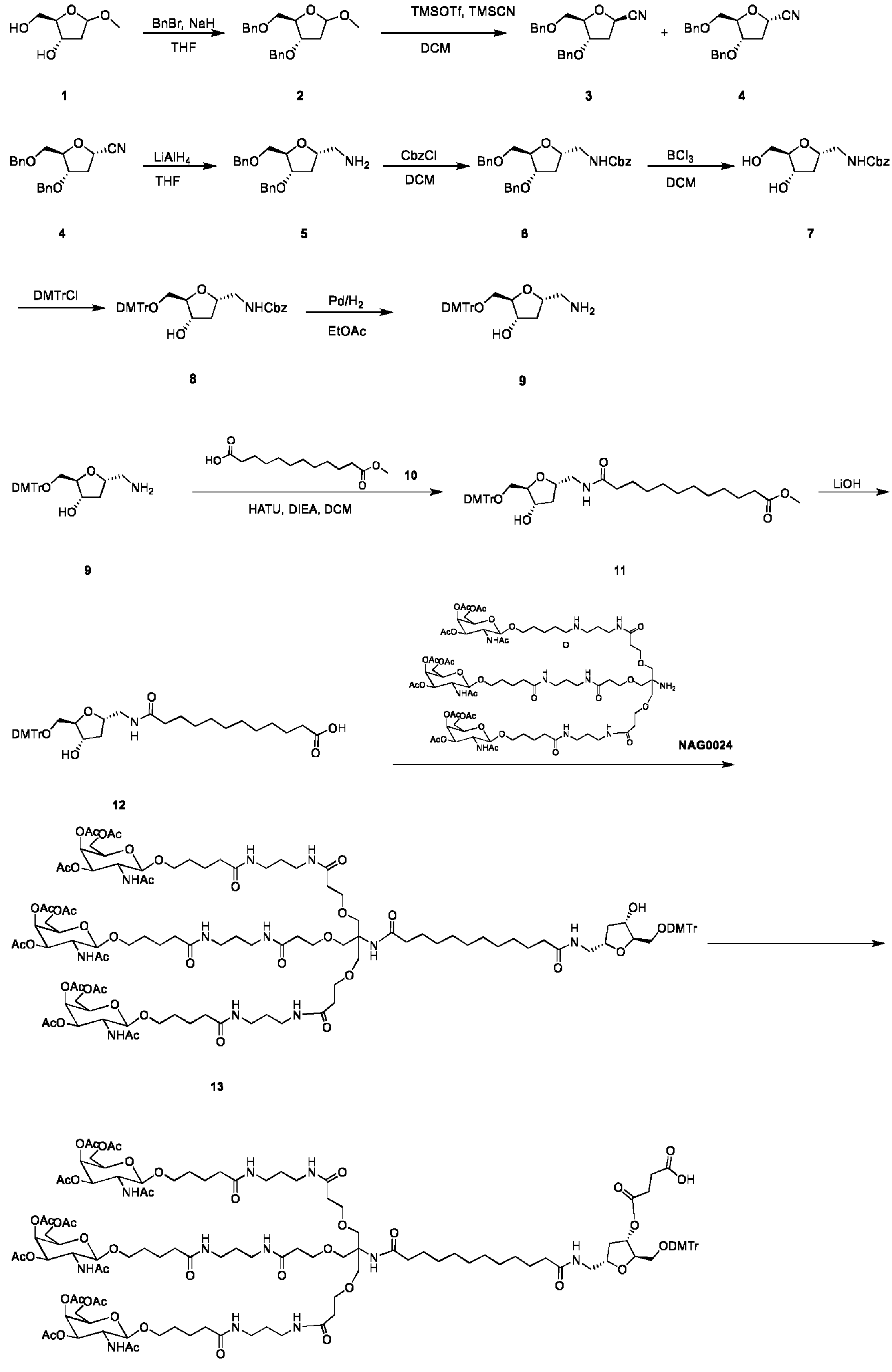


NAG0052

The specific preparation procedure of NAG0052 is as follows:

**Compound 2**

NaH (12.2 g, 304 mmol, purity 60%) was added in batches to a solution of compound 1 (12.3 mL, 101 mmol) in THF (300 mL) at 0 °C in a nitrogen atmosphere. The mixture was stirred at 20 °C for 1 h and then cooled to 0 °C again. Then benzyl bromide (36.3 mL, 304 mmol) was added dropwise to the system, and the mixture was stirred at 20 °C for 12 h. The reaction mixture was quenched with $H_2O$ (100 mL) and then extracted with EtOAc (200 mL × 2). The combined organic phases were washed with saturated brine (100 mL), dried over $Na_2SO_4$, filtered, and concentrated, and the resulting residue was separated by silica gel column chromatography to give the target compound **2** (20.0 g, 51.8 mmol, yield 51%).

**LCMS:** $t_R$ = 2.615 and 2.820 min in 30-90AB_7 min_220&254_Shimadzu.lcm (Xtimate C18, 3um, 2.1*30mm), MS (ESI) m/z = 351.2 $[M+Na]^+$.

**$^1$H NMR:** (400 MHz, $CDCl_3$) $\delta$ ppm 7.35-7.12 (m, 10H), 5.06-4.95 (m, 1H), 4.51-4.39 (m, 4H), 4.24-3.87 (m, 2H), 3.50-3.40 (m, 2H), 3.38-3.20 (m, 3H), 2.20-1.91 (m, 2H).

**Compound 3 and Compound 4**

TMSCN (13.5 mL, 101 mmol) was added in one go to a solution of compound 2 (13.0 g, 33.6 mmol) in DCM (300 mL) at 20 °C in a nitrogen atmosphere, and a solution of TMSOTf(9.14 mL, 50.5 mmol) in DCM (30 mL) was then added dropwise. The reaction mixture was stirred at 20 °C for 15 h. After the reaction was complete, the system was quenched with saturated aqueous $NaHCO_3$ solution (80 mL) and extracted with DCM (150 mL × 2). The combined organic phases were washed with saturated brine (80 mL), dried over $Na_2SO_4$, filtered, and concentrated. The residue was separated by silica gel column chromatography to give the target compound 3 (3.30 g, 9.18 mmol, yield 27%) and compound 4 (8.50 g, 9.18 mmol, yield 70%) as a pale yellow oily liquid.

**Compound 3**

$^1$H **NMR:** (400 MHz, $CDCl_3$) $\delta$ ppm 7.42-7.29 (m, 10H), 4.81 (t, *J* = 7.8 Hz, 1H), 4.65-4.49 (m, 4H), 4.30-4.21 (m, 2H), 3.65-3.57 (m, 1H), 3.57-3.49 (m, 1H), 2.49-2.40 (m, 2H).

**Compound 4**

$^1$H **NMR:** (400 MHz, $CDCl_3$) $\delta$ ppm 7.42-7.26 (m, 10H), 4.93-4.87 (m, 1H), 4.65-4.48 (m, 4H), 4.43-4.38 (m, 1H), 4.21-4.17 (m, 1H), 3.79-3.70 (m, 1H), 3.54 (d, *J* = 4.0 Hz, 1H), 2.45-2.37 (m, 2H).

**Compound 5**

A solution of compound **4** (3.00 g, 9.28 mmol) in THF (15 mL) was added dropwise to a solution of $LiAlH_4$ (0.79 g, 20.9 mmol) in THF (15 mL) at 0 °C in a nitrogen atmosphere. After the dropwise addition, the system was left to react at 0 °C for 1 h. TLC monitoring (PE:EtOAc = 3:1) showed the starting material was completely consumed. Sodium sulfate decahydrate was slowly added to the reaction mixture until no more bubbles were produced. The reaction mixture was then filtered, and the filter cake was washed three times with dichloromethane (60 mL). The filtrate was collected and concentrated to dryness by rotary evaporation to give compound **5** (3.00 g, yield 90%).

**$^1$H NMR:** (400 MHz, DMSO-$d_6$) $\delta$ ppm 7.40-7.14 (m, 10H), 4.54-4.38 (m, 4H), 4.06-3.99 (m, 2H), 3.91 (q, *J*= 6.4 Hz, 1H), 3.48-3.37 (m, 2H), 2.67-2.52 (m, 2H), 2.21-2.18 (m, 1H), 1.77-1.73 (m, 1H).

**Compound 6**

Compound **5** (3.00 g, 8.25 mmol) was dissolved in DCM (30 mL) in a nitrogen atmosphere, and TEA (3.44 mL, 24.7 mmol) and CbzCl (1.76 mL, 12.4 mmol) were added. The mixture was left to react at 20 °C for 2 h. LCMS analysis showed the reaction had been complete. The reaction mixture was added to dichloromethane (30 mL) and water (60 mL) for extraction. The organic phase was washed three times with water (60 mL × 3), dried over anhydrous sodium sulfate, and concentrated, and the residue was purified using a normal-phase column (PE:EtOAc = 1:1) to give the target compound 6 (2.5 g, yield 90%).

**LCMS:** $t_R$ = 0.810 min in 5-95AB_1min, MS (ESI) m/z =462.2 $[M+H]^+$.

**$^1$H NMR:** (400 MHz, $CDCl_3$) $\delta$ ppm 7.39-7.29 (m, 15H), 5.35 (s, 1H), 5.15-5.01 (m, 2H), 4.72 (d, *J*= 6.0 Hz, 1H), 4.54-4.40 (m, 3H), 4.26 (s, 1H), 4.23-4.18 (m, 1H), 4.11-4.04 (m, 1H), 3.54-3.41 (m, 3H), 3.37-3.25 (m, 1H), 2.34-2.23 (m, 1H), 1.85-1.79 (m, 1H).

**Compound 7**

Compound 6 (2.00 g, 3.90 mmol) was dissolved in DCM (5 mL) in a nitrogen atmosphere, and a solution of $BCl_3$ in THF (1 M, 27.3 mL) was added at -78 °C. The mixture was left to react for 1 h. TLC monitoring (DCM:MeOH = 10:1) showed the starting material was completely consumed. The reaction mixture was quenched with methanol (20 mL) at - 78 °C and concentrated, and the residue was purified using a normal-phase column (DCM:MeOH = 10:1) to give the target compound **7** (2.00 g, yield 60%).

**$^1$H NMR:** (400 MHz, $CD_3OD$) δ ppm 7.41-7.23 (m, 5H), 5.08 (s, 2H), 4.25-4.07 (m, 2H), 3.85-3.75 (m, 1H), 3.63-3.56 (m, 1H), 3.54-3.48 (m, 1H), 3.30-3.27 (m, 2H), 2.34-2.21 (m, 1H), 1.71-1.64 (m, 1H).

**Compound 8**

Compound 7 (0.50 g, 1.78 mmol) was dissolved in pyridine (5 mL) in a nitrogen atmosphere, and 4A molecular sieve (500 mg) and DMTrCl (0.66 mL, 2.13 mmol) were added at 0 °C. Then the mixture was warmed to 20 °C and left to react for 1.5 h. TLC monitoring (PE:EtOAc = 2:1) showed the starting material was completely consumed. The reaction mixture was added to ethyl acetate (60 mL) and water (60 mL) for extraction. The organic phase was washed three times with water (60 mL × 3), then dried over anhydrous sodium sulfate, and concentrated, and the residue was purified using a normal-phase column (PE:EtOAc = 1:1) to give the target compound **8** (800 mg, yield 90%).

**$^1$H NMR:** (400 MHz, $CDCl_3$) δ ppm 7.44 (d, J = 7.6 Hz, 2H), 7.37-7.23 (m, 11H), 7.22-7.15 (m, 1H), 6.84 (d, J = 8.8 Hz, 4H), 5.09 (s, 2H), 4.31-4.17 (m, 2H), 4.02-3.91 (m, 1H), 3.84-3.73 (m, 6H), 3.33 (s, 1H), 3.28 (s, 1H), 3.19-3.01 (m, 2H), 2.34-2.25 (m, 1H), 1.70-1.62 (m, 1H).

**Compound 9**

Compound **8** (800 mg, 1.234 mmol) was dissolved in EtOAc (5 mL), and Pd/C 10% (800 mg, 7.517 mmol) was added. The mixture was left to react in a $H_2$ atmosphere (15 Psi) at 20 °C for 1 h. LCMS analysis showed the reaction had been complete. The reaction mixture was filtered, and the filter cake was washed three times with dichloromethane (100 mL) and methanol (100 mL). The filtrate was concentrated, and the residue was separated using a reversed-phase column to give compound **9** (300 mg, 54%).

**LCMS:** $t_R$ = 2.586 min in 10-80CD_3min MS (ESI) m/z = 450.2 $[M+H]^+$.

**Compound 11**

Compound **10** (435 mg, 1.780 mmol) was dissolved in DCM (10 mL), and DIEA (0.441 mL, 2.67 mmol) and HATU (677 mg, 1.78 mmol) were added. Then compound **9** (400 mg, 0.890 mmol) was added. The mixture was left to react at 20 °C for 1 h. TLC monitoring (DCM:MeOH = 10:1) showed the reaction had been complete. The reaction mixture was added to dichloromethane (60 mL) and water (60 mL) for extraction. The organic phase was washed three times with water (60 mL × 3), dried over anhydrous sodium sulfate, and concentrated, and the residue was purified using a normal-phase column (elution with PE:EtOAc = 0:1, product peak at 100%) to give the target compound **11** (600 mg, yield 90%).

**LCMS:** $t_R$ = 2.745 min in 30-90CD_3min, MS (ESI) m/z =698.4 $[M+Na]^+$.

**$^1$H NMR:** (400 MHz, $CD_3OD$) δ ppm 7.46-7.38 (m, 2H), 7.35-7.24 (m, 6H), 7.22-7.16 (m, 1H), 6.90-6.78 (m, 4H), 4.29-4.21 (m, 2H), 4.02-3.95 (m, 1H), 3.77 (s, 6H), 3.66-3.62 (m, 3H), 3.41 (s, 1H), 3.18-3.04 (m, 2H), 2.36-2.17 (m, 5H), 1.71-1.50 (m, 5H), 1.39-1.25 (m, 14H).

**Compound 12**

Compound 11 (600 mg, 0.799 mmol) was dissolved in THF (3 mL) and $H_2O$ (1 mL), and LiOH.HzO (134 mg, 3.20 mmol) was added. The mixture was left to react at 20 °C for 12 h. TLC analysis (DCM:MeOH = 10:1) showed the reaction had been complete. The reaction mixture was concentrated to dryness by rotary evaporation. The residue was dissolved in water (5 mL) and methanol (5 mL) and purified using a reversed-phase column ($H_2O$:$CH_3CN$ = 1:1, peak at around 35%) to give the target compound **12** (460 mg, yield 100%, lithium salt).

**LCMS:** $t_R$ = 1.346 min in 10-80CD_3min, MS (ESI) m/z = 684.3 $[M+Na]^+$.

**HPLC:** $t_R$ = 1.879 min in 10-80CD_6min.

**$^1$H NMR:** (400 MHz, $CD_3OD$) δ ppm 7.47-7.39 (m, 2H), 7.35-7.24 (m, 6H), 7.22-7.15 (m, 1H), 6.91-6.79 (m, 4H), 4.31-4.18 (m, 2H), 4.02-3.95 (m, 1H), 3.78 (s, 6H), 3.44-3.33 (m, 2H), 3.18-3.04 (m, 2H), 2.35-2.27 (m, 1H), 2.24-2.10 (m, 4H), 1.70-1.51 (m, 5H), 1.31-1.23 (m, 12H).

**Compound 13**

The compound **NAG0024** (271 mg, 0.151 mmol) was dissolved in anhydrous THF (2 mL) and anhydrous DMF (4 mL) at room temperature in a nitrogen atmosphere, and 3A molecular sieve was added. Then compound **12** (100 mg, 0.151 mmol), HOBt (25 mg, 0.181 mmol), DCC (38 mg, 0.181 mmol), and DIEA (39 mg, 0.302 mmol) were sequentially added. The reaction mixture was left to react at 45 °C for 16 h. After LC-MS analysis showed the reaction had been complete, the reaction mixture was quenched with water and filtered. The filtrate was concentrated, and the residue was then purified using a C18 reversed-phase column ($H_2O$/MeCN) to give compound **13** (210 mg, yield 57%).

**Compound NAG0052**

Compound **13** (230 mg, 0.094 mmol) was dissolved in pyridine (5 mL) at room temperature, and a molecular sieve was added. DMAP (12 mg, 0.283 mmol) and succinic anhydride (28 mg, 0.283 mmol) were added. The reaction mixture was stirred at 50 °C for 16 h in a nitrogen atmosphere. LCMS analysis showed the reaction had been complete. The reaction mixture was filtered and concentrated to dryness by rotary evaporation. After purification on a C18 reversed-phase column, secondary purification was performed by preparative HPLC to give the target compound NAG0052 (123 mg, 0.048 mmol, yield 51%).

MS (ESI) m/z = 2535.3 $[M-1]^-$. Calculated: 2536.2.

$^1H$ NMR (400 MHz, acetonitrile-$d_3$) $\delta$ 7.48-7.43 (m, 2H), 7.37-7.12 (m, 11H), 7.00-6.85 (m, 10H), 6.66 (s, 1H), 5.31 (dd, *J* = 3.4, 1.1 Hz, 3H), 5.20-5.13 (m, 1H), 5.05 (dd, *J* = 11.3, 3.4 Hz, 3H), 4.56 (d, *J* = 8.5 Hz, 3H), 4.30 (dd, *J* = 7.7, 5.3 Hz, 1H), 4.18-3.93 (m, 14H), 3.79 (s, 10H), 3.65 (q, *J* = 4.7, 3.6 Hz, 13H), 3.56-3.07 (m, 24H), 2.56 (s, 6H), 2.37 (t, *J*= 5.8 Hz, 10H), 2.17 (t, *J*= 7.5 Hz, 9H), 2.02-1.96 (m, 20H), 1.88 (s, 8H), 1.82-1.73 (m, 2H), 1.60 (dt, *J*= 15.0, 7.3 Hz, 16H), 1.27 (s, 13H).

**Example 8. Synthesis of siRNA Conjugates**

1. In-house preparation of resin with support

The carboxylic acid group-containing compound **NAG0052** (157 mg, 0.062 mmol) was dissolved in anhydrous DMF (3 mL). After the substrate was completely dissolved, anhydrous acetonitrile (4 mL), DIEA (0.03 mL, 0.154 mmol, 2.5 eq), and HBTU (35 mg, 0.093 mmol, 1.5 eq) were sequentially added. After the reaction mixture was mixed well, macroporous aminomethyl resin (476 mg, blank loading 0.41 mmol/g, target loading 0.1 mmol/g) was added. The reaction mixture was shaken overnight on a shaker (temperature: 25 °C; rotational speed: 200 rpm). The reaction mixture was filtered, and the filter cake was washed with DCM and then with anhydrous acetonitrile. The solid was collected and dried overnight under vacuum.

The solid from the previous step was dispersed in anhydrous acetonitrile (5 mL), and pyridine (0.18 mL), DMAP (3 mg), NMI (0.12 mL), and CapB1 (2.68 mL) were sequentially added. The reaction mixture was shaken on a shaker (temperature: 25 °C; rotational speed: 200 rpm) for 2 h. The reaction mixture was filtered, and the filter cake was washed with anhydrous acetonitrile. The solid was collected and dried overnight under vacuum to give a resin with a support. The loading was measured at 0.1 mmol/g. 2. For NAG0052 that had been attached to the resin, the resin was used as a start, and nucleoside monomers were attached one by one in the 3'-5' direction in the order in which nucleotides were arranged. Each time a nucleoside monomer was attached, four reactions, i.e., deprotection, coupling, capping, and oxidation or sulfurization, were involved. The compound NAG0052 was attached to the sequence through solid-phase synthesis, and after aminolysis, part of functional groups of the structure of NAG0052 were removed, thus forming NAG0052'.

The prepared siRNA conjugates had sense and antisense strands as shown in Table 11 and Table 12.

Table 11. siRNA conjugates

| siRNA conjugate No. | Sense strand No. | Antisense strand No. |
|---|---|---|
| TRD002218 | TJR4373-SS | TJR0414-AS |
| TRD007205 | TJR013485S | TJR0414-AS |

Table 12. The nucleic acid sequences of the sense and antisense strands

| | No. | **Sequence direction 5'-3'** (SEQ ID NO:) |
|---|---|---|
| **Sense strand** | TJR4373-SS | CmsAmsGm UmGfUm UfCfUf UmGmCm UmCmUm AmUmAm Am-**L96** (SEQ ID NO:92) |
| | TJR013485S | CmsAmsGm UmGfUm UfCfUf UmGmCm UmCmUm AmUmAms Ams-**NAG0052'** (SEQ ID NO:93) |
| **Antisense strand** | TJR0414-AS | UmsUfsAm UmAmGf AmGmCm AmAmGm AmAfCm AfCmUm GmsUmsUm (SEQ ID NO:94) |

Table 13. The structures of conjugates

| siRNA conjugate antisense strand No. | Structure |
|---|---|
| TJR4373-SS | 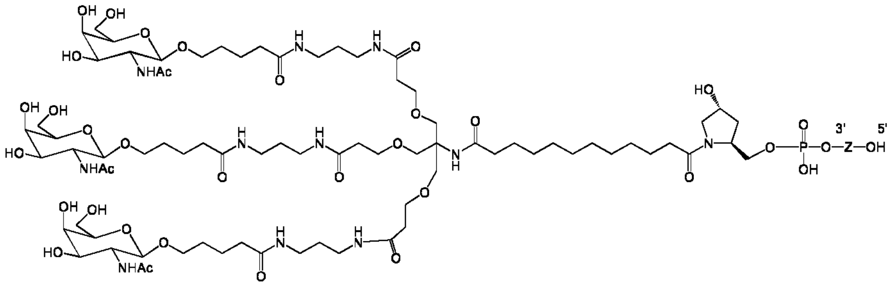 |
| TJR013485S | 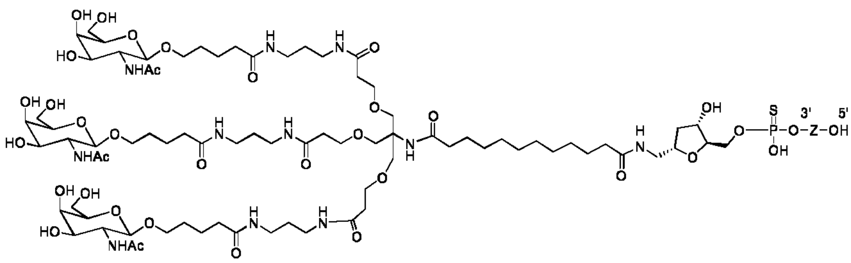 |

The conjugate TRD002218 was used as a reference positive compound, and Z represented siRNA.

### Example 9. *In Vivo* Inhibition of Target Gene mRNA Expression Level by siRNA Conjugates

In this experiment, the *in vivo* inhibition efficiency of the siRNA conjugates of the present disclosure that were conjugated with different structures against the target gene mRNA expression level was investigated.

Male C57BL/6 mice aged 6-8 weeks were randomized into groups of 6, 3 mice per time point, and each group of mice was given the conjugate of the present disclosure TRD007205, the reference positive nucleic acid ligand conjugate TRD002218, and PBS. All the animals were dosed once by subcutaneous injection based on their body weight. The siRNA conjugates were administered at a dose of 1 mg/kg (calculated based on siRNA) in a volume of 5 mL/kg. The mice were sacrificed 7 days or 28 days after administration, and their livers were collected and stored with RNA later (Sigma Aldrich). Then the liver tissue was homogenized using a tissue homogenizer, and the total RNA was extracted from the liver tissue using a tissue RNA extraction kit (FireGen Biomedicals, FG0412) by following the procedure described in the instructions. The total RNA was reverse-transcribed into cDNA, and the TTR mRNA expression level in liver tissue was measured by real-time fluorescence quantitative PCR. In the fluorescence quantitative PCR method, the glyceraldehyde 3-phosphate dehydrogenase (GAPDH) gene was used as an internal reference gene, and the TTR and GAPDH mRNA expression levels were measured using Taqman probe primers for TTR and GAPDH, respectively. The sequences of the detection primers were the same as those shown in Table 9.

Table 14. The compound groups for the *in vivo* experiment on mice

| Compound No. | Dose | mRNA quantification | Number of animals | Note |
|---|---|---|---|---|
| PBS | - | D7, 28 | 6 | 3 mice per time point |
| TRD002218 | 1mpk s.c. | D7, 28 | 6 | 3 mice per time point |
| TRD007205 | 1mpk s.c. | D7, 28 | 6 | 3 mice per time point |

The TTR mRNA expression level was calculated according to the equation below: TTR mRNA expression level = [(TTR mRNA expression level of test group/GAPDH mRNA expression level of test group)/(TTR mRNA expression level of control group/GAPDH mRNA expression level of control group)] × 100%.

The *in vivo* inhibition efficiency of the siRNA conjugates of the present disclosure that were conjugated with different structures against the target gene mRNA expression level 7 days and 28 days after administration is shown in FIG. 1 and FIG. 2, respectively.

As can be seen from the results in FIG. 1, the conjugate TRD007205 well inhibited TTR mRNA expression 7 days after administration, indicating that it can mediate more efficient delivery. As can be seen from FIG. 2, 28 days after administration, TRD007205 inhibited the target gene mRNA expression level better than TRD002218.

**Part Three. siRNAs Targeting LPA and Screening and Activity Verification of siRNA Conjugates**

**Example 10. Design and Synthesis of Human LPA siRNAs**

1) **siRNA design:** With the human LPA (NM_005577.3) as a target gene, siRNAs of 19/21 nt were designed in accordance with the general rules for active siRNAs. The sequences of unmodified sense strands and antisense strands are detailed in Table 15 and Table 16. In synthesizing the modified nucleotide at position 7 of the 5' end of the AS strand, the original nucleotide of the parent sequence was replaced with a phosphoramidite monomer synthesized in Example 1 or a 2'-methoxy-modified phosphamide monomer. The sequences of antisense strands subjected to AS strand modification at position 7 of the 5' end were detailed in Table 15, wherein W' is selected from the group consisting of a 2'-methoxy-modified nucleotide or a nucleotide comprising a chemical modification shown as

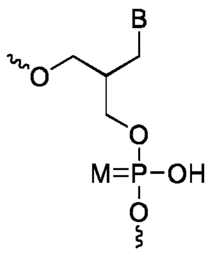 , 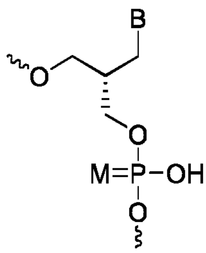 , or 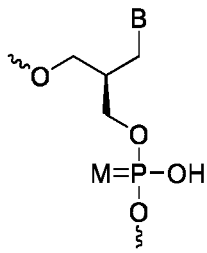

or a tautomeric modification thereof;

wherein: M is O or S; wherein: in SEQ ID NO: 5, when W' is selected from the group consisting of

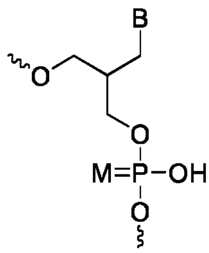 , 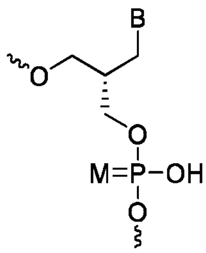 , or 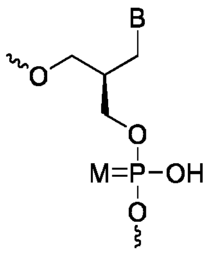 ,

B is selected from A; in SEQ ID NO: 6, when W' is selected from the group consisting of

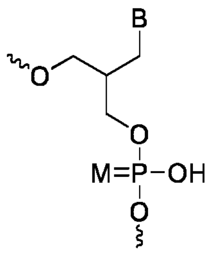,

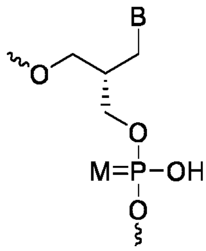, or 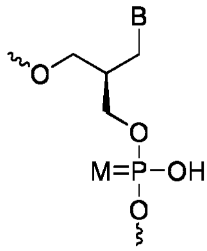,

B is selected from G;

Table 15. Unmodified sense and antisense strands of human LPA siRNAs; antisense strands with a chemical modification at position 7

| Double strand No. of naked sequences | SEQ ID NO: | Unmodified sense strand (5’-3’) | SEQ ID NO: | Unmodified antisense strand (5’-3’) | SEQ ID NO: | Antisense strand with modification at position 7 (5’-3’) corresponding to the naked sequence |
|---|---|---|---|---|---|---|
| TJR100373 | 1 | GCUCCUUAUUG UUAUACGA | 3 | UCGUAUAACAA UAAGGAGCUG | 5 | UCGUAUW'ACAA UAAGGAGCUG |
| TJR100366 | 2 | ACACCACAUCAA CAUAAUA | 4 | UAUUAUGUUGA UGUGGUGUCA | 6 | UAUUAUW'UUGA UGUGGUGUCA |

Table 16. Unmodified sense and antisense strands of human LPA siRNAs (control group)

| Double strand No. of naked sequences | SEQ ID NO: | Unmodified sense strand (5’-3’) | SEQ ID NO: | Unmodified antisense strand (5’-3’) |
|---|---|---|---|---|
| | 7 | GGCAGCUCCUUAUUGUUAU | 23 | AUAACAAUAAGGAGCUGCCAC |
| | 8 | AUGACACCACAUCAACAUA | 24 | UAUGUUGAUGUGGUGUCAUAG |
| | 9 | GACACCACAUCAACAUAAU | 25 | AUUAUGUUGAUGUGGUGUCAU |
| TJR100367 | 10 | CACCACAUCAACAUAAUAG | 26 | CUAUUAUGUUGAUGUGGUGUC |
| TJR100368 | 11 | ACCACAUCAACAUAAUAGG | 27 | CCUAUUAUGUUGAUGUGGUGU |
| TJR100369 | 12 | GACACCACAUCAACAUAAU | 28 | AUUAUGUUGAUGUGGUGUCAU |
| TJR100370 | 13 | UGACACCACAUCAACAUAA | 29 | UUAUGUUGAUGUGGUGUCAUA |
| TJR100371 | 14 | ACACCACAUCAACAUAAUAGG | 30 | UAUUAUGUUGAUGUGGUGUCA |
| TJR100372 | 15 | GACACCACAUCAACAUAAUAG | 31 | AUUAUGUUGAUGUGGUGUCAU |
| TJR100374 | 16 | CUCCUUAUUGUUAUACGAG | 32 | CUCGUAUAACAAUAAGGAGCU |

(continued)

| Double strand No. of naked sequences | SEQ ID NO: | Unmodified sense strand (5'-3') | SEQ ID NO: | Unmodified antisense strand (5'-3') |
|---|---|---|---|---|
| TJR100375 | 17 | UCCUUAUUGUUAUACGAGG | 33 | CCUCGUAUAACAAUAAGGAGC |
| TJR100376 | 18 | AGCUCCUUAUUGUUAUACG | 34 | CGUAUAACAAUAAGGAGCUGC |
| TJR100377 | 19 | CAGCUCCUUAUUGUUAUAC | 35 | GUAUAACAAUAAGGAGCUGCC |
| TJR100378 | 20 | GCGCCUUAUUGUUAUACGA | 36 | UCGUAUAACAAUAAGGCGCUG |
| TJR100379 | 21 | GCACCUUAUUGUUAUACGA | 37 | UCGUAUAACAAUAAGGUGCUG |
| TJR100380 | 22 | CAGCCCCUUAUUGUUAUACGA | 38 | UCGUAUAACAAUAAGGGGCUG |

The sequences of the sense strands and the antisense strands of the LPA siRNAs subjected to modification with 2'-fluoro, 2'-methoxy, and the like are detailed in Table 17 and Table 20; the optical changes of the modifications at position 7 of the antisense strands are detailed in Table 18; the sequences of modified sense and antisense strands of LPA siRNA conjugates are detailed in Table 19 and Table 21.

Table 17. Modified sense and antisense strands of human LPA siRNAs

| Double strand No. | SEQ ID NO: | Sense strand (5'-3') | SEQ ID NO: | Antisense strand (5'-3') |
|---|---|---|---|---|
| TRD001326 | 39 | GmsCmsUmCmCfUmUfAfUfUmGmUmUmAmUmAmCmGmAm | 44 | UmsCfsGmUfAmUfAmAmCfAmAmUfAmAfGmGfAmGfCmsUmsGm |
| TRD001307 | 40 | AmsCmsAmCmCfAmCfAfUfCmAmAmCmAmUmAmAmUmAm | 45 | UmsAfsUmUfAmUfGmUmUfGmAmUfGmUfGmGfUmGfUmsCmsAm |
| | 39 | GmsCmsUmCmCfUmUfAfUfUmGmUmUmAmUmAmCmGmAm | 46 | UmsCfsGmUfAmUfAmAfCmAfAmUfAmAfGmGfAmGfCmsUfsGm |
| | 40 | AmsCmsAmCmCfAmCfAfUfCmAmAmCmAmUmAmAmUmAm | 47 | UmsAfsUmUfAmUfGmUfUmGfAmUfGmUfGmGfUmGfUmsCfsAm |
| | 41 | AmsCmsAmCmCmAmCfAfUfCmAmAmCmAmUmAmAmUmsAm | 48 | UmsAfsUmUfAmUfGmUmUmGfAmUfGmUfGmGfUmGfUmsCmsAm |
| | 42 | GmsCmsUmCmCmUmUfAfUfUmGmUmUmAmUmAmCmGmsAm | 49 | UmsCfsGmUfAmUf(-)hmpNA(A)AmCmAfAmUfAmAfGmGfAmGfCmsUmsGm |
| | 43 | GmsCmsUmCmCmUmUfAfUfUmGmUmUmAmUmAmCmGmAm | 50 | UmsCfsGmUfAmUfAmAmCmAfAmUfAmAfGmGfAmGfCmsUmsGm |
| | 42 | GmsCmsUmCmCmUmUfAfUfUmGmUmUmAmUmAmCmGmsAm | 50 | UmsCfsGmUfAmUfAmAmCmAfAmUfAmAfGmGfAmGfCmsUmsGm |
| | 43 | GmsCmsUmCmCmUmUfAfUfUmGmUmUmAmUmAmCmGmAm | 49 | UmsCfsGmUfAmUf(-)hmpNA(A)AmCmAfAmUfAmAfGmGfAmGfCmsUmsGm |

Table 18. Differential optical chemical modifications at position 7 of AS strands

| SEQ ID NO: | Antisense strand (5'-3') | SEQ ID NO: | Antisense strand (5'-3') | SEQ ID NO: | Antisense strand (5'-3') |
|---|---|---|---|---|---|
| 49 | UmsCfsGmUfAmUf(-)hmpNA(A)AmCmAfAmUfAmAfGmGfAmGfCmsUmsGm | 51 | UmsCfsGmUfAmUf(+)hmpNA(A)AmCmAfAmUfAmAfGmGfAmGfCmsUmsGm | 52 | UmsCfsGmUfAmUfhmpNA(A)AmCmAfAmUfAmAfGmGfAmGfCmsUmsGm |

Table 19. Modified sense and antisense strands of human LPA siRNA conjugates

| Double strand No. | SEQ ID NO: | Sense strand (5'-3') | SEQ ID NO: | Antisense strand (5'-3') |
|---|---|---|---|---|
| TJR100391 | 53 | GmsCmsUmCmCfUmUfAfUfUmGmUmUmAmUmAmCmGmAm-NAG0052' | 46 | UmsCfsGmUfAmUfAmAfCmAfAmUfAmAfGmGfAmGfCmsUfsGm |
| TJR100392 | 54 | AmsCmsAmCmCfAmCfAfUfCmAmAmCmAmUmAmAmUmAm-NAG0052' | 47 | UmsAfsUmUfAmUfGmUfUmGfAmUfGmUfGmGfUmGfUmsCfsAm |
| TJR100395 | 55 | AmsCmsAmCmCmAmCfAfUfCmAmAmCmAmUmAmAmUmsAm-NAG0052' | 48 | UmsAfsUmUfAmUfGmUmUmGfAmUfGmUfGmGfUmGfUmsCmsAm |
| TJR100396 | 56 | GmsCmsUmCmCmUmUfAfUfUmGmUmUmAmUmAmCmGmsAm-NAG0052' | 49 | UmsCfsGmUfAmUf(-)hmpNA(A)AmCmAfAmUfAmAfGmGfAmGfCmsUmsGm |
| TJR100436 | 57 | GmsCmsUmCmCmUmUfAfUfUmGmUmUmAmUmAmCmGmAm-NAG0052' | 50 | UmsCfsGmUfAmUfAmAmCmAfAmUfAmAfGmGfAmGfCmsUmsGm |
| TJR100397 | 56 | GmsCmsUmCmCmUmUfAfUfUmGmUmUmAmUmAmCmGmsAm-NAG0052' | 50 | UmsCfsGmUfAmUfAmAmCmAfAmUfAmAfGmGfAmGfCmsUmsGm |
| TJR100437 | 57 | GmsCmsUmCmCmUmUfAfUfUmGmUmUmAmUmAmCmGmAm-NAG0052' | 49 | UmsCfsGmUfAmUf(-)hmpNA(A)AmCmAfAmUfAmAfGmGfAmGfCmsUmsGm |

Table 20. Modified sense and antisense strands of human LPA siRNAs (control group)

| Double strand No. | SEQ ID NO: | Sense strand (5'-3') | SEQ ID NO: | Antisense strand (5'-3') |
|---|---|---|---|---|
| TRD001324 | 58 | GmsGmsCmAmGfCmUfCfCfUmUmAmUmUmGmUmUmAmUm | 61 | AmsUfsAmAfCmAfAmUmAfAmGmGfAmGfCmUfGmCfCmsAmsCm |

(continued)

| Double strand No. | SEQ ID NO: | Sense strand (5'-3') | SEQ ID NO: | Antisense strand (5'-3') |
|---|---|---|---|---|
| TRD001304 | 59 | AmsUmsGmAmCfAmCfCfAfCmAmUmCmAmAmCmAmUmAm | 62 | UmsAfsUmGfUmUfGmAmUfGmUmGfGmUfGmUfCmAfUmsAmsGm |
| TRD001306 | 60 | GmsAmsCmAmCfCmAfCfAfUmCmAmAmCmAmUmAmAmUm | 63 | AmsUfsUmAfUmGfUmUmGfAmUmGfUmGfGmUfGmUfCmsAmsUm |

Table 21. Modified sense and antisense strands of human LPA siRNA conjugates (control group)

| Double strand No. | SEQ ID NO: | Sense strand (5'-3') | SEQ ID NO: | Antisense strand (5'-3') |
|---|---|---|---|---|
| TJR100390 | 64 | CmsAmsGmCmCmCmCfUmUfAfUfUmGmUmUmAmUmAmCmGmAm-NAG0052' | 66 | UmsCfsGmUfAmUfAmAfCmAfAmUfAmAfGmGfGmGfCmsUfsGm |
| TJR100394 | 65 | GmsAmsCmAmCfCmAfCfAfUmCmAmAmCmAmUmAmAmUmAmGm-NAG0052' | 67 | AmsUfsUmAfUmGfUmUfGmAfUmGfUmGmGmUfGmUfCmsAfsUm |

In Table 17 to Table 21, the nucleotide synthesized using 2-hydroxymethyl-1,3-propanediol as the starting material was defined as hmpNA; hmpNA was a racemic structure;

(-)hmpNA(A) was obtained by solid-phase synthesis using the nucleoside phosphoramidite monomer 1-1a of example section 1.1; (+)hmpNA(A) was an optical isomer;
The lowercase letter m indicates that the left nucleotide adjacent to the letter m is a 2'-methoxy-modified nucleotide;
the lowercase letter f indicates that the left nucleotide adjacent to the letter f is a 2'-fluoro-modified nucleotide;
the lowercase letter s, when present between uppercase letters, indicates that the two nucleotides adjacent to the letter s are linked by a phosphorothioate group;
the lowercase letter s, when being the first at the 3' end, indicates that the left nucleotide adjacent to the letter s ends in a phosphorothioate group.

The structure of NAG0052' is:

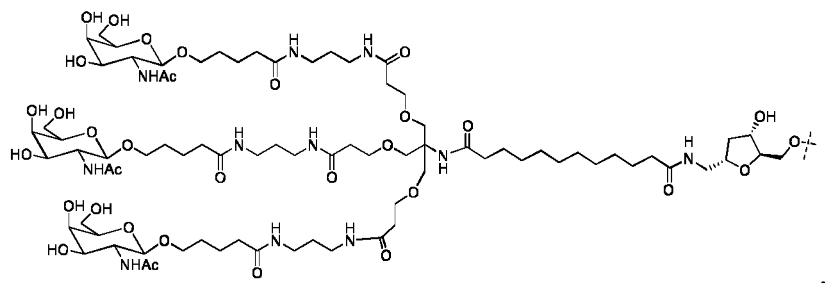

.

**Example 11. Determination of On-Target Activity of siRNAs at 5 Concentration Points Using psiCHECK**

HEK293A cells were cultured at 37 °C with 5% $CO_2$ in a DMEM high glucose medium containing 10% fetal bovine serum. 24 h prior to transfection, the HEK293A cells were seeded into a 96-well plate at a density of $8 \times 10^3$ cells per well, with each well containing 100 μL of medium.

The cells were co-transfected with the siRNAs and the corresponding plasmids using Lipofectamine2000 (ThermoFisher, 11668019) according to the instructions. 0.3 μL of Lipofectamine2000 was used for each well. The amount

of plasmid for transfection was 40 ng per well. For the on-target plasmid, a total of 5 concentration points were set up for the siRNAs, with the highest concentration point final concentration being 10nM. A 10-fold serial dilution was performed (10 nM, 1 nM, 0.1 nM, 0.01 nM, and 0.001 nM). 24 h after transfection, the on-target activity was determined using Dual-Luciferase Reporter Assay System (Promega, E2940). The results are shown in Table 22. The results show that the siRNA TRD001307 had significant activity.

Table 22. psiCHECK results for multi-dose on-target activity of siRNAs

| Double strand No. | Remaining percentage of target gene mRNA expression (mean) | | | | | IC50 value (nM) |
|---|---|---|---|---|---|---|
| | 10 nM | 1 nM | 0.1 nM | 0.01 nM | 0.001 nM | |
| TRD001304 | 30.29 | 49.07 | 88.62 | 97.57 | 103.35 | 1.253 |
| TRD001306 | 21.27 | 29.60 | 58.24 | 98.23 | 102.28 | 0.219 |
| TRD001307 | 15.61 | 18.65 | 45.60 | 88.80 | 87.29 | 0.093 |
| TRD001324 | 22.21 | 30.51 | 58.12 | 93.39 | 98.36 | 0.214 |

**Example 12. Determination of On-Target Activity of siRNAs/siRNA Conjugates at 9 Concentration Points Using psiCHECK**

The siRNAs/siRNA conjugates were subjected to an *in vitro* molecular-level simulation of on-target activity screening in HEK293A cells using 9 concentration gradients. HEK293A cells were cultured at 37 °C with 5% $CO_2$ in a DMEM high glucose medium containing 10% fetal bovine serum. 24 h prior to transfection, the HEK293A cells were seeded into a 96-well plate at a density of $8 \times 10^3$ cells per well, with each well containing 100 μL of medium.

The cells were co-transfected with the siRNAs/siRNA conjugates and the corresponding plasmids using Lipofectamine2000 (ThermoFisher, 11668019) according to the instructions. 0.3 μL of Lipofectamine2000 was used for each well. The amount of plasmid for transfection was 40 ng per well. For the on-target sequence plasmid, a total of 9 concentration points were set up for the siRNAs/siRNA conjugates, with the highest concentration point final concentration being 20 nM. A 3-fold serial dilution was performed, resulting in 20 nM, 6.666666667 nM, 2.222222222 nM, 0.740740741 nM, 0.24691358 nM, 0.082304527 nM, 0.027434842 nM, 0.009144947 nM, and 0.003048316 nM. 24 h after transfection, the on-target levels were determined using Dual-Luciferase Reporter Assay System (Promega, E2940).

In this example, tests were performed using TJR00366 (comprising the sense strand set forth in SEQ ID NO: 2 and the antisense strand set forth in SEQ ID NO: 4) and TJR00373 (comprising the sense strand set forth in SEQ ID NO: 1 and the antisense strand set forth in SEQ ID NO: 3) unmodified in both the sense strand and the antisense strand and TJR100391 (comprising the sense strand set forth in SEQ ID NO: 53 and the antisense strand set forth in SEQ ID NO: 46) and TJR100392 (comprising the sense strand set forth in SEQ ID NO: 54 and the antisense strand set forth in SEQ ID NO: 47) comprising modified sense and antisense strands and conjugated to NAG0052'.

The results show that unmodified siRNAs (naked sequences) TJR100366 and TJR100373 both had significant activity compared with respective similar unmodified siRNA sequences (TJR100367-TJR100372 and TJR100374-TJR100380, respectively), and that TJR100391 and TJR100392 obtained by modifying the naked sequences also had significant activity compared with respective similar siRNA conjugates (TJR100390 and TJR100394, respectively).

Table 23. psi-Check system results for activity of siRNAs/siRNA conjugates at 9 concentration points

| Remaining percentage of target gene mRNA (GSCM) expression (mean) | | | | | |
|---|---|---|---|---|---|
| Double strand code | 20 nM | 6.6667 nM | 2.2222 nM | 0.7407 nM | 0.2469 nM |
| TJR100366 | 20.20% | 29.01% | 31.10% | 24.97% | 35.94% |
| TJR100367 | 40.74% | 44.82% | 38.32% | 45.22% | 72.12% |
| TJR100368 | 44.03% | 61.36% | 61.29% | 73.86% | 89.65% |
| TJR100369 | 39.56% | 43.31% | 41.39% | 30.96% | 54.25% |
| TJR100370 | 35.94% | 29.41% | 34.38% | 38.72% | 34.43% |
| TJR100371 | 32.00% | 38.03% | 38.02% | 40.56% | 51.04% |
| TJR100372 | 23.61% | 24.14% | 16.10% | 26.83% | 39.81% |
| TJR100373 | 15.98% | 11.76% | 13.59% | 9.17% | 14.48% |

(continued)

| Remaining percentage of target gene mRNA (GSCM) expression (mean) | | | | | |
|---|---|---|---|---|---|
| Double strand code | 20 nM | 6.6667 nM | 2.2222 nM | 0.7407 nM | 0.2469 nM |
| TJR100374 | 13.34% | 18.81% | 16.75% | 23.25% | 19.18% |
| TJR100375 | 22.80% | 16.76% | 16.20% | 24.98% | 35.53% |
| TJR100376 | 25.77% | 21.32% | 20.52% | 20.19% | 22.31% |
| TJR100377 | 27.50% | 16.65% | 24.07% | 30.53% | 30.15% |
| TJR100378 | 24.50% | 28.13% | 26.93% | 20.54% | 34.72% |
| TJR100379 | 23.79% | 21.57% | 22.88% | 19.63% | 23.04% |
| TJR100380 | 23.56% | 16.49% | 21.94% | 17.29% | 29.64% |
| TJR100390 | 21.27% | 26.99% | 41.27% | 54.59% | 73.86% |
| TJR100391 | 17.07% | 15.35% | 21.62% | 27.79% | 44.88% |
| TJR100392 | 18.51% | 16.84% | 17.79% | 20.59% | 28.30% |
| TJR100394 | 44.63% | 49.03% | 53.47% | 66.51% | 85.05% |
| TJR100397 | 19.37% | 19.29% | 19.66% | 29.00% | 46.23% |
| TJR100436 | 13.15% | 17.00% | 19.86% | 28.76% | 35.32% |
| TJR100437 | 22.99% | 17.51% | 25.75% | 35.42% | 58.94% |
| Double strand code | 0.0823 nM | 0.0274 nM | 0.0091 nM | 0.0030 nM | GSCM IC50 (nM) |
| TJR100366 | 40.42% | 70.03% | 87.00% | 99.70% | 0.0593 |
| TJR100367 | 73.25% | 100.71% | 103.40% | 96.76% | 0.6989 |
| TJR100368 | 102.47% | 121.88% | 104.45% | 127.00% | 16.2555 |
| TJR100369 | 73.59% | 74.71% | 115.48% | 104.38% | 0.2480 |
| TJR100370 | 65.07% | 84.84% | 101.76% | 128.80% | 0.1449 |
| TJR100371 | 77.52% | 121.88% | 127.03% | 137.12% | 0.2297 |
| TJR100372 | 60.49% | 78.26% | 81.84% | 72.39% | 0.1440 |
| TJR100373 | 21.75% | 28.83% | 48.99% | 85.47% | 0.0085 |
| TJR100374 | 39.96% | 53.93% | 80.13% | 97.25% | 0.0373 |
| TJR100375 | 46.77% | 56.75% | 88.33% | 80.00% | 0.0679 |
| TJR100376 | 25.74% | 55.63% | 54.66% | 95.83% | 0.0157 |
| TJR100377 | 58.65% | 85.13% | 143.41% | 86.67% | 0.1033 |
| TJR100378 | 45.69% | 59.79% | 91.77% | 128.43% | 0.0479 |
| TJR100379 | 55.27% | 98.91% | 91.07% | 100.67% | 0.0908 |
| TJR100380 | 52.96% | 73.05% | 93.73% | 88.47% | 0.0916 |
| TJR100390 | 99.18% | 124.78% | 109.91% | 110.53% | 0.9087 |
| TJR100391 | 70.80% | 92.34% | 91.90% | 96.62% | 0.2021 |
| TJR100392 | 49.39% | 77.73% | 101.18% | 106.62% | 0.0815 |
| TJR100394 | 104.56% | 91.63% | 105.20% | 107.20% | 3.4364 |
| TJR100397 | 79.34% | 96.20% | 105.62% | 98.74% | 0.2234 |
| TJR100436 | 61.22% | 91.54% | 89.91% | 90.43% | 0.1406 |
| TJR100437 | 72.08% | 98.87% | 113.75% | 99.54% | 0.3112 |

**Example 13. Inhibition of Human LPA in Primary Human Hepatocytes (PHHs) by siRNAs - 7-Concentration-Point Inhibitory Activity**

In primary human hepatocytes cells, the siRNA sequences were subjected to PHH activity screening using 7 concentration gradients. The initial final concentration for transfection of each siRNA sample was 20 nM, and 5-fold serial dilution was performed to obtain 7 concentration points.

The PHHs were cryopreserved in liquid nitrogen. 24 h prior to transfection, the PHHs were thawed and then seeded into a 96-well plate at a density of $3 \times 10^4$ cells per well, with each well containing 80 μL of medium.

The cells were transfected with the siRNAs using Lipofectamine RNAi MAX (ThermoFisher, 13778150) by following the instruction manual of the product, with the final gradient concentrations of the siRNAs for transfection being 20 nM 4 nM, 0.8 nM, 0.16 nM, 0.032 nM, 0.0064 nM, and 0.00128 nM. 24 h after treatment, the total cellular RNA was extracted using a high-throughput cellular RNA extraction kit, and RNA reverse transcription and quantitative real-time PCR detection were carried out. The mRNA level of human LPA was measured, and the mRNA level of human LPA was corrected based on the GAPDH internal reference gene level.

In the quantitative real-time PCR detection, the probe Q-PCR detection was carried out, and the information about primers is shown in Table 24 and Table 25.

After the Taqman probe Q-PCR detection was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as $2^{-\triangle\triangle Ct}$, $\triangle\triangle Ct$ = [(target gene of Ct experimental group - internal reference of Ct experimental group) - (target gene of Ct control group - internal reference of Ct control group)]. Inhibition rate (%) = (1 - remaining level of target gene expression) × 100%. The results are expressed relative to the remaining percentage of human LPA mRNA expression in cells treated with the control siRNA. The inhibition rate IC50 results are shown in Table 26.

The results show that TJR100373 had significantly advantageous activity.

Table 24. Taqman LPA probe primers

| Primer name | Primer sequence |
|---|---|
| hLPA-PF-MGB | TGGAGGGCCTCTGGTTTG (SEQ ID NO:95) |
| hLPA-PR-MGB | GGCGTGCACAGCCAAGAC (SEQ ID NO:96) |
| hLPA-P-MGB | 5'6-FAM- CATTTTACAAGGAGTCACTTC-3'MGB (SEQ ID NO:97) |

Table 25. Taqman GAPDH probe primer

| Primer name | Brand | Cat. No. |
|---|---|---|
| Human GAPDH TaqMan Probe | Thermo | 4326317E |

Table 26. Multi-dose inhibitory activity of siRNAs against human LPA in primary human hepatocytes (PHHs)

| Double strand No. | 20 nM | 4nM | 0.8 nM | 0.16 nM | 0.032 nM | 0.0064 nM | 0.00128 nM | Primary human hepatocyte IC50 value (nM) |
|---|---|---|---|---|---|---|---|---|
| TJR100373 | 29.07% | 36.59% | 31.87% | 39.70% | 45.57% | 71.39% | 79.77% | 0.0263 |
| TJR100374 | 34.58% | 38.30% | 46.88% | 57.16% | 62.79% | 81.55% | 100.41% | 0.3483 |
| TJR100375 | 51.18% | 51.65% | 52.71% | 59.77% | 78.92% | 96.54% | 114.58% | 2.7102 |
| TJR100376 | 44.89% | 46.56% | 44.72% | 46.04% | 65.10% | 85.41% | 112.03% | 0.1503 |
| TJR100377 | 40.07% | 44.51% | 54.34% | 55.58% | 69.48% | 100.48% | 115.74% | 0.4786 |
| TJR100378 | 44.43% | 55.45% | 54.96% | 59.76% | 71.64% | 88.84% | 92.48% | 5.2723 |

(continued)

| Double strand No. | 20 nM | 4nM | 0.8 nM | 0.16 nM | 0.032 nM | 0.0064 nM | 0.00128 nM | Primary human hepatocyte IC50 value (nM) |
|---|---|---|---|---|---|---|---|---|
| TJR100379 | 41.66% | 59.93% | 56.35% | 61.95% | 73.63% | 80.10% | 104.42% | 7.2444 |
| TJR100380 | 38.56% | 50.74% | 56.68% | 63.08% | 79.36% | 91.70% | 112.31% | 2.2542 |

## Claims

1. An siRNA targeting LPA, comprising a sense strand and an antisense strand forming a double-stranded region, wherein

   the sense strand comprises at least 15 contiguous nucleotides and differs by no more than 3 nucleotides from any one of the nucleotide sequences of SEQ ID NO: 1 or SEQ ID NO: 2; and
   the antisense strand comprises at least 15 contiguous nucleotides and differs by no more than 3 nucleotides from any one of the nucleotide sequences of SEQ ID NO: 3 or SEQ ID NO: 4.

2. The siRNA according to claim 1, comprising or selected from the group consisting of the nucleotide sequences set forth in any one of the following groups:

   group 1), a sense strand set forth in SEQ ID NO: 1 and an antisense strand set forth in SEQ ID NO: 3;
   group 2), a sense strand set forth in SEQ ID NO: 2 and an antisense strand set forth in SEQ ID NO: 4.

3. The siRNA according to any one of the preceding claims, wherein at least one nucleotide in the sense strand and/or the antisense strand is a modified nucleotide.

4. The siRNA according to any one of the preceding claims, wherein at least one nucleotide at positions 2 to 8 of the 5' end of the antisense strand is a modified nucleotide, wherein:

   - the modified nucleotide is a 2'-methoxy-modified nucleotide, or
   - the modified nucleotide comprises a chemical modification of formula (I) or a tautomeric modification thereof, the chemical modification of formula (I) being selected from the group consisting of:

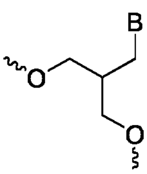 , 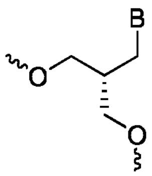 , or 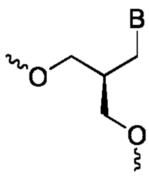 ;

   each B is independently selected from the group consisting of bases corresponding to positions 2 to 8 of the 5' end of the antisense strand.

5. The siRNA according to any one of the preceding claims, wherein the nucleotide at position 5, 6, or 7 of the 5' end of the antisense strand

   - is a 2'-methoxy-modified nucleotide, or
   - comprises the chemical modification of formula (I) or the tautomeric modification thereof as defined in claim 5;

   when the chemical modification of formula (I) or the tautomeric modification thereof is at position 5 of the 5' end, B is the base at position 5 of the 5' end of the antisense strand; when the chemical modification of formula (I) or the tautomeric modification thereof is at position 6 of the 5' end, B is the base at position 6 of the 5' end of the antisense

strand; when the chemical modification of formula (I) or the tautomeric modification thereof is at position 7 of the 5' end, B is the base at position 7 of the 5' end of the antisense strand.

**6.** The siRNA according to any one of the preceding claims, wherein three contiguous nucleotides in the sense strand are 2'-fluoro-modified nucleotides; and/or

in a 5'-end to 3'-end direction, nucleotides at positions 2, 4, 6, 9, 12, 14, 16, and 18 of the antisense strand are each independently a 2'-fluoro-modified nucleotide; or
in a 5'-end to 3'-end direction, nucleotides at positions 2, 4, 6, 10, 12, 14, 16, and 18 of the antisense strand are each independently a 2'-fluoro-modified nucleotide.

**7.** The siRNA according to any one of the preceding claims, wherein the sense strand comprises or is selected from a nucleotide sequence of the formula shown below:

5'-$N_aN_aN_aN_aN_aN_aN_bN_bN_bN_aN_aN_aN_aN_aN_aN_aN_aN_aN_a$-3'; or,
5'-$N_aN_aN_aN_aN_bN_aN_bN_bN_bN_aN_aN_aN_aN_aN_aN_aN_aN_aN_a$-3';
wherein $N_a$ is a 2'-methoxy-modified nucleotide, and $N_b$ is a 2'-fluoro-modified nucleotide.

**8.** The siRNA according to any one of the preceding claims, wherein the antisense strand comprises or is selected from a nucleotide sequence of the formula shown below:

5'-$N_a'N_b'N_a'N_b'N_a'N_b'W'N_a'N_a'N_b'N_a'N_b'N_a'N_b'N_a'N_b'N_a'N_b'N_a'N_a'N_a'$-3'; or,
5'-$N_a'N_b'N_a'N_b'N_a'N_b'W'N_a'N_b'N_a'N_a'N_b'N_a'N_b'N_a'N_b'N_a'N_b'N_a'N_a'N_a'$-3';
wherein,

$N_a'$ is a 2'-methoxy-modified nucleotide;
$N_b'$ is a 2'-fluoro-modified nucleotide;
W' is a 2'-methoxy-modified nucleotide, or W' is a nucleotide with the chemical modification of formula (I) or the tautomeric modification thereof as defined in claim 4 or 5.

**9.** The siRNA according to claim 8, wherein W' is selected from a nucleotide comprising a

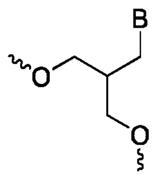 , 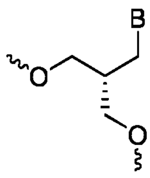 , or 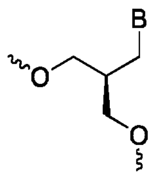

modification; wherein: B is a base; preferably, B is selected from the base corresponding to position 7 of the 5' end of the antisense strand.

**10.** The siRNA according to claim 8, wherein W' is selected from a 2'-methoxy-modified nucleotide.

**11.** The siRNA according to any one of the preceding claims, wherein at least one phosphoester group in the sense strand and/or the antisense strand is a phosphoester group with a modification group, preferably a phosphorothioate group.

**12.** The siRNA according to any one of the preceding claims, wherein

the sense strand comprises the nucleotide sequence set forth in any one of SEQ ID NO: 39 to SEQ ID NO: 43;
the antisense strand comprises the nucleotide sequence set forth in any one of SEQ ID NO: 44 to SEQ ID NO: 52;
preferably, the siRNA comprises a sense strand and an antisense strand of any one of the following groups:

group 1), a sense strand set forth in SEQ ID NO: 42 and an antisense strand set forth in SEQ ID NO: 49;
group 2), a sense strand set forth in SEQ ID NO: 43 and an antisense strand set forth in SEQ ID NO: 50;
group 3), a sense strand set forth in SEQ ID NO: 42 and an antisense strand set forth in SEQ ID NO: 50;
group 4), a sense strand set forth in SEQ ID NO: 43 and an antisense strand set forth in SEQ ID NO: 49;

group 5), a sense strand set forth in SEQ ID NO: 39 and an antisense strand set forth in SEQ ID NO: 44;
group 6), a sense strand set forth in SEQ ID NO: 40 and an antisense strand set forth in SEQ ID NO: 45;
group 7), a sense strand set forth in SEQ ID NO: 39 and an antisense strand set forth in SEQ ID NO: 46;
group 8), a sense strand set forth in SEQ ID NO: 40 and an antisense strand set forth in SEQ ID NO: 47;
group 9), a sense strand set forth in SEQ ID NO: 41 and an antisense strand set forth in SEQ ID NO: 48.

**13.** An siRNA conjugate, comprising:

the siRNA according to any one of claims 1-12 and a targeting ligand linked to the end of the siRNA;
wherein preferably, the targeting ligand is linked to the 3' end of the sense strand of the siRNA.

**14.** The siRNA conjugate according to claim 13, wherein:

the targeting ligand comprises at least one targeting moiety, and
the targeting moieties are each independently selected from the group consisting of: galactose, galactosamine, N-formyl-galactosamine, N-acetyl-galactosamine, N-propionyl-galactosamine, N-n-butyryl-galactosamine, and N-isobutyryl-galactosamine; preferably, the targeting moiety is N-acetyl-galactosamine;
more preferably, the targeting ligand comprises three identical or different targeting moieties.

**15.** The siRNA conjugate according to claim 14, wherein the targeting ligand is a compound of formula (II) or a pharmaceutically acceptable salt thereof, wherein the formula (II) is:

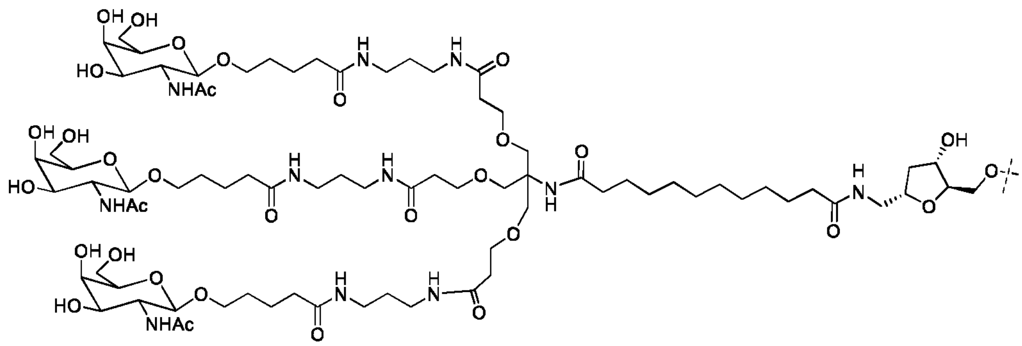

.

**16.** An siRNA conjugate, comprising a sense strand and an antisense strand forming a double-stranded region, wherein

the sense strand comprises any one of SEQ ID NO: 53 to SEQ ID NO: 57;
the antisense strand comprises any one of SEQ ID NO: 49 to SEQ ID NO: 52 and SEQ ID NO: 58 to SEQ ID NO: 60;
preferably, the siRNA conjugate comprises a sense strand and an antisense strand of any one of the following groups:

group 1), a sense strand set forth in SEQ ID NO: 56 and an antisense strand set forth in SEQ ID NO: 49;
group 2), a sense strand set forth in SEQ ID NO: 57 and an antisense strand set forth in SEQ ID NO: 50;
group 3), a sense strand set forth in SEQ ID NO: 56 and an antisense strand set forth in SEQ ID NO: 50;
group 4), a sense strand set forth in SEQ ID NO: 57 and an antisense strand set forth in SEQ ID NO: 49;
group 5), a sense strand set forth in SEQ ID NO: 53 and an antisense strand set forth in SEQ ID NO: 46;
group 6), a sense strand set forth in SEQ ID NO: 54 and an antisense strand set forth in SEQ ID NO: 47;
group 7), a sense strand set forth in SEQ ID NO: 55 and an antisense strand set forth in SEQ ID NO: 48.

**17.** A pharmaceutical composition, comprising the siRNA according to any one of claims 1-12 or the siRNA conjugate according to any one of claims 13-16, and a pharmaceutically acceptable carrier.

**18.** A cell, comprising the siRNA according to any one of claims 1-12.

**19.** A kit, comprising the siRNA according to any one of claims 1-12 or the siRNA conjugate according to any one of claims 13-16.

**20.** A method for inhibiting the expression of LPA, comprising administering to a subject an effective amount or an effective dose of the siRNA according to any one of claims 1-12, or the siRNA conjugate according to any one of claims 13-16, or the pharmaceutical composition according to claim 17.

**21.** A method for treating and/or preventing a disease associated with elevated lipoprotein(a) and/or apolipoprotein(a) levels in a subject, comprising administering to the subject an effective amount or an effective dose of the siRNA according to any one of claims 1-12, or the siRNA conjugate according to any one of claims 13-16, or the pharmaceutical composition according to claim 17, wherein preferably, the disease associated with elevated lipoprotein (a) and/or apolipoprotein (a) levels is selected from a cardiovascular disease; more preferably, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

**22.** A method for treating and/or preventing a disease, comprising administering to a subject an effective amount or an effective dose of the siRNA according to any one of claims 1-12, or the siRNA conjugate according to any one of claims 13-16, or the pharmaceutical composition according to claim 17, wherein the disease is selected from a cardiovascular disease; preferably, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

**23.** A method for reducing lipoprotein(a) and/or apolipoprotein(a) levels, comprising administering to a subject an effective amount or an effective dose of the siRNA according to any one of claims 1-12, or the siRNA conjugate according to any one of claims 13-16, or the pharmaceutical composition according to claim 17.

**24.** A method for delivering an siRNA that inhibits the expression and/or replication of LPA to the liver *in vivo,* comprising administering to a subject the siRNA according to any one of claims 1-12, or the siRNA conjugate according to any one of claims 13-16, or the pharmaceutical composition according to claim 17.

**25.** A method for preparing an siRNA or an siRNA conjugate, comprising synthesizing the siRNA according to any one of claims 1-12, or the siRNA conjugate according to any one of claims 13-16, or the pharmaceutical composition according to claim 17.

**26.** A vector, comprising the siRNA according to any one of claims 1-12.

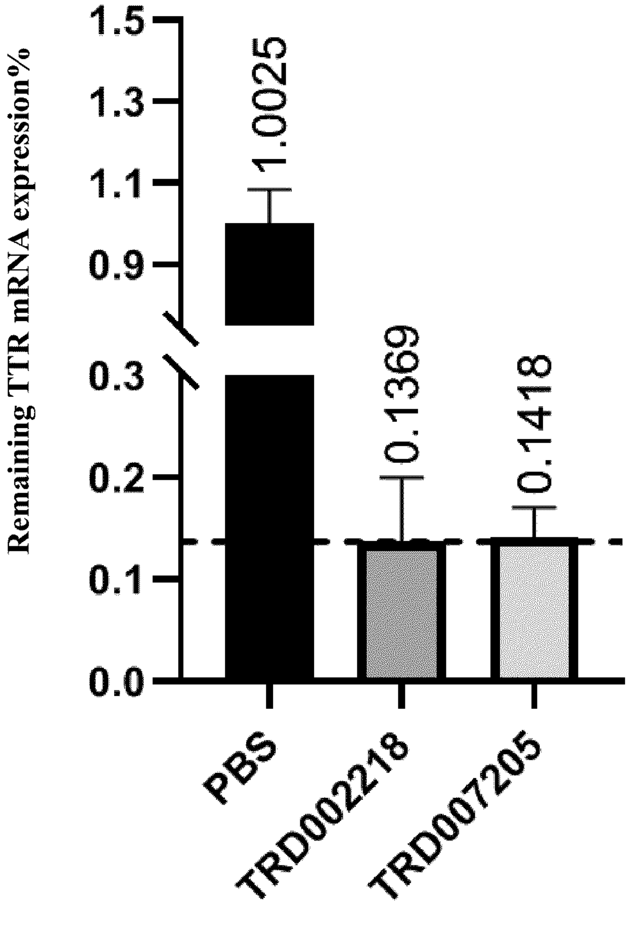
Remaining TTR mRNA expression%
1.5
1.3
1.1
0.9
0.3
0.2
0.1
0.0
1.0025
0.1369
0.1418
PBS
TRD002218
TRD007205

FIG. 1

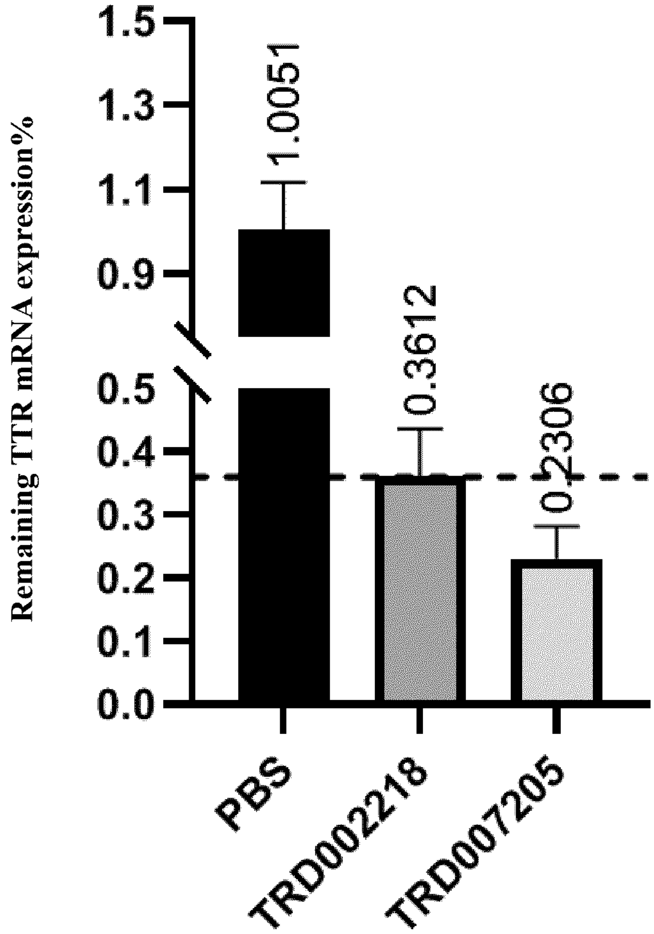
Remaining TTR mRNA expression%
1.5
1.3
1.1
0.9
0.5
0.4
0.3
0.2
0.1
0.0
1.0051
0.3612
0.2306
PBS
TRD002218
TRD007205

FIG. 2

**INTERNATIONAL SEARCH REPORT**

International application No.
**PCT/CN2022/139500**

**A. CLASSIFICATION OF SUBJECT MATTER**

C12N15/113(2010.01)i;A61K 47/54(2017.01)i;A61K 48/00(2006.01)i;A61P 9/10(2006.01)i

According to International Patent Classification (IPC) or to both national classification and IPC

**B. FIELDS SEARCHED**

Minimum documentation searched (classification system followed by classification symbols)

IPC:C12N; A61K; A61P

Documentation searched other than minimum documentation to the extent that such documents are included in the fields searched

Electronic data base consulted during the international search (name of data base and, where practicable, search terms used)

CNKI, CNABS, CNTXT, DWPI, SIPOABS, EPTXT, WOTXT, USTXT, ELSEVIER, ISI Web of Knowledge, PubMed, 中国专利生物序列检索系统, China Patent Biological Sequence Search System, NCBI, STN: LPA, Apo(a), siRNA, GalNac, GCUCCUUAUUGUUAUACGA, UCGUAUAACAAUAAGGAGCUG, ACACCACAUCAACAUAAUA, UAUUAUGUUGAUGUGGUGUCA, 2'-F, 2'-OMe

**C. DOCUMENTS CONSIDERED TO BE RELEVANT**

| Category* | Citation of document, with indication, where appropriate, of the relevant passages | Relevant to claim No. |
|---|---|---|
| Y | CN 112921036 A (IONIS PHARMACEUTICALS, INC.) 08 June 2021 (2021-06-08) description, paragraphs 0016-0020, 0022, 0027-0028, 0262, 0707, 1135-1163 and 1214-1217 | 1-26 |
| Y | WO 2019204668 A1 (CASEBIA THERAPEUTICS LIMITED LIABILITY PARTNERSHIP) 24 October 2019 (2019-10-24) claims 1-51, description, paragraph 0502, table 3, SEQ ID NO: 108 | 1-26 |
| Y | CN 112876534 A (SUZHOU RIBO LIFE SCIENCE CO., LTD.) 01 June 2021 (2021-06-01) claims 1-21 | 1-26 |
| A | CN 111378655 A (SUZHOU RIBO LIFE SCIENCE CO., LTD.) 07 July 2020 (2020-07-07) entire document | 1-26 |
| A | WO 2018013525 A1 (TRANSLATE BIO MA, INC.) 18 January 2018 (2018-01-18) entire document | 1-26 |
| A | WO 2021142454 A1 (ONCOLMMUNIN, INC.) 15 July 2021 (2021-07-15) entire document | 1-26 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 February 2023** | **07 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/139500**

**C. DOCUMENTS CONSIDERED TO BE RELEVANT**

| Category* | Citation of document, with indication, where appropriate, of the relevant passages | Relevant to claim No. |
|---|---|---|
| Y | GenBank. "NM_005577.3"<br>*NCBI,* 13 August 2019 (2019-08-13),<br>entire document | 1-26 |

Form PCT/ISA/210 (second sheet) (July 2022)

**INTERNATIONAL SEARCH REPORT**

International application No.
**PCT/CN2022/139500**

**Box No. II** **Observations where certain claims were found unsearchable (Continuation of item 2 of first sheet)**

This international search report has not been established in respect of certain claims under Article 17(2)(a) for the following reasons:

1. ☑ Claims Nos.: **21-24**
because they relate to subject matter not required to be searched by this Authority, namely:

Claim 21 relates to a method for treating and/or preventing diseases associated with elevated levels of lipoproteins (a) and/or apolipoproteins (a) in a subject, claim 22 relates to a method for treating and/or preventing diseases, claim 23 relates to a method for reducing the level of lipoproteins (a) and/or apolipoproteins (a), and claim 24 relates to a method for in-vivo delivery of siRNA inhibiting LPA expression and/or replication to a liver, belonging to the subject matter defined in PCT Rule 39.1(iv). The present search report is made on the basis of the pharmaceutical use of siRNA, an siRNA conjugate or a pharmaceutical composition thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/139500**

| Patent document cited in search report | Publication date (day/month/year) | Patent family member(s) | Publication date (day/month/year) |
|---|---|---|---|
| CN 112921036 A | 08 June 2021 | EA 201891479 A1 | 30 November 2018 |
| | | EA 036584 B1 | 26 November 2020 |
| | | ES 2778442 T3 | 10 August 2020 |
| | | MX 2020004209 A | 13 August 2020 |
| | | JP 2020058370 A | 16 April 2020 |
| | | RU 2019124314 A | 21 August 2019 |
| | | US 2016076032 A1 | 17 March 2016 |
| | | US 9714421 B2 | 25 July 2017 |
| | | IL 261901 A | 31 October 2018 |
| | | IL 261901 B | 31 May 2020 |
| | | MX 2019010441 A | 17 October 2019 |
| | | RS 60796 B1 | 30 October 2020 |
| | | IL 263843 A | 31 January 2019 |
| | | IL 263843 B | 31 March 2020 |
| | | WO 2014179620 A1 | 06 November 2014 |
| | | KR 20230006933 A | 11 January 2023 |
| | | JP 2016526874 A | 08 September 2016 |
| | | JP 6387084 B2 | 05 September 2018 |
| | | KR 20190084138 A | 15 July 2019 |
| | | KR 102212275 B1 | 05 February 2021 |
| | | IL 242124 B | 28 February 2019 |
| | | US 2016017323 A1 | 21 January 2016 |
| | | JP 2016523515 A | 12 August 2016 |
| | | JP 6456362 B2 | 23 January 2019 |
| | | EP 2991656 A2 | 09 March 2016 |
| | | EP 2991656 A4 | 22 February 2017 |
| | | EP 2991656 B1 | 18 December 2019 |
| | | HRP 20201378 T1 | 27 November 2020 |
| | | HUE 050394 T2 | 30 November 2020 |
| | | IL 296543 A | 01 November 2022 |
| | | EP 3633039 A1 | 08 April 2020 |
| | | US 2015176007 A1 | 25 June 2015 |
| | | US 9145558 B2 | 29 September 2015 |
| | | US 2018273953 A1 | 27 September 2018 |
| | | US 10883104 B2 | 05 January 2021 |
| | | AU 2017200365 A1 | 23 February 2017 |
| | | AU 2017200365 B2 | 08 November 2018 |
| | | AU 2017200365 C1 | 18 April 2019 |
| | | JP 2021020901 A | 18 February 2021 |
| | | JP 7177127 B2 | 22 November 2022 |
| | | AU 2014259759 A1 | 22 October 2015 |
| | | AU 2014259759 B2 | 18 June 2020 |
| | | US 2021130823 A1 | 06 May 2021 |
| | | IL 274064 A | 30 June 2020 |
| | | IL 274064 B | 30 June 2021 |
| | | IL 242132 B | 31 October 2018 |
| | | US 2021395734 A1 | 23 December 2021 |
| | | PT 2992098 T | 05 July 2019 |
| | | AU 2022202770 A1 | 19 May 2022 |
| | | HK 1221404 A1 | 02 June 2017 |

Form PCT/ISA/210 (patent family annex) (July 2022)

**INTERNATIONAL SEARCH REPORT**
**Information on patent family members**

International application No.
**PCT/CN2022/139500**

| Patent document cited in search report | Publication date (day/month/year) | Patent family member(s) | Publication date (day/month/year) |
|---|---|---|---|
| | | CA 2921162 A1 | 06 November 2014 |
| | | US 2018273952 A1 | 27 September 2018 |
| | | US 10927372 B2 | 23 February 2021 |
| | | AU 2020207820 A1 | 06 August 2020 |
| | | UA 120287 C2 | 11 November 2019 |
| | | LT 2992098 T | 10 July 2019 |
| | | IL 264241 A | 28 February 2019 |
| | | IL 264241 B | 30 April 2020 |
| | | JP 2020039355 A | 19 March 2020 |
| | | AU 2014259757 A1 | 22 October 2015 |
| | | AU 2014259757 B2 | 02 March 2017 |
| | | PT 3524680 T | 04 January 2021 |
| | | ES 2819213 T3 | 15 April 2021 |
| | | MY 178929 A | 23 October 2020 |
| | | US 2020224198 A1 | 16 July 2020 |
| | | AU 2014259755 A1 | 22 October 2015 |
| | | AU 2014259755 B2 | 30 August 2018 |
| | | CR 20190269 A | 13 September 2019 |
| | | KR 20210151260 A | 13 December 2021 |
| | | US 2016076030 A1 | 17 March 2016 |
| | | US 9932580 B2 | 03 April 2018 |
| | | AU 2019200820 A1 | 28 February 2019 |
| | | AU 2019200820 B2 | 30 April 2020 |
| | | CL 2015003217 A1 | 08 July 2016 |
| | | HK 1221486 A1 | 02 June 2017 |
| | | EP 2992009 A1 | 09 March 2016 |
| | | EP 2992009 A4 | 28 December 2016 |
| | | EP 2992009 B1 | 24 June 2020 |
| | | AU 2019203674 A1 | 27 June 2019 |
| | | AU 2019203674 B2 | 25 March 2021 |
| | | AU 2018267625 A1 | 13 December 2018 |
| | | AU 2018267625 B2 | 10 September 2020 |
| | | MX 2015015263 A | 16 December 2016 |
| | | SG 11201508800 WA | 27 November 2015 |
| | | JP 2020007361 A | 16 January 2020 |
| | | HK 1221475 A1 | 02 June 2017 |
| | | NZ 740338 A | 29 April 2022 |
| | | BR 112015027319 A2 | 26 September 2017 |
| | | HUE 043697 T2 | 30 September 2019 |
| | | RU 2018136140 A | 17 December 2018 |
| | | RU 2018136140 A3 | 27 April 2022 |
| | | US 2021024923 A1 | 28 January 2021 |
| | | US 2022275365 A9 | 01 September 2022 |
| | | IL 264580 A | 28 February 2019 |
| | | IL 264580 B | 30 April 2020 |
| | | MX 2020002184 A | 14 July 2020 |
| | | IL 273184 A | 30 April 2020 |
| | | IL 273184 B | 29 July 2021 |
| | | DOP 2021000095 A | 15 September 2021 |
| | | EP 2991661 A1 | 09 March 2016 |

Form PCT/ISA/210 (patent family annex) (July 2022)

**INTERNATIONAL SEARCH REPORT**
**Information on patent family members**

International application No.
**PCT/CN2022/139500**

| Patent document cited in search report | Publication date (day/month/year) | Patent family member(s) | | | Publication date (day/month/year) |
|---|---|---|---|---|---|
| | | EP | 2991661 | A4 | 15 February 2017 |
| | | EP | 2991661 | B1 | 13 March 2019 |
| | | CL | 2016002262 | A1 | 09 June 2017 |
| | | IL | 273205 | A | 30 April 2020 |
| | | RU | 2015151203 | A | 02 June 2017 |
| | | RU | 2650510 | C2 | 16 April 2018 |
| | | BR | 112015027321 | A2 | 26 September 2017 |
| | | NZ | 631512 | A | 28 October 2016 |
| | | EP | 2992097 | A2 | 09 March 2016 |
| | | EP | 2992097 | A4 | 04 January 2017 |
| | | EP | 2992097 | B1 | 06 November 2019 |
| | | KR | 20210014758 | A | 09 February 2021 |
| | | PE | 20152002 | A1 | 21 January 2016 |
| | | WO | 2014179629 | A2 | 06 November 2014 |
| | | WO | 2014179629 | A3 | 22 January 2015 |
| | | WO | 2014179629 | A8 | 02 June 2016 |
| | | EP | 3828275 | A1 | 02 June 2021 |
| | | KR | 20180051678 | A | 16 May 2018 |
| | | KR | 102138781 | B1 | 28 July 2020 |
| | | SG | 11201508870 | VA | 27 November 2015 |
| | | KR | 20160002976 | A | 08 January 2016 |
| | | KR | 102315836 | B1 | 22 October 2021 |
| | | IL | 283660 | A | 29 July 2021 |
| | | PH | 12015502493 | A1 | 22 February 2016 |
| | | ES | 2730015 | T3 | 07 November 2019 |
| | | IL | 242125 | B | 28 February 2019 |
| | | MX | 2015015234 | A | 03 October 2016 |
| | | DK | 3524680 | T3 | 14 December 2020 |
| | | PE | 20161430 | A1 | 06 January 2017 |
| | | ME | 03390 | B | 20 January 2020 |
| | | AU | 2017203436 | A1 | 08 June 2017 |
| | | AU | 2017203436 | B2 | 18 October 2018 |
| | | HK | 1221403 | A1 | 02 June 2017 |
| | | KR | 20220108195 | A | 02 August 2022 |
| | | RU | 2015151199 | A | 05 June 2017 |
| | | RU | 2015151199 | A3 | 27 March 2018 |
| | | RU | 2697152 | C2 | 12 August 2019 |
| | | JP | 2019056001 | A | 11 April 2019 |
| | | JP | 6639629 | B2 | 05 February 2020 |
| | | WO | 2014179627 | A2 | 06 November 2014 |
| | | WO | 2014179627 | A9 | 26 February 2015 |
| | | WO | 2014179627 | A3 | 16 April 2015 |
| | | SG | 10201801507 | RA | 28 March 2018 |
| | | BR | 112015027369 | A2 | 26 September 2017 |
| | | BR | 112015027369 | B1 | 08 June 2021 |
| | | JP | 2021107408 | A | 29 July 2021 |
| | | NZ | 753018 | A | 28 January 2022 |
| | | EP | 2992098 | A2 | 09 March 2016 |
| | | EP | 2992098 | A4 | 11 January 2017 |
| | | EP | 2992098 | B1 | 27 March 2019 |

Form PCT/ISA/210 (patent family annex) (July 2022)

**INTERNATIONAL SEARCH REPORT**
**Information on patent family members**

International application No.
**PCT/CN2022/139500**

| Patent document cited in search report | Publication date (day/month/year) | Patent family member(s) | | | Publication date (day/month/year) |
|---|---|---|---|---|---|
| | | JP | 2018027091 | A | 22 February 2018 |
| | | JP | 6592486 | B2 | 16 October 2019 |
| | | JP | 2016522817 | A | 04 August 2016 |
| | | JP | 6216444 | B2 | 18 October 2017 |
| | | PL | 2992098 | T3 | 30 September 2019 |
| | | WO | 2014179626 | A2 | 06 November 2014 |
| | | WO | 2014179626 | A3 | 26 February 2015 |
| | | US | 2018044676 | A1 | 15 February 2018 |
| | | US | 10683499 | B2 | 16 June 2020 |
| | | DOP | 2016000287 | A | 15 February 2017 |
| | | DK | 2992098 | T3 | 17 June 2019 |
| | | JP | 2016522683 | A | 04 August 2016 |
| | | JP | 6995478 | B2 | 14 January 2022 |
| | | NZ | 725538 | A | 26 February 2021 |
| | | JP | 2020074787 | A | 21 May 2020 |
| | | US | 2016090596 | A1 | 31 March 2016 |
| | | US | 9957504 | B2 | 01 May 2018 |
| | | CA | 2921514 | A1 | 06 November 2014 |
| | | JP | 2018183184 | A | 22 November 2018 |
| | | JP | 6652602 | B2 | 26 February 2020 |
| | | SG | 10201906382 | QA | 27 August 2019 |
| | | AU | 2021204244 | A1 | 22 July 2021 |
| | | RU | 2018112167 | A | 07 March 2019 |
| | | SI | 2992009 | T1 | 30 October 2020 |
| | | NZ | 631552 | A | 24 February 2017 |
| | | IL | 273312 | A | 30 April 2020 |
| | | US | 2015126719 | A1 | 07 May 2015 |
| | | US | 9163239 | B2 | 20 October 2015 |
| | | PH | 12019501191 | A1 | 01 March 2021 |
| | | WO | 2014179625 | A1 | 06 November 2014 |
| | | NZ | 712737 | A | 27 August 2021 |
| | | CA | 2921518 | A1 | 06 November 2014 |
| | | NZ | 728517 | A | 24 December 2021 |
| | | ZA | 201507216 | B | 30 August 2017 |
| | | EP | 3524680 | A1 | 14 August 2019 |
| | | EP | 3524680 | B1 | 11 November 2020 |
| | | AU | 2017200950 | A1 | 02 March 2017 |
| | | AU | 2017200950 | B2 | 17 January 2019 |
| | | US | 2016090595 | A1 | 31 March 2016 |
| | | US | 9932581 | B2 | 03 April 2018 |
| | | US | 2014343123 | A1 | 20 November 2014 |
| | | US | 9127276 | B2 | 08 September 2015 |
| | | US | 2018002693 | A1 | 04 January 2018 |
| | | ES | 2885174 | T3 | 13 December 2021 |
| | | JP | 2020039354 | A | 19 March 2020 |
| | | JP | 6866459 | B2 | 28 April 2021 |
| | | IL | 272617 | A | 31 March 2020 |
| | | BR | 112015027377 | A2 | 29 August 2017 |
| | | BR | 112015027377 | A8 | 03 October 2017 |
| | | BR | 112015027377 | B1 | 10 January 2023 |

Form PCT/ISA/210 (patent family annex) (July 2022)

**INTERNATIONAL SEARCH REPORT**
**Information on patent family members**

International application No.
**PCT/CN2022/139500**

| Patent document cited in search report | Publication date (day/month/year) | Patent family member(s) | | | Publication date (day/month/year) |
|---|---|---|---|---|---|
| | | US | 2015126720 | A1 | 07 May 2015 |
| | | US | 9181550 | B2 | 10 November 2015 |
| | | AU | 2014259756 | A1 | 22 October 2015 |
| | | AU | 2014259756 | B2 | 23 February 2017 |
| | | UA | 121017 | C2 | 25 March 2020 |
| | | EP | 3690049 | A1 | 05 August 2020 |
| | | US | 2019367914 | A1 | 05 December 2019 |
| | | US | 10844379 | B2 | 24 November 2020 |
| | | MX | 2015015264 | A | 12 August 2016 |
| | | KR | 20210037752 | A | 06 April 2021 |
| | | JP | 2022017514 | A | 25 January 2022 |
| | | EA | 201592093 | A1 | 30 June 2016 |
| | | EA | 031393 | B1 | 28 December 2018 |
| | | MX | 2015015220 | A | 12 January 2016 |
| | | CA | 2921167 | A1 | 06 November 2014 |
| | | DK | 2992009 | T3 | 14 September 2020 |
| | | AU | 2020233603 | A1 | 01 October 2020 |
| | | PL | 2992009 | T3 | 30 November 2020 |
| | | JP | 2021074021 | A | 20 May 2021 |
| | | HK | 1221485 | A1 | 02 June 2017 |
| | | KR | 20200090966 | A | 29 July 2020 |
| | | IL | 284593 | A | 31 August 2021 |
| | | IL | 284593 | B | 01 October 2022 |
| | | KR | 20160002977 | A | 08 January 2016 |
| | | IL | 242126 | B | 31 January 2019 |
| | | AU | 2014259750 | A1 | 22 October 2015 |
| | | AU | 2014259750 | B2 | 28 February 2019 |
| | | MX | 2015015239 | A | 03 October 2016 |
| | | AU | 2020217347 | A1 | 27 August 2020 |
| | | AU | 2019204784 | A1 | 25 July 2019 |
| | | AU | 2019204784 | B2 | 27 January 2022 |
| | | AU | 2019204784 | C1 | 03 November 2022 |
| | | RU | 2015151204 | A | 02 June 2017 |
| | | RU | 2015151204 | A3 | 27 March 2018 |
| | | RU | 2686080 | C2 | 24 April 2019 |
| | | KR | 20160003723 | A | 11 January 2016 |
| | | KR | 102424855 | B1 | 26 July 2022 |
| | | SI | 2992098 | T1 | 28 June 2019 |
| | | PH | 12018501963 | A1 | 20 July 2020 |
| | | RU | 2015151202 | A | 06 June 2017 |
| | | RU | 2015151202 | A3 | 27 March 2018 |
| | | RU | 2670614 | C2 | 24 October 2018 |
| | | RU | 2670614 | C9 | 23 November 2018 |
| | | PT | 2992009 | T | 21 September 2020 |
| | | US | 2015126718 | A1 | 07 May 2015 |
| | | US | 9181549 | B2 | 10 November 2015 |
| | | DOP | 2015000268 | A | 30 November 2015 |
| | | NZ | 631537 | A | 26 May 2017 |
| | | BR | 112015027322 | A2 | 26 September 2017 |
| | | AU | 2019202598 | A1 | 02 May 2019 |

Form PCT/ISA/210 (patent family annex) (July 2022)

**INTERNATIONAL SEARCH REPORT**
**Information on patent family members**

International application No.
**PCT/CN2022/139500**

| Patent document cited in search report | Publication date (day/month/year) | Patent family member(s) | Publication date (day/month/year) |
|---|---|---|---|
| | | RU 2019110030 A | 06 May 2019 |
| | | US 11299736 B1 | 12 April 2022 |
| | | RS 58981 B1 | 30 August 2019 |
| | | KR 20160002975 A | 08 January 2016 |
| | | KR 101857707 B1 | 14 May 2018 |
| | | LT 2992009 T | 10 November 2020 |
| | | CR 20150612 A | 03 March 2016 |
| | | RU 2015151200 A3 | 14 January 2019 |
| | | RU 2015151200 A | 11 September 2019 |
| | | US 2019055554 A1 | 21 February 2019 |
| | | IL 284000 A | 29 July 2021 |
| | | CA 2921509 A1 | 06 November 2014 |
| | | MX 2019010443 A | 17 October 2019 |
| | | JP 2016526018 A | 01 September 2016 |
| | | JP 6769866 B2 | 14 October 2020 |
| | | HRP 20190987 T1 | 20 September 2019 |
| | | DK 2991656 T3 | 23 March 2020 |
| | | IL 270464 B | 29 July 2021 |
| | | US 2021087566 A1 | 25 March 2021 |
| | | EP 3546579 A1 | 02 October 2019 |
| | | SG 10201801813 YA | 27 April 2018 |
| | | KR 20210129257 A | 27 October 2021 |
| | | KR 102482890 B1 | 30 December 2022 |
| | | KR 20160002974 A | 08 January 2016 |
| | | KR 102235678 B1 | 05 April 2021 |
| WO 2019204668 A1 | 24 October 2019 | None | |
| CN 112876534 A | 01 June 2021 | None | |
| CN 111378655 A | 07 July 2020 | None | |
| WO 2018013525 A1 | 18 January 2018 | AU 2017296195 A1 | 24 January 2019 |
| | | EP 3481430 A1 | 15 May 2019 |
| | | EP 3481430 A4 | 01 April 2020 |
| | | CA 3030701 A1 | 18 January 2018 |
| | | US 2022218829 A1 | 14 July 2022 |
| | | US 2019224326 A1 | 25 July 2019 |
| | | US 11253601 B2 | 22 February 2022 |
| WO 2021142454 A1 | 15 July 2021 | EP 4087587 A1 | 16 November 2022 |
| | | WO 2021142454 A8 | 21 July 2022 |
| | | CA 3167444 A1 | 15 July 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202111545699
- WO 2021254360 A1
- WO 2014025805 A1

### Non-patent literature cited in the description


- **BERG K.** A new serum type system in man-the Lp system. *Acta Pathol Microbiol Scand,* 1963, vol. 59, 369-82
- **SCHMIDT K ; NOUREEN A ; KRONENBERG F et al.** Structure, Function, and Genetics of Lipoprotein(a), [J. *Journal of Lipid Research,* 2016, vol. 57 (8), 1339
- **ENAS EA ; VARKEY B ; DHARMARAJAN T S et al.** Lipoprotein(a): An independent, genetic, and causal factor for cardiovascular disease and acute myocardial infarction[J. *Indian Heart Journal,* 2019, vol. 71 (2
- **ALBERT YOUNGWOO JANG ; SEUNG HWAN HAN ; IL SUK SOHN et al.** Lipoprotein(a) and Cardiovascular Diseases[J. *Circulation Journal,* 2020, vol. 84, 867-874
- Antibodies: A Laboratory Manual and Molecular Cloning: A Laboratory Manual. Cold Spring Harbor Laboratory